# EUROPÄISCHE PATENTANMELDUNG

(11) **EP 2 471 930 A1**
(43) Veröffentlichungstag der Anmeldung: **04.07.2012**
(21) Anmeldenummer: 11194084.7
(22) Anmeldetag: 19.12.2003
(51) Int. Cl.: C12N 15/82, C12N 9/88, C12N 9/10, C12N 9/92, C12N 9/02, C12P 13/08

(54) **Verfahren zur Herstellung von Aminosäuren**

(30) Priorität: 20.12.2002 DE 10261188
(62) Teilanmeldung aus: 03785902.2
(71) Anmelder: Metanomics GmbH & Co. KGaA, 10589 Berlin (DE)
(72) Erfinder: Schmitz, Oliver, 14624 Dallgow-Döberitz (DE); Puzio, Piotr, 9030 Mariakerke (Gent) (BE); Blau, Astrid, 14532 Stahnsdorf (DE); Looser, Ralf, 13158 Berlin (DE); Wendel, Birgit, 13437 Berlin (DE); Kamlage, Beate, 12161 Berlin (DE); Plesch, Gunnar, 14482 Potsdam (DE)
(74) Vertreter: Heistracher, Elisabeth

(57) **Zusammenfassung**

Verfahren zur Herstellung von L-Lysin, L-Threonin oder L-Methionin in transgenen Organismen dadurch gekennzeichnet, dass das Verfahren folgende Schritte umfasst:
a) Einbringen einer Nukleinsäuresequenz, die für ein Lysin-abbauendes Protein codiert, oder
b) Einbringen einer Nukleinsäuresequenz, die den Lysinabbau in den transgenen Organismen erhöht und
c) Expression einer unter (a) oder (b) genannten Nukleinsäuresequenz im transgenen Organismus.

Vorteilhaft wird in Verfahrensschritt (a) eine Nukleinsäuresequenz ausgewählt aus der Gruppe:
i) einer Nukleinsäuresequenz mit der in, SEQ ID NO: 11, SEQ ID NO: 17, SEQ ID NO: 19, SEQ ID NO: 21, SEQ ID NO: 23 oder SEQ ID NO: 25 dargestellten Sequenz;
ii) einer Nukleinsäuresequenz, die aufgrund des degenerierten genetischen Codes durch Rückübersetzung der in SEQ ID NO: 12, SEQ ID NO: 18, SEQ ID NO: 20, SEQ ID NO: 22, SEQ ID NO: 24 oder SEQ ID NO: 26 dargestellten Aminosäuresequenz erhalten wird; und
iii) eines Derivats der in SEQ ID NO: 11, SEQ ID NO: 17, SEQ ID NO: 19, SEQ ID NO: 21, SEQ ID NO: 23 oder SEQ ID NO: 25 dargestellten Nukleinsäuresequenz, die für Polypeptide mit der in, SEQ ID NO: 12, SEQ ID NO: 18, SEQ ID NO: 20, SEQ ID NO: 22, SEQ ID NO: 24 oder SEQ ID NO: 26 dargestellten Aminosäuresequenz codiert und mindestens 50 % Homologie auf - Aminosäureebene aufweisen, ohne dass die biologische Aktivität der Polypeptide wesentlich reduziert ist;

eingebracht.

SEQUENCE LISTING

<110> Metanomics GmbH & Co. KGaA

<120> Verfahren zur Herstellung von Aminosäuren

<130> 2002_960

<140> PF54195

<141> 2002-12-20

<160> 26

<170> PatentIn version 3.1

<210> 1
<211> 1164
<212> DNA
<213> Saccharomyces cerevisiae

<220>

<221> CDS

<222> (1)..(1164)

<223> Threonin-Aldolase

<400> 1
atg act gaa ttc gaa ttg cct cca aaa tat atc acc gct gct aac gac 48
Met Thr Glu Phe Glu Leu Pro Pro Lys Tyr Ile Thr Ala Ala Asn Asp
1               5                   10                  15

ttg cgg tca gac aca ttc acc act cca act gca gag atg atg gag gcc 96
Leu Arg Ser Asp Thr Phe Thr Thr Pro Thr Ala Glu Met Met Glu Ala
            20                  25                  30

gct tta gag gcc tct atc ggt gac gct gtc tac ggt gaa gat gtt gac 144
Ala Leu Glu Ala Ser Ile Gly Asp Ala Val Tyr Gly Glu Asp Val Asp
        35                  40                  45

acc gtt agg ctc gaa cag acc gtt gcc cgc atg gct ggc aaa gaa gca 192
Thr Val Arg Leu Glu Gln Thr Val Ala Arg Met Ala Gly Lys Glu Ala
    50                  55                  60

ggt ttg ttc tgt gtc tct ggg act ttg tcc aac cag att gcc atc aga 240
Gly Leu Phe Cys Val Ser Gly Thr Leu Ser Asn Gln Ile Ala Ile Arg
65                  70                  75                  80

act cac ttg atg caa cct cca tac tct att cta tgt gat tac agg gct 288
Thr His Leu Met Gln Pro Pro Tyr Ser Ile Leu Cys Asp Tyr Arg Ala
                85                  90                  95

cac gtt tac act cac gaa gcc gct gga ctg gcg atc ttg tct caa gcg 336
His Val Tyr Thr His Glu Ala Ala Gly Leu Ala Ile Leu Ser Gln Ala
            100                 105                 110

atg gtg gtt cct gtg gtt cct tcc aac ggt gac tac ttg acc ttg gaa 384
Met Val Val Pro Val Val Pro Ser Asn Gly Asp Tyr Leu Thr Leu Glu
        115                 120                 125

gac atc aag tca cac tac gtc cca gac gac ggt gat att cac ggt gcc 432
Asp Ile Lys Ser His Tyr Val Pro Asp Asp Gly Asp Ile His Gly Ala
    130                 135                 140

ccc acc aga ttg att tct ctg gaa aac act tta cac ggt att gtt tat 480
Pro Thr Arg Leu Ile Ser Leu Glu Asn Thr Leu His Gly Ile Val Tyr
145                 150                 155                 160

cca ttg gaa gaa ctg gtc cgc atc aaa gct tgg tgt atg gaa aat ggt 528
Pro Leu Glu Glu Leu Val Arg Ile Lys Ala Trp Cys Met Glu Asn Gly
                165                 170                 175

ctc aaa cta cat tgt gac ggt gcc aga atc tgg aat gcc gct gca caa 576
Leu Lys Leu His Cys Asp Gly Ala Arg Ile Trp Asn Ala Ala Ala Gln
            180                 185                 190

tct ggc gtg cca tta aag caa tat ggg gaa atc ttc gac tcc atc tcc 624
Ser Gly Val Pro Leu Lys Gln Tyr Gly Glu Ile Phe Asp Ser Ile Ser
        195                 200                 205

atc tgt cta tcc aag tct atg ggt gct cct att ggg tcc gtc ttg gtt 672
Ile Cys Leu Ser Lys Ser Met Gly Ala Pro Ile Gly Ser Val Leu Val
    210                 215                 220

ggg aac ctt aag ttt gtc aag aag gcc acc cat ttc aga aaa caa caa 720
Gly Asn Leu Lys Phe Val Lys Lys Ala Thr His Phe Arg Lys Gln Gln
225                 230                 235                 240

ggt ggt ggt att aga caa tct ggt atg atg gct aga atg gct ctt gta 768
Gly Gly Gly Ile Arg Gln Ser Gly Met Met Ala Arg Met Ala Leu Val
                245                 250                 255

aac atc aac aac gat tgg aag tcc caa ttg ctg tac tcg cac tct ttg 816
Asn Ile Asn Asn Asp Trp Lys Ser Gln Leu Leu Tyr Ser His Ser Leu
            260                 265                 270

gct cat gaa tta gcc gaa tat tgt gag gca aag ggc atc ccg cta gag 864
Ala His Glu Leu Ala Glu Tyr Cys Glu Ala Lys Gly Ile Pro Leu Glu
        275                 280                 285

tct cca gca gac acc aac ttt gtc ttt att aac ctg aag gcc gct aga 912
Ser Pro Ala Asp Thr Asn Phe Val Phe Ile Asn Leu Lys Ala Ala Arg
    290                 295                 300

atg gac cca gat gtc ctt gtt aag aag ggt ttg aag tac aac gtt aag 960
Met Asp Pro Asp Val Leu Val Lys Lys Gly Leu Lys Tyr Asn Val Lys
305                 310                 315                 320

cta atg ggt ggt aga gtc tcg ttc cac tat caa gtc acc aga gat act 1008
Leu Met Gly Gly Arg Val Ser Phe His Tyr Gln Val Thr Arg Asp Thr

                325                 330                 335

ttg gaa aaa gtc aaa ttg gcc atc tcc gag gcc ttc gac tat gct aaa 1056
Leu Glu Lys Val Lys Leu Ala Ile Ser Glu Ala Phe Asp Tyr Ala Lys
            340                 345                 350

gaa cat cct ttc gac tgt aac gga cct acc cag att tac cgt agt gaa 1104
Glu His Pro Phe Asp Cys Asn Gly Pro Thr Gln Ile Tyr Arg Ser Glu
        355                 360                 365

tcc acc gag gtc gac gtt gat ggc aac gct atc cgc gaa ata aaa acc 1152
Ser Thr Glu Val Asp Val Asp Gly Asn Ala Ile Arg Glu Ile Lys Thr
    370                 375                 380

tac aaa tac tga 1164
Tyr Lys Tyr
385

<210> 2
<211> 387
<212> PRT
<213> Saccharomyces cerevisiae

<400> 2

Met Thr Glu Phe Glu Leu Pro Pro Lys Tyr Ile Thr Ala Ala Asn Asp
1               5                   10                  15

Leu Arg Ser Asp Thr Phe Thr Thr Pro Thr Ala Glu Met Met Glu Ala
            20                  25                  30

Ala Leu Glu Ala Ser Ile Gly Asp Ala Val Tyr Gly Glu Asp Val Asp
        35                  40                  45

Thr Val Arg Leu Glu Gln Thr Val Ala Arg Met Ala Gly Lys Glu Ala
    50                  55                  60

Gly Leu Phe Cys Val Ser Gly Thr Leu Ser Asn Gln Ile Ala Ile Arg
65                  70                  75                  80

Thr His Leu Met Gln Pro Pro Tyr Ser Ile Leu Cys Asp Tyr Arg Ala
                85                  90                  95

His Val Tyr Thr His Glu Ala Ala Gly Leu Ala Ile Leu Ser Gln Ala
            100                 105                 110

Met Val Val Pro Val Val Pro Ser Asn Gly Asp Tyr Leu Thr Leu Glu
        115                 120                 125

Asp Ile Lys Ser His Tyr Val Pro Asp Asp Gly Asp Ile His Gly Ala
    130                 135                 140

Pro Thr Arg Leu Ile Ser Leu Glu Asn Thr Leu His Gly Ile Val Tyr
145                 150                 155                 160

Pro Leu Glu Glu Leu Val Arg Ile Lys Ala Trp Cys Met Glu Asn Gly
                165                 170                 175

Leu Lys Leu His Cys Asp Gly Ala Arg Ile Trp Asn Ala Ala Ala Gln
            180                 185                 190

Ser Gly Val Pro Leu Lys Gln Tyr Gly Glu Ile Phe Asp Ser Ile Ser
        195                 200                 205

Ile Cys Leu Ser Lys Ser Met Gly Ala Pro Ile Gly Ser Val Leu Val
    210                 215                 220

Gly Asn Leu Lys Phe Val Lys Lys Ala Thr His Phe Arg Lys Gln Gln
225                 230                 235                 240

Gly Gly Gly Ile Arg Gln Ser Gly Met Met Ala Arg Met Ala Leu Val
                245                 250                 255

Asn Ile Asn Asn Asp Trp Lys Ser Gln Leu Leu Tyr Ser His Ser Leu
            260                 265                 270

Ala His Glu Leu Ala Glu Tyr Cys Glu Ala Lys Gly Ile Pro Leu Glu
        275                 280                 285

Ser Pro Ala Asp Thr Asn Phe Val Phe Ile Asn Leu Lys Ala Ala Arg
    290                 295                 300

Met Asp Pro Asp Val Leu Val Lys Lys Gly Leu Lys Tyr Asn Val Lys
305                 310                 315                 320

Leu Met Gly Gly Arg Val Ser Phe His Tyr Gln Val Thr Arg Asp Thr
                325                 330                 335

Leu Glu Lys Val Lys Leu Ala Ile Ser Glu Ala Phe Asp Tyr Ala Lys
            340                 345                 350

Glu His Pro Phe Asp Cys Asn Gly Pro Thr Gln Ile Tyr Arg Ser Glu
        355                 360                 365

Ser Thr Glu Val Asp Val Asp Gly Asn Ala Ile Arg Glu Ile Lys Thr
    370                 375                 380

Tyr Lys Tyr
385

<210> 3
<211> 376
<212> PRT
<213> Canola

<400> 3

Gly Cys Phe Ala Cys Tyr Leu Val Gly Gly Phe Ser Val Gln Glu Lys
1               5                   10                  15

Met Val Thr Arg Ile Val Asp Leu Arg Ser Asp Thr Val Thr Lys Pro
            20                  25                  30

Thr Glu Ala Met Arg Ala Ala Met Ala Ser Ala Glu Val Asp Asp Asp
        35                  40                  45

Val Leu Gly Tyr Asp Pro Thr Ala Phe Arg Leu Glu Thr Glu Met Ala
    50                  55                  60

Lys Thr Met Gly Lys Glu Ala Ala Leu Phe Val Pro Ser Gly Thr Met
65                  70                  75                  80

Gly Asn Leu Val Ser Val Leu Val His Cys Asp Val Arg Gly Ser Glu
                85                  90                  95

Val Ile Leu Gly Asp Asn Cys His Ile Asn Ile Phe Glu Asn Gly Gly
            100                 105                 110

Ile Ala Thr Ile Gly Gly Val His Pro Arg Gln Val Lys Asn Asn Asp
        115                 120                 125

Asp Gly Thr Met Asp Ile Asp Leu Ile Glu Ala Ala Ile Arg Asp Pro
    130                 135                 140

Met Gly Glu Leu Phe Tyr Pro Thr Thr Lys Leu Ile Cys Leu Glu Asn
145                 150                 155                 160

Thr His Ala Asn Ser Gly Gly Arg Cys Leu Ser Val Glu Tyr Thr Asp
                165                 170                 175

Arg Val Gly Glu Leu Ala Lys Lys His Gly Leu Lys Leu His Ile Asp
            180                 185                 190

Gly Ala Arg Ile Phe Asn Ala Ser Val Ala Leu Gly Val Pro Val Asp
        195                 200                 205

Arg Leu Val Gln Ala Ala Asp Ser Val Ser Val Cys Leu Ser Lys Gly
    210                 215                 220

Ile Gly Ala Pro Val Gly Ser Val Ile Val Gly Ser Lys Asn Phe Ile
225                 230                 235                 240

Ala Lys Ala Arg Arg Leu Arg Lys Thr Leu Gly Gly Gly Met Arg Gln
                245                 250                 255

Ile Gly Leu Leu Cys Ala Ala Ala Leu Val Ala Leu Gln Glu Asn Val
            260                 265                 270

Gly Lys Leu Glu Ser Asp His Lys Lys Ala Arg Leu Leu Ala Asp Gly
        275                 280                 285

Leu Asn Glu Val Lys Gly Leu Arg Val Asp Ala Cys Ser Val Glu Thr
    290                 295                 300

Asn Met Val Phe Ile Asp Ile Glu Glu Gly Thr Lys Thr Arg Ala Glu
305                 310                 315                 320

Lys Ile Cys Lys Tyr Met Glu Glu Arg Gly Ile Leu Val Met Gln Glu
                325                 330                 335

Ser Ser Ser Arg Met Arg Val Val Leu His His Gln Ile Ser Ala Ser
            340                 345                 350

Asp Val Gln Tyr Ala Leu Ser Cys Phe Gln Gln Ala Leu Ala Val Lys
        355                 360                 365

Gly Val Gln Lys Glu Met Gly Asn
    370                 375

<210> 4
<211> 115
<212> PRT
<213> Soybean

<400> 4

Leu Phe Gly Leu Leu Ala Ile Leu Leu Glu Tyr Leu Glu Lys Met Val
1               5                   10                  15

Pro Arg Ile Val Asp Leu Arg Ser Asp Thr Val Thr Lys Pro Ser Glu
            20                  25                  30

Ala Met Arg Ala Ala Met Ala Ser Ala Glu Val Asp Asp Asp Val Leu
        35                  40                  45

Gly Arg Asp Pro Ser Cys Phe Arg Leu Glu Thr Glu Met Ala Lys Ile
    50                  55                  60

Leu Gly Lys Glu Gly Ala Leu Phe Val Pro Ser Gly Thr Met Ala Asn
65                  70                  75                  80

Leu Ile Ser Val Leu Val His Cys Asp Ile Arg Gly Ser Glu Val Ile
                85                  90                  95

Leu Gly Asp Asn Ser His Ile His Ile Tyr Glu Asn Gly Gly Ile Ala
            100                 105                 110

Thr Leu Gly
        115

<210> 5
<211> 127
<212> PRT
<213> Rice

<220>

<221> misc_feature

<222> (1)..(127)

<223> unbekannt oder sonstige

<400> 5

Lys Thr Leu Xaa Gly Gly Met Arg Gln Val Gly Ile Leu Cys Ala Ala
1               5                   10                  15

Ala Leu Val Ala Leu Gln Glu Asn Val Gly Lys Leu Gln Ser Asp His
            20                  25                  30

Asn Lys Ala Lys Leu Leu Ala Asp Gly Leu Asn Glu Ile Lys Gly Leu
        35                  40                  45

Arg Val Asp Ile Ser Ser Val Glu Thr Asn Ile Ile Tyr Val Glu Val
    50                  55                  60

Glu Glu Gly Ser Arg Ala Thr Ala Ala Lys Leu Cys Lys Asp Leu Glu
65                  70                  75                  80

Asp Tyr Gly Ile Leu Leu Met Pro Met Gly Ser Ser Arg Leu Arg Ile
                85                  90                  95

Val Phe His His Gln Ile Ser Ala Ser Asp Val Gln Tyr Ala Leu Ser
            100                 105                 110

Cys Phe Gln Gln Ala Val Asn Gly Val Arg Asn Glu Asn Gly Asn
        115                 120                 125


<210>  6
<211>  147
<212>  PRT
<213>  Rice


<400>  6

Gly Arg Arg Phe Arg Ala Ile Arg Asp Pro Met Gly Glu Leu Phe Tyr
1               5                   10                  15

Pro Thr Thr Lys Leu Ile Cys Leu Glu Asn Thr His Ala Asn Ser Gly
            20                  25                  30

Gly Arg Cys Leu Ser Val Glu Tyr Thr Asp Arg Val Gly Glu Leu Ala
        35                  40                  45

Lys Lys His Gly Leu Lys Leu His Ile Asp Gly Ala Arg Ile Phe Asn
    50                  55                  60

Ala Ser Val Ala Leu Gly Val Pro Val Asp Arg Leu Val Gln Ala Ala
65                  70                  75                  80

Asp Ser Val Ser Val Cys Leu Ser Lys Gly Ile Gly Ala Pro Val Gly
                85                  90                  95

Ser Val Ile Val Gly Ser Lys Asn Phe Ile Ala Lys Ala Arg Arg Leu
            100                 105                 110

Arg Lys Thr Leu Gly Gly Gly Met Arg Gln Ile Gly Leu Leu Cys Ala
        115                 120                 125

Ala Ala Leu Val Ala Leu Gln Glu Asn Val Gly Lys Leu Glu Ser Asp
    130                 135                 140

His Lys Lys
145

<210>  7
<211>  169
<212>  PRT
<213>  Canola

<220>

<221>  misc_feature

<222>  (1)..(169)

<223>  unbekannt oder sonstige

<400>  7

Gly Ile Pro Gly Xaa Thr Phe Arg Gly Asp Val Ala Lys Ser His Gly
1               5                   10                  15

Leu Lys Leu His Ile Asp Gly Ala Arg Ile Phe Asn Ala Ser Val Ala
            20                  25                  30

Leu Gly Val Pro Val His Arg Leu Val Lys Ala Ala Asp Ser Val Ser
        35                  40                  45

Val Cys Ile Ser Lys Gly Leu Gly Ala Pro Val Gly Ser Val Ile Val
    50                  55                  60

Gly Ser Thr Ala Phe Ile Glu Lys Ala Lys Ile Leu Thr Lys Thr Leu
65                  70                  75                  80

Gly Gly Gly Met Arg Gln Val Gly Ile Leu Cys Ala Ala Ala Tyr Val
                85                  90                  95

Ala Val Arg Asp Thr Val Gly Lys Leu Ala Asp Asp His Arg Arg Ala
            100                 105                 110

Lys Val Leu Ala Asp Gly Leu Lys Lys Ile Lys His Phe Arg Val Asp
        115                 120                 125

Thr Thr Ser Val Glu Thr Asn Met Val Phe Phe Asp Ile Val Asp Ser
    130                 135                 140

Arg Ile Ser Pro Asp Lys Leu Cys Gln Val Leu Glu Gln Arg Asn Val
145                 150                 155                 160

Leu Ala Met Pro Ala Gly Ser Lys Arg
                165

<210> 8
<211> 362
<212> PRT
<213> Canola

<400> 8

Ile Glu Ile Lys Met Val Met Arg Thr Val Asp Leu Arg Ser Asp Thr
1               5                   10                  15

Val Thr Arg Pro Thr Asp Ala Met Arg Glu Ala Met Gly Ser Ala Glu
            20                  25                  30

Val Asp Asp Asp Val Leu Gly Tyr Asp Pro Thr Ala Arg Arg Leu Glu
        35                  40                  45

Glu Glu Ile Ala Lys Met Met Gly Lys Glu Ala Ala Leu Phe Val Pro
    50                  55                  60

Ser Gly Thr Met Gly Asn Leu Ile Cys Val Met Val His Cys Asp Val
65                  70                  75                  80

Arg Gly Ser Glu Val Ile Leu Gly Asp Asn Cys His Ile His Val Tyr
                85                  90                  95

Glu Asn Gly Gly Ile Ser Thr Ile Gly Gly Val His Pro Lys Thr Ile
            100                 105                 110

Lys Asn Glu Glu Asp Gly Thr Met Asp Leu Gly Ala Ile Glu Ala Ala
        115                 120                 125

Ile Arg Asp Pro Lys Gly Ser Thr Phe Tyr Pro Ser Thr Arg Leu Ile
    130                 135                 140

Cys Leu Glu Asn Thr His Ala Asn Ser Gly Gly Arg Cys Leu Ser Ala
145                 150                 155                 160

Glu Tyr Thr Asp Arg Val Gly Glu Ile Ala Lys Arg His Gly Leu Lys
                165                 170                 175

Leu His Ile Asp Gly Ala Arg Leu Phe Asn Ala Ser Ile Ala Leu Gly
            180                 185                 190

Val Pro Val His Arg Leu Val Gln Ala Ala Asp Ser Val Ser Val Cys
        195                 200                 205

Leu Ser Lys Gly Leu Gly Ala Pro Ile Gly Ser Val Val Val Gly Ser
    210                 215                 220

Gln Ser Phe Ile Glu Lys Ala Lys Thr Leu Arg Lys Thr Leu Gly Gly
225                 230                 235                 240

Gly Met Arg Gln Ile Gly Val Leu Cys Ala Ala Ala Leu Val Ala Leu
                245                 250                 255

Gln Glu Asn Leu Pro Lys Leu Gln Phe Asp His Lys Lys Thr Lys Leu
            260                 265                 270

Leu Ala Glu Gly Leu Asn Gln Met Lys Gly Ile Arg Val Asn Val Ala
        275                 280                 285

Ala Met Glu Thr Asn Met Ile Phe Met Asp Met Glu Asp Gly Ser Lys
290                 295                 300

Leu Thr Ala Glu Lys Leu Arg Lys Ser Leu Thr Glu His Gly Ile Leu
305                 310                 315                 320

Val Ile Pro Glu Asn Ser Thr Arg Ile Arg Met Val Leu His His Gln
                325                 330                 335

Ile Thr Thr Ser Asp Val His Tyr Thr Leu Ser Cys Leu Gln Gln Ala
            340                 345                 350

Val Gln Thr Ile His Glu Pro Cys Gln Asn
        355                 360

<210> 9
<211> 196
<212> PRT
<213> Canola

<400> 9

Gly Phe Leu Leu Lys His Lys Tyr Ile Tyr Tyr Cys Cys Tyr Leu Phe
1               5                   10                  15

Glu Ser Lys Ser Asn Asn Phe Leu Phe Ser Val Ile Lys Met Val Thr
            20                  25                  30

Pro Val Ile Arg Thr Val Asp Leu Arg Ser Asp Thr Val Thr Lys Pro
        35                  40                  45

Thr Glu Ser Met Arg Ser Ala Met Ala Asn Ala Glu Val Asp Asp Asp
    50                  55                  60

Val Leu Gly Asn Asp Pro Thr Ala Val Leu Leu Glu Arg Glu Val Ala
65                  70                  75                  80

Glu Ile Ala Gly Lys Glu Ala Ala Met Phe Val Pro Ser Gly Thr Met
                85                  90                  95

Gly Asn Leu Ile Ser Val Leu Val His Cys Asp Glu Arg Gly Ser Glu
            100                 105                 110

Val Ile Leu Gly Asp Asp Ser His Ile His Ile Tyr Glu Asn Gly Gly
        115                 120                 125

Val Ser Ser Leu Gly Gly Val His Pro Arg Thr Val Lys Asn Glu Glu
    130                 135                 140

Asp Gly Thr Met Glu Ile Ser Ser Ile Glu Ala Ala Val Arg Ser Pro
145                 150                 155                 160

Thr Gly Asp Leu His Tyr Pro Val Thr Lys Leu Ile Cys Leu Glu Asn
                165                 170                 175

Thr Gln Ala Asn Cys Gly Gly Arg Cys Leu Pro Ile Glu Tyr Ile Asp
            180                 185                 190

Lys Val Gly Glu
        195

<210> 10
<211> 104
<212> PRT
<213> Soybean

<400> 10

Ile Gly Ile Lys Met Val Met Arg Ile Val Asp Leu Arg Ser Asp Thr
1               5                   10                  15

Val Thr Arg Pro Thr Asp Ala Met Arg Glu Ala Met Ala Ser Ala Glu
            20                  25                  30

Val Asp Asp Asp Val Leu Gly Tyr Asp Pro Thr Ala Arg Gly Leu Glu
        35                  40                  45

Glu Glu Met Ala Lys Met Met Gly Lys Glu Ala Ala Leu Phe Val Pro
    50                  55                  60

Ser Gly Thr Met Gly Asn Leu Ile Cys Val Met Val His Cys Asp Val
65                  70                  75                  80

Arg Gly Ser Glu Val Ile Leu Gly Asp Thr Cys His Ile His Val Tyr
                85                  90                  95

Glu Asn Gly Gly Ile Ser Thr Ile
            100

<210> 11
<211> 738
<212> DNA
<213> Saccharomyces cerevisiae

<220>

<221> CDS

<222> (1)..(738)

<223> Lysin-Decarboxylase ähnliches Protein

<400> 11
atg aca atg gaa aaa aat gga ggt aat agc agc cgt ggt ggc caa gta      48
Met Thr Met Glu Lys Asn Gly Gly Asn Ser Ser Arg Gly Gly Gln Val
1               5                   10                  15

ggc ggc aag tct gtg tgt gtt tac tgc ggg tct tca ttt ggc gct aag      96
Gly Gly Lys Ser Val Cys Val Tyr Cys Gly Ser Ser Phe Gly Ala Lys
            20                  25                  30

gcg cta tac tca gaa agt gca gaa gaa tta gga gcc ctt ttc cat aag      144
Ala Leu Tyr Ser Glu Ser Ala Glu Glu Leu Gly Ala Leu Phe His Lys
        35                  40                  45

ctg gga tgg aaa ttg gta tac ggt gga ggc act act ggt ttg atg ggc 192
Leu Gly Trp Lys Leu Val Tyr Gly Gly Gly Thr Thr Gly Leu Met Gly
    50                  55                  60

aag ata gca agg tct acg atg gga cct gat tta agc gga cag gtt cac 240
Lys Ile Ala Arg Ser Thr Met Gly Pro Asp Leu Ser Gly Gln Val His
65                  70                  75                  80

ggt atc att cca aat gca ctt gtg tct aag gaa agg aca gac gag gat 288
Gly Ile Ile Pro Asn Ala Leu Val Ser Lys Glu Arg Thr Asp Glu Asp
                85                  90                  95

aaa gaa gat gtt aat aaa gca ttg ttg gag tct gta gaa aat cat aag 336
Lys Glu Asp Val Asn Lys Ala Leu Leu Glu Ser Val Glu Asn His Lys
            100                 105                 110

ggc gcc act cct att tct gaa gag tat ggg gaa aca acg att gta cca 384
Gly Ala Thr Pro Ile Ser Glu Glu Tyr Gly Glu Thr Thr Ile Val Pro
        115                 120                 125

gat atg cat acg aga aaa aga atg atg gca aat ttg agt gac gcg ttt 432
Asp Met His Thr Arg Lys Arg Met Met Ala Asn Leu Ser Asp Ala Phe
    130                 135                 140

gtt gct atg cct ggt gga tac ggg act ttt gaa gaa atc atg gaa tgt 480
Val Ala Met Pro Gly Gly Tyr Gly Thr Phe Glu Glu Ile Met Glu Cys
145                 150                 155                 160

atc acg tgg tcg caa ctg ggg att cat aat aaa cca att atc ttg ttc 528
Ile Thr Trp Ser Gln Leu Gly Ile His Asn Lys Pro Ile Ile Leu Phe
                165                 170                 175

aat atc gat ggg ttc tat gac aaa tta ttg gag ttc ctc aaa cac tct 576
Asn Ile Asp Gly Phe Tyr Asp Lys Leu Leu Glu Phe Leu Lys His Ser
            180                 185                 190

att caa gaa cgg ttc atc agt gtg aag aat ggt gaa atc att caa gtt 624
Ile Gln Glu Arg Phe Ile Ser Val Lys Asn Gly Glu Ile Ile Gln Val
        195                 200                 205

gcc tcc act ccg cag gaa gtt gtt gat aaa ata gag aag tac gtc gtt 672
Ala Ser Thr Pro Gln Glu Val Val Asp Lys Ile Glu Lys Tyr Val Val
    210                 215                 220

cca gag ggc cgt ttc aat ttg aat tgg agc gac gaa ggt cac gct cac 720
Pro Glu Gly Arg Phe Asn Leu Asn Trp Ser Asp Glu Gly His Ala His
225                 230                 235                 240

gag gat tgt gct aaa taa 738
Glu Asp Cys Ala Lys
                245

<210> 12
<211> 245
<212> PRT
<213> Saccharomyces cerevisiae

<400> 12

Met Thr Met Glu Lys Asn Gly Gly Asn Ser Ser Arg Gly Gly Gln Val
1               5                   10                  15

Gly Gly Lys Ser Val Cys Val Tyr Cys Gly Ser Ser Phe Gly Ala Lys
            20                  25                  30

Ala Leu Tyr Ser Glu Ser Ala Glu Glu Leu Gly Ala Leu Phe His Lys
        35                  40                  45

Leu Gly Trp Lys Leu Val Tyr Gly Gly Gly Thr Thr Gly Leu Met Gly
    50                  55                  60

Lys Ile Ala Arg Ser Thr Met Gly Pro Asp Leu Ser Gly Gln Val His
65                  70                  75                  80

Gly Ile Ile Pro Asn Ala Leu Val Ser Lys Glu Arg Thr Asp Glu Asp
                85                  90                  95

Lys Glu Asp Val Asn Lys Ala Leu Leu Glu Ser Val Glu Asn His Lys
            100                 105                 110

Gly Ala Thr Pro Ile Ser Glu Glu Tyr Gly Glu Thr Thr Ile Val Pro
        115                 120                 125

Asp Met His Thr Arg Lys Arg Met Met Ala Asn Leu Ser Asp Ala Phe
    130                 135                 140

Val Ala Met Pro Gly Gly Tyr Gly Thr Phe Glu Glu Ile Met Glu Cys
145                 150                 155                 160

Ile Thr Trp Ser Gln Leu Gly Ile His Asn Lys Pro Ile Ile Leu Phe
                165                 170                 175

Asn Ile Asp Gly Phe Tyr Asp Lys Leu Leu Glu Phe Leu Lys His Ser
            180                 185                 190

Ile Gln Glu Arg Phe Ile Ser Val Lys Asn Gly Glu Ile Ile Gln Val
        195                 200                 205

Ala Ser Thr Pro Gln Glu Val Val Asp Lys Ile Glu Lys Tyr Val Val
    210                 215                 220

Pro Glu Gly Arg Phe Asn Leu Asn Trp Ser Asp Glu Gly His Ala His
225                 230                 235                 240

Glu Asp Cys Ala Lys
                245

<210> 13
<211> 1083
<212> DNA
<213> Glycine max

<220>

<221> CDS

<222> (1)..(1083)

<223> Threonin-Aldolase

<400> 13
atg gta act aga att gtg gat ctt cgg tca gac aca gtt aca aag cca      48
Met Val Thr Arg Ile Val Asp Leu Arg Ser Asp Thr Val Thr Lys Pro
1               5                   10                  15

act gaa gca atg aga gct gct atg gca agt gct gaa gtt gat gac gat      96
Thr Glu Ala Met Arg Ala Ala Met Ala Ser Ala Glu Val Asp Asp Asp
            20                  25                  30

gtt cta ggc tat gat cca act gct ttt cgc tta gaa aca gag atg gca      144
Val Leu Gly Tyr Asp Pro Thr Ala Phe Arg Leu Glu Thr Glu Met Ala
        35                  40                  45

aag aca atg ggc aaa gaa gct gct ctt ttt gtt cca tct ggc act atg      192
Lys Thr Met Gly Lys Glu Ala Ala Leu Phe Val Pro Ser Gly Thr Met
    50                  55                  60

ggg aac ctt gta tct gta ctt gtt cat tgt gat gtc agg gga agt gag      240
Gly Asn Leu Val Ser Val Leu Val His Cys Asp Val Arg Gly Ser Glu
65                  70                  75                  80

gtt att ctt gga gac aat tgc cat atc aac att ttt gag aat gga ggc      288
Val Ile Leu Gly Asp Asn Cys His Ile Asn Ile Phe Glu Asn Gly Gly
                85                  90                  95

att gca acc att ggg gga gtg cat cca aga caa gtg aaa aat aac gat      336
Ile Ala Thr Ile Gly Gly Val His Pro Arg Gln Val Lys Asn Asn Asp
            100                 105                 110

gat gga acc atg gac att gat ttg att gag gct gct atc agg gac cca      384
Asp Gly Thr Met Asp Ile Asp Leu Ile Glu Ala Ala Ile Arg Asp Pro
        115                 120                 125

atg ggg gag cta ttc tat cca acc acc aag ctt att tgc ttg gaa aat      432
Met Gly Glu Leu Phe Tyr Pro Thr Thr Lys Leu Ile Cys Leu Glu Asn
    130                 135                 140

act cat gca aac tct ggt ggc aga tgc ctc tca gtt gaa tat aca gac      480
Thr His Ala Asn Ser Gly Gly Arg Cys Leu Ser Val Glu Tyr Thr Asp
145                 150                 155                 160

aga gtt gga gag tta gct aag aag cat gga ctg aag ctt cac att gat      528
Arg Val Gly Glu Leu Ala Lys Lys His Gly Leu Lys Leu His Ile Asp
                165                 170                 175

ggg gcc cgt att ttt aac gca tca gtt gca ctt ggt gtt cca gtg gat      576
Gly Ala Arg Ile Phe Asn Ala Ser Val Ala Leu Gly Val Pro Val Asp
            180                 185                 190

agg ctt gtc cag gcg gct gat tca gtt tcc gtt tgc cta tct aaa ggt      624
Arg Leu Val Gln Ala Ala Asp Ser Val Ser Val Cys Leu Ser Lys Gly
        195                 200                 205

ata ggt gct cca gtt gga tct gtt att gtt ggt tcc aag aat ttt att      672
Ile Gly Ala Pro Val Gly Ser Val Ile Val Gly Ser Lys Asn Phe Ile
    210                 215                 220

gcc aag gct aga cga ctc cgg aaa acc tta gga ggt gga atg aga cag      720
Ala Lys Ala Arg Arg Leu Arg Lys Thr Leu Gly Gly Gly Met Arg Gln
225                 230                 235                 240

att ggc ctc ctt tgt gcc gct gca ctt gtt gcc ttg cag gaa aat gtt      768

Ile Gly Leu Leu Cys Ala Ala Ala Leu Val Ala Leu Gln Glu Asn Val
                245                 250                 255

ggg aag ctg gaa agt gat cac aag aaa gct aga ctt ttg gct gat gga 816
Gly Lys Leu Glu Ser Asp His Lys Lys Ala Arg Leu Leu Ala Asp Gly
            260                 265                 270

tta aac gaa gtt aaa gga ttg aga gtg gat gcc tgt tct gtg gag acc 864
Leu Asn Glu Val Lys Gly Leu Arg Val Asp Ala Cys Ser Val Glu Thr
        275                 280                 285

aat atg gta ttt att gac att gaa gag ggt aca aag act aga gca gaa 912
Asn Met Val Phe Ile Asp Ile Glu Glu Gly Thr Lys Thr Arg Ala Glu
    290                 295                 300

aag ata tgc aag tac atg gaa gaa cgt ggt atc ctt gtg atg caa gag 960
Lys Ile Cys Lys Tyr Met Glu Glu Arg Gly Ile Leu Val Met Gln Glu
305                 310                 315                 320

agt tca tca aga atg aga gtt gtt ctc cat cac caa ata tca gca agt 1008
Ser Ser Ser Arg Met Arg Val Val Leu His His Gln Ile Ser Ala Ser
                325                 330                 335

gat gtg caa tat gcc ttg tcg tgc ttt cag caa gct cta gct gtc aaa 1056
Asp Val Gln Tyr Ala Leu Ser Cys Phe Gln Gln Ala Leu Ala Val Lys
            340                 345                 350

gga gta caa aag gaa atg ggc aac taa 1083
Gly Val Gln Lys Glu Met Gly Asn
        355                 360

<210> 14
<211> 360
<212> PRT
<213> Glycine max

<400> 14

Met Val Thr Arg Ile Val Asp Leu Arg Ser Asp Thr Val Thr Lys Pro
1               5                   10                  15

Thr Glu Ala Met Arg Ala Ala Met Ala Ser Ala Glu Val Asp Asp Asp
            20                  25                  30

Val Leu Gly Tyr Asp Pro Thr Ala Phe Arg Leu Glu Thr Glu Met Ala
        35                  40                  45

Lys Thr Met Gly Lys Glu Ala Ala Leu Phe Val Pro Ser Gly Thr Met
    50                  55                  60

Gly Asn Leu Val Ser Val Leu Val His Cys Asp Val Arg Gly Ser Glu
65                  70                  75                  80

Val Ile Leu Gly Asp Asn Cys His Ile Asn Ile Phe Glu Asn Gly Gly
                85                  90                  95

Ile Ala Thr Ile Gly Gly Val His Pro Arg Gln Val Lys Asn Asn Asp
            100                 105                 110

Asp Gly Thr Met Asp Ile Asp Leu Ile Glu Ala Ala Ile Arg Asp Pro
        115                 120                 125

Met Gly Glu Leu Phe Tyr Pro Thr Thr Lys Leu Ile Cys Leu Glu Asn
    130                 135                 140

Thr His Ala Asn Ser Gly Gly Arg Cys Leu Ser Val Glu Tyr Thr Asp
145                 150                 155                 160

Arg Val Gly Glu Leu Ala Lys Lys His Gly Leu Lys Leu His Ile Asp
                165                 170                 175

Gly Ala Arg Ile Phe Asn Ala Ser Val Ala Leu Gly Val Pro Val Asp
            180                 185                 190

Arg Leu Val Gln Ala Ala Asp Ser Val Ser Val Cys Leu Ser Lys Gly
        195                 200                 205

Ile Gly Ala Pro Val Gly Ser Val Ile Val Gly Ser Lys Asn Phe Ile
    210                 215                 220

Ala Lys Ala Arg Arg Leu Arg Lys Thr Leu Gly Gly Gly Met Arg Gln
225                 230                 235                 240

Ile Gly Leu Leu Cys Ala Ala Ala Leu Val Ala Leu Gln Glu Asn Val
                245                 250                 255

Gly Lys Leu Glu Ser Asp His Lys Lys Ala Arg Leu Leu Ala Asp Gly
            260                 265                 270

Leu Asn Glu Val Lys Gly Leu Arg Val Asp Ala Cys Ser Val Glu Thr
        275                 280                 285

Asn Met Val Phe Ile Asp Ile Glu Glu Gly Thr Lys Thr Arg Ala Glu
    290                 295                 300

Lys Ile Cys Lys Tyr Met Glu Glu Arg Gly Ile Leu Val Met Gln Glu
305                 310                 315                 320

Ser Ser Ser Arg Met Arg Val Val Leu His His Gln Ile Ser Ala Ser
                325                 330                 335

Asp Val Gln Tyr Ala Leu Ser Cys Phe Gln Gln Ala Leu Ala Val Lys
            340                 345                 350

Gly Val Gln Lys Glu Met Gly Asn
        355                 360


<210>  15
<211>  1077
<212>  DNA
<213>  Brassica napus


<220>

<221>  CDS

<222>  (1)..(1077)

<223>  Threonin-Aldolase

<400> 15
atg gtg atg cga act gtg gat cta cgg tca gac acc gtg act aga cct 48
Met Val Met Arg Thr Val Asp Leu Arg Ser Asp Thr Val Thr Arg Pro
1               5                   10                  15

acc gat gcc atg cgt gaa gca atg gga agc gca gaa gta gac gat gac 96
Thr Asp Ala Met Arg Glu Ala Met Gly Ser Ala Glu Val Asp Asp Asp
            20                  25                  30

gtc ctc ggc tac gac cca acg gct cga cgt ctt gaa gag gag ata gcc 144
Val Leu Gly Tyr Asp Pro Thr Ala Arg Arg Leu Glu Glu Glu Ile Ala
        35                  40                  45

aag atg atg ggg aaa gaa gca gct ctc ttc gtg cca tct ggt aca atg 192
Lys Met Met Gly Lys Glu Ala Ala Leu Phe Val Pro Ser Gly Thr Met
    50                  55                  60

ggg aac ctc ata tgc gtt atg gtt cac tgc gac gtg aga ggc agc gag 240
Gly Asn Leu Ile Cys Val Met Val His Cys Asp Val Arg Gly Ser Glu
65                  70                  75                  80

gtg att ctt gga gac aac tgt cac atc cat gtc tac gag aac gga ggg 288
Val Ile Leu Gly Asp Asn Cys His Ile His Val Tyr Glu Asn Gly Gly
                85                  90                  95

ata tca acg ata gga ggc gtg cat ccc aag aca atc aag aat gaa gaa 336
Ile Ser Thr Ile Gly Gly Val His Pro Lys Thr Ile Lys Asn Glu Glu
            100                 105                 110

gac ggg aca atg gac ttg ggg gct ata gaa gca gct att aga gat cct 384
Asp Gly Thr Met Asp Leu Gly Ala Ile Glu Ala Ala Ile Arg Asp Pro
        115                 120                 125

aaa gga agc acg ttt tat cca tca aca agg ttg att tgt ttg gag aac 432
Lys Gly Ser Thr Phe Tyr Pro Ser Thr Arg Leu Ile Cys Leu Glu Asn
    130                 135                 140

aca cat gcc aac tct ggt ggg aga tgt ttg agt gcg gaa tac aca gat 480
Thr His Ala Asn Ser Gly Gly Arg Cys Leu Ser Ala Glu Tyr Thr Asp
145                 150                 155                 160

aga gtt gga gag att gcc aag aga cat gga tta aag ctt cat atc gat 528
Arg Val Gly Glu Ile Ala Lys Arg His Gly Leu Lys Leu His Ile Asp
                165                 170                 175

gga gct cgc ctt ttt aat gct tcc att gca ctt gga gtt cca gtc cat 576
Gly Ala Arg Leu Phe Asn Ala Ser Ile Ala Leu Gly Val Pro Val His
            180                 185                 190

agg ctt gta cag gct gct gac tct gtt tcg gtg tgt ctc tct aaa ggt 624
Arg Leu Val Gln Ala Ala Asp Ser Val Ser Val Cys Leu Ser Lys Gly
        195                 200                 205

ctt gga gct cca ata gga tct gta gtc gtt ggt tca cag agt ttc ata 672
Leu Gly Ala Pro Ile Gly Ser Val Val Val Gly Ser Gln Ser Phe Ile
    210                 215                 220

gaa aag gcg aaa acg tta aga aaa aca tta ggt gga gga atg aga caa 720
Glu Lys Ala Lys Thr Leu Arg Lys Thr Leu Gly Gly Gly Met Arg Gln
225                 230                 235                 240

ata ggc gtc ctg tgc gca gcc gct ttg gtc gca ctt caa gag aat ctc 768
Ile Gly Val Leu Cys Ala Ala Ala Leu Val Ala Leu Gln Glu Asn Leu
                245                 250                 255

cca aag tta caa ttt gac cac aag aag aca aaa ttg tta gct gaa ggg 816
Pro Lys Leu Gln Phe Asp His Lys Lys Thr Lys Leu Leu Ala Glu Gly
            260                 265                 270

ttg aat caa atg aaa ggg att aga gtg aac gtt gca gcc atg gag acc 864
Leu Asn Gln Met Lys Gly Ile Arg Val Asn Val Ala Ala Met Glu Thr
        275                 280                 285

aac atg ata ttc atg gat atg gag gat gga tca aaa ctg acc gct gaa 912
Asn Met Ile Phe Met Asp Met Glu Asp Gly Ser Lys Leu Thr Ala Glu
    290                 295                 300

aaa ctc cgc aag agt cta acg gag cat ggc att ctc gtc atc cct gaa 960
Lys Leu Arg Lys Ser Leu Thr Glu His Gly Ile Leu Val Ile Pro Glu
305                 310                 315                 320

aac tct acc cga atc aga atg gtt cta cac cac cag ata aca aca agt 1008
Asn Ser Thr Arg Ile Arg Met Val Leu His His Gln Ile Thr Thr Ser
                325                 330                 335

gat gtg cat tac aca ttg tct tgc tta caa caa gca gtg cag acg att 1056
Asp Val His Tyr Thr Leu Ser Cys Leu Gln Gln Ala Val Gln Thr Ile
            340                 345                 350

cat gaa cca tgc caa aac taa 1077
His Glu Pro Cys Gln Asn
        355

<210> 16
<211> 358
<212> PRT
<213> Brassica napus

<400> 16

Met Val Met Arg Thr Val Asp Leu Arg Ser Asp Thr Val Thr Arg Pro
1               5                   10                  15

Thr Asp Ala Met Arg Glu Ala Met Gly Ser Ala Glu Val Asp Asp Asp
            20                  25                  30

Val Leu Gly Tyr Asp Pro Thr Ala Arg Arg Leu Glu Glu Glu Ile Ala
        35                  40                  45

Lys Met Met Gly Lys Glu Ala Ala Leu Phe Val Pro Ser Gly Thr Met
    50                  55                  60

Gly Asn Leu Ile Cys Val Met Val His Cys Asp Val Arg Gly Ser Glu
65                  70                  75                  80

Val Ile Leu Gly Asp Asn Cys His Ile His Val Tyr Glu Asn Gly Gly
                85                  90                  95

Ile Ser Thr Ile Gly Gly Val His Pro Lys Thr Ile Lys Asn Glu Glu
            100                 105                 110

Asp Gly Thr Met Asp Leu Gly Ala Ile Glu Ala Ala Ile Arg Asp Pro
        115                 120                 125

Lys Gly Ser Thr Phe Tyr Pro Ser Thr Arg Leu Ile Cys Leu Glu Asn
    130                 135                 140

Thr His Ala Asn Ser Gly Gly Arg Cys Leu Ser Ala Glu Tyr Thr Asp
145                 150                 155                 160

Arg Val Gly Glu Ile Ala Lys Arg His Gly Leu Lys Leu His Ile Asp
                165                 170                 175

Gly Ala Arg Leu Phe Asn Ala Ser Ile Ala Leu Gly Val Pro Val His
            180                 185                 190

Arg Leu Val Gln Ala Ala Asp Ser Val Ser Val Cys Leu Ser Lys Gly
        195                 200                 205

Leu Gly Ala Pro Ile Gly Ser Val Val Val Gly Ser Gln Ser Phe Ile
    210                 215                 220

Glu Lys Ala Lys Thr Leu Arg Lys Thr Leu Gly Gly Gly Met Arg Gln
225                 230                 235                 240

Ile Gly Val Leu Cys Ala Ala Ala Leu Val Ala Leu Gln Glu Asn Leu
                245                 250                 255

Pro Lys Leu Gln Phe Asp His Lys Lys Thr Lys Leu Leu Ala Glu Gly
            260                 265                 270

Leu Asn Gln Met Lys Gly Ile Arg Val Asn Val Ala Ala Met Glu Thr
        275                 280                 285

Asn Met Ile Phe Met Asp Met Glu Asp Gly Ser Lys Leu Thr Ala Glu
    290                 295                 300

Lys Leu Arg Lys Ser Leu Thr Glu His Gly Ile Leu Val Ile Pro Glu
305                 310                 315                 320

Asn Ser Thr Arg Ile Arg Met Val Leu His His Gln Ile Thr Thr Ser
                325                 330                 335

Asp Val His Tyr Thr Leu Ser Cys Leu Gln Gln Ala Val Gln Thr Ile
            340                 345                 350

His Glu Pro Cys Gln Asn
        355


<210>  17
<211>  570
<212>  DNA
<213>  Glycine max



<220>

<221>  CDS

<222>  (1)..(570)

<223>  Lysin-Decarboxylase

<400> 17
atg gaa ata agg gtt tca aag ttc aag agg att tgt gtc ttc tgt ggg 48
Met Glu Ile Arg Val Ser Lys Phe Lys Arg Ile Cys Val Phe Cys Gly
1               5                   10                  15

agt agc cct ggc aaa aag aga agc tac caa gat gct gcc att gaa ctt 96
Ser Ser Pro Gly Lys Lys Arg Ser Tyr Gln Asp Ala Ala Ile Glu Leu
            20                  25                  30

ggc aat gaa ttg gtc tca agg aac att gat ctg gtg tat gga ggg gga 144
Gly Asn Glu Leu Val Ser Arg Asn Ile Asp Leu Val Tyr Gly Gly Gly
        35                  40                  45

agc att ggt cta atg ggt tta gtt tca caa gct gtt cat gat ggc ggt 192
Ser Ile Gly Leu Met Gly Leu Val Ser Gln Ala Val His Asp Gly Gly
    50                  55                  60

cgg cat gtc atc gga gtt att ccc aag acc ctc atg cct cga gag cta 240
Arg His Val Ile Gly Val Ile Pro Lys Thr Leu Met Pro Arg Glu Leu
65                  70                  75                  80

act ggt gaa aca gtg gga gaa gta aaa gct gtt gct gat atg cac caa 288
Thr Gly Glu Thr Val Gly Glu Val Lys Ala Val Ala Asp Met His Gln
                85                  90                  95

agg aag gca gag atg gcc aag cat tca gac gcc ttt att gcc tta cca 336
Arg Lys Ala Glu Met Ala Lys His Ser Asp Ala Phe Ile Ala Leu Pro
            100                 105                 110

ggt gga tat ggg act cta gag gag ctt ctt gaa gtc ata acc tgg gca 384
Gly Gly Tyr Gly Thr Leu Glu Glu Leu Leu Glu Val Ile Thr Trp Ala
        115                 120                 125

caa ctt ggg att cat gac aag ccg gtg gga tta gta aat gtt gat gga 432
Gln Leu Gly Ile His Asp Lys Pro Val Gly Leu Val Asn Val Asp Gly
    130                 135                 140

tac ttt aat tcc ttg ctg tca ttt att gac aaa gct gtg gaa gag gga 480
Tyr Phe Asn Ser Leu Leu Ser Phe Ile Asp Lys Ala Val Glu Glu Gly
145                 150                 155                 160

ttt atc agt cca aat gct cgc cac ata att gta tca gca ccc aca gca 528
Phe Ile Ser Pro Asn Ala Arg His Ile Ile Val Ser Ala Pro Thr Ala
                165                 170                 175

aaa gag ttg gtg aag aaa ttg gag gat tac gtt ccc tgt taa 570
Lys Glu Leu Val Lys Lys Leu Glu Asp Tyr Val Pro Cys
            180                 185

<210> 18
<211> 189
<212> PRT
<213> Glycine max

<400> 18

Met Glu Ile Arg Val Ser Lys Phe Lys Arg Ile Cys Val Phe Cys Gly
1               5                   10                  15

Ser Ser Pro Gly Lys Lys Arg Ser Tyr Gln Asp Ala Ala Ile Glu Leu
            20                  25                  30

Gly Asn Glu Leu Val Ser Arg Asn Ile Asp Leu Val Tyr Gly Gly Gly
        35                  40                  45

Ser Ile Gly Leu Met Gly Leu Val Ser Gln Ala Val His Asp Gly Gly
    50                  55                  60

Arg His Val Ile Gly Val Ile Pro Lys Thr Leu Met Pro Arg Glu Leu
65                  70                  75                  80

Thr Gly Glu Thr Val Gly Glu Val Lys Ala Val Ala Asp Met His Gln
                85                  90                  95

Arg Lys Ala Glu Met Ala Lys His Ser Asp Ala Phe Ile Ala Leu Pro
            100                 105                 110

Gly Gly Tyr Gly Thr Leu Glu Glu Leu Leu Glu Val Ile Thr Trp Ala
        115                 120                 125

Gln Leu Gly Ile His Asp Lys Pro Val Gly Leu Val Asn Val Asp Gly
    130                 135                 140

Tyr Phe Asn Ser Leu Leu Ser Phe Ile Asp Lys Ala Val Glu Glu Gly
145                 150                 155                 160

Phe Ile Ser Pro Asn Ala Arg His Ile Ile Val Ser Ala Pro Thr Ala
                165                 170                 175

Lys Glu Leu Val Lys Lys Leu Glu Asp Tyr Val Pro Cys
            180                 185

<210>  19
<211>  675
<212>  DNA
<213>  Hordeum vulgare

<220>

<221>  CDS

<222>  (1)..(675)

<223>  Lysin-Decarboxylase

<400>  19
atg ggc gac acc acc gcg ccc tcg ccg ccg agg agg ttc ggc agg atc      48
Met Gly Asp Thr Thr Ala Pro Ser Pro Pro Arg Arg Phe Gly Arg Ile
1               5                   10                  15

tgc gtc ttc tgc ggc agg aac tcc ggc aac cgc gcc gtg ttc ggc gac      96
Cys Val Phe Cys Gly Arg Asn Ser Gly Asn Arg Ala Val Phe Gly Asp
            20                  25                  30

gcc gcg ctc gag ctc ggc cag ggc ctg gtg acg agg ggg gtc gat ctg 144
Ala Ala Leu Glu Leu Gly Gln Gly Leu Val Thr Arg Gly Val Asp Leu
        35                  40                  45

gtc tac ggc ggc ggc agt atc ggg ctg atg ggc ctg atc gcg cag acg 192
Val Tyr Gly Gly Gly Ser Ile Gly Leu Met Gly Leu Ile Ala Gln Thr
    50                  55                  60

gtt ctc gac ggc ggc tgc cgc gtc ctc ggg gtg att cca aga gca ctc 240
Val Leu Asp Gly Gly Cys Arg Val Leu Gly Val Ile Pro Arg Ala Leu
65                  70                  75                  80

atg ccc ctc gag ata tcc ggt gca agt gtt gga gaa gta aag att gtc 288
Met Pro Leu Glu Ile Ser Gly Ala Ser Val Gly Glu Val Lys Ile Val
                85                  90                  95

tcc gac atg cat gag agg aaa gct gag atg gcg cga caa gcc gat gca 336
Ser Asp Met His Glu Arg Lys Ala Glu Met Ala Arg Gln Ala Asp Ala
            100                 105                 110

ttc att gct ctt ccg ggt ggg tat gga aca atg gaa gag ctg gta gag 384
Phe Ile Ala Leu Pro Gly Gly Tyr Gly Thr Met Glu Glu Leu Val Glu
        115                 120                 125

atg atc act tgg tcg cag ctt gga atc cat gac aaa ccg gtc ggg ttg 432
Met Ile Thr Trp Ser Gln Leu Gly Ile His Asp Lys Pro Val Gly Leu
    130                 135                 140

cta aac gtc gat ggg tac tat gat ccg tta ctc gcg ctg ttc gac aag 480
Leu Asn Val Asp Gly Tyr Tyr Asp Pro Leu Leu Ala Leu Phe Asp Lys
145                 150                 155                 160

ggc gcg ggg gaa ggg ttt ttt aag gcc gat tgc agg ccg ata atc gtg 528
Gly Ala Gly Glu Gly Phe Phe Lys Ala Asp Cys Arg Pro Ile Ile Val
                165                 170                 175

tcg gca cca act gcc cac gaa ctg ctg aca aaa atg gag caa tac acc 576
Ser Ala Pro Thr Ala His Glu Leu Leu Thr Lys Met Glu Gln Tyr Thr
            180                 185                 190

cgt tca ccc cgg gag gtg gcc tcg cgg acg agc tgg gag atg acc gag 624
Arg Ser Pro Arg Glu Val Ala Ser Arg Thr Ser Trp Glu Met Thr Glu
        195                 200                 205

atg ggc tcc ggg aaa gca ccg gag ccg gag gag gag gcg gcg gca tcg 672
Met Gly Ser Gly Lys Ala Pro Glu Pro Glu Glu Glu Ala Ala Ala Ser
    210                 215                 220

taa 675

<210> 20
<211> 224
<212> PRT
<213> Hordeum vulgare

<400> 20

Met Gly Asp Thr Thr Ala Pro Ser Pro Pro Arg Arg Phe Gly Arg Ile
1               5                   10                  15

Cys Val Phe Cys Gly Arg Asn Ser Gly Asn Arg Ala Val Phe Gly Asp
            20                  25                  30

Ala Ala Leu Glu Leu Gly Gln Gly Leu Val Thr Arg Gly Val Asp Leu
        35                  40                  45

Val Tyr Gly Gly Gly Ser Ile Gly Leu Met Gly Leu Ile Ala Gln Thr
    50                  55                  60

Val Leu Asp Gly Gly Cys Arg Val Leu Gly Val Ile Pro Arg Ala Leu
65                  70                  75                  80

Met Pro Leu Glu Ile Ser Gly Ala Ser Val Gly Glu Val Lys Ile Val
                85                  90                  95

Ser Asp Met His Glu Arg Lys Ala Glu Met Ala Arg Gln Ala Asp Ala
            100                 105                 110

Phe Ile Ala Leu Pro Gly Gly Tyr Gly Thr Met Glu Glu Leu Val Glu
        115                 120                 125

Met Ile Thr Trp Ser Gln Leu Gly Ile His Asp Lys Pro Val Gly Leu
    130                 135                 140

Leu Asn Val Asp Gly Tyr Tyr Asp Pro Leu Leu Ala Leu Phe Asp Lys
145                 150                 155                 160

Gly Ala Gly Glu Gly Phe Phe Lys Ala Asp Cys Arg Pro Ile Ile Val
                165                 170                 175

Ser Ala Pro Thr Ala His Glu Leu Leu Thr Lys Met Glu Gln Tyr Thr
            180                 185                 190

Arg Ser Pro Arg Glu Val Ala Ser Arg Thr Ser Trp Glu Met Thr Glu
        195                 200                 205

Met Gly Ser Gly Lys Ala Pro Glu Pro Glu Glu Glu Ala Ala Ala Ser
    210                 215                 220

<210>  21
<211>  717
<212>  DNA
<213>  artifiziell

<220>

<221>  CDS

<222>  (1)..(717)

<223>  Lysin-Decarboxylase

<400>  21
atg gag gag aat caa gag aag ttt gct ccg gag agc agc ggc ggc gac        48
Met Glu Glu Asn Gln Glu Lys Phe Ala Pro Glu Ser Ser Gly Gly Asp
1               5                   10                  15

ggt ggt ggc tcg gtg aga acg atc tgc gtc ttc tgc ggc agc agg ccg     96
Gly Gly Gly Ser Val Arg Thr Ile Cys Val Phe Cys Gly Ser Arg Pro
        20                  25                  30

ggg aac cgg ccg tcc ttc agc gct gcg gcg ctc gac ctg ggg aag cag     144
Gly Asn Arg Pro Ser Phe Ser Ala Ala Ala Leu Asp Leu Gly Lys Gln
        35                  40                  45

ctg gtc gag agg cag atg aac ctg gtg tac ggc ggc ggc agc ggc ggg     192
Leu Val Glu Arg Gln Met Asn Leu Val Tyr Gly Gly Gly Ser Gly Gly
    50                  55                  60

ctg atg ggc ctg gtg tcc aag gcc gtc tac gaa ggc ggc cgc cac gtc     240
Leu Met Gly Leu Val Ser Lys Ala Val Tyr Glu Gly Gly Arg His Val
65                  70                  75                  80

ctc ggg gtc atc cct acc gcc ctc cta cct gaa gag gtg tca ggg gag     288
Leu Gly Val Ile Pro Thr Ala Leu Leu Pro Glu Glu Val Ser Gly Glu
                85                  90                  95

aca ttg gga gag gtg aaa gtg gtc agg gac atg cat cag cgc aag gcg     336
Thr Leu Gly Glu Val Lys Val Val Arg Asp Met His Gln Arg Lys Ala
            100                 105                 110

gaa atg gcg aaa cat gcc gac gct ttc atc gcc ctg cca ggt ggt tac     384
Glu Met Ala Lys His Ala Asp Ala Phe Ile Ala Leu Pro Gly Gly Tyr
        115                 120                 125

ggg aca atc gaa gaa ctg ctg gag atc ata gcg tgg gcg cag ctg ggc     432
Gly Thr Ile Glu Glu Leu Leu Glu Ile Ile Ala Trp Ala Gln Leu Gly
    130                 135                 140

atc cac agc aaa ccg gtg ggg ttg ctc aac gtg gac ggc tac tac aac     480
Ile His Ser Lys Pro Val Gly Leu Leu Asn Val Asp Gly Tyr Tyr Asn
145                 150                 155                 160

agc ctg ctc tcg ctg ttc gac aag gct gtc gag gag ggc ttc atc gac     528
Ser Leu Leu Ser Leu Phe Asp Lys Ala Val Glu Glu Gly Phe Ile Asp
                165                 170                 175

acc aag gca cgg aac atc ttc gtc ctc gct gac acc gcc gcc gac ctg     576
Thr Lys Ala Arg Asn Ile Phe Val Leu Ala Asp Thr Ala Ala Asp Leu
            180                 185                 190

ctg act agg ctc acc atg atg gcg cgc ctg gca gcc gac gac gac gat     624
Leu Thr Arg Leu Thr Met Met Ala Arg Leu Ala Ala Asp Asp Asp Asp
        195                 200                 205

gct act act acc ccc aga gga gac gga gac gga gac gga gac gaa cac     672
Ala Thr Thr Thr Pro Arg Gly Asp Gly Asp Gly Asp Gly Asp Glu His
    210                 215                 220

aag ggg gcc acc acc gct gca ggc gtc aaa agg aaa agg ggc taa         717
Lys Gly Ala Thr Thr Ala Ala Gly Val Lys Arg Lys Arg Gly
225                 230                 235

<210> 22
<211> 238
<212> PRT
<213> artifiziell

<400> 22

Met Glu Glu Asn Gln Glu Lys Phe Ala Pro Glu Ser Ser Gly Gly Asp
1               5                   10                  15

Gly Gly Gly Ser Val Arg Thr Ile Cys Val Phe Cys Gly Ser Arg Pro
            20                  25                  30

Gly Asn Arg Pro Ser Phe Ser Ala Ala Ala Leu Asp Leu Gly Lys Gln
        35                  40                  45

Leu Val Glu Arg Gln Met Asn Leu Val Tyr Gly Gly Gly Ser Gly Gly
    50                  55                  60

Leu Met Gly Leu Val Ser Lys Ala Val Tyr Glu Gly Gly Arg His Val
65                  70                  75                  80

Leu Gly Val Ile Pro Thr Ala Leu Leu Pro Glu Glu Val Ser Gly Glu
                85                  90                  95

Thr Leu Gly Glu Val Lys Val Val Arg Asp Met His Gln Arg Lys Ala
            100                 105                 110

Glu Met Ala Lys His Ala Asp Ala Phe Ile Ala Leu Pro Gly Gly Tyr
        115                 120                 125

Gly Thr Ile Glu Glu Leu Leu Glu Ile Ile Ala Trp Ala Gln Leu Gly
    130                 135                 140

Ile His Ser Lys Pro Val Gly Leu Leu Asn Val Asp Gly Tyr Tyr Asn
145                 150                 155                 160

Ser Leu Leu Ser Leu Phe Asp Lys Ala Val Glu Glu Gly Phe Ile Asp
                165                 170                 175

Thr Lys Ala Arg Asn Ile Phe Val Leu Ala Asp Thr Ala Ala Asp Leu
            180                 185                 190

Leu Thr Arg Leu Thr Met Met Ala Arg Leu Ala Ala Asp Asp Asp Asp
        195                 200                 205

Ala Thr Thr Thr Pro Arg Gly Asp Gly Asp Gly Asp Gly Asp Glu His
    210                 215                 220

Lys Gly Ala Thr Thr Ala Ala Gly Val Lys Arg Lys Arg Gly
225                 230                 235

<210> 23
<211> 717
<212> DNA
<213> Zea mays

<220>

<221> CDS

<222> (1)..(717)

<223> Lysin-Decarboxylase

<400> 23
atg gag gag aat caa gag aag ttt gct ccg gag agc agc ggc ggc gac 48
Met Glu Glu Asn Gln Glu Lys Phe Ala Pro Glu Ser Ser Gly Gly Asp
1               5                   10                  15

ggt ggt ggc tcg gtg aga acg atc tgc gtc ttc tgc ggc agc agg ccg 96
Gly Gly Gly Ser Val Arg Thr Ile Cys Val Phe Cys Gly Ser Arg Pro
            20                  25                  30

ggg aac cgg ccg tcc ttc agc gct gcg gcg ctc gac ctg ggg aag cag 144
Gly Asn Arg Pro Ser Phe Ser Ala Ala Ala Leu Asp Leu Gly Lys Gln
        35                  40                  45

ctg gtc gag agg cag atg aac ctg gtg tac ggc ggc ggc agc ggc ggg 192
Leu Val Glu Arg Gln Met Asn Leu Val Tyr Gly Gly Gly Ser Gly Gly
    50                  55                  60

ctg atg ggc ctg gtg tcc aag gcc gtc tac gaa ggc ggc cgc cac gtc 240
Leu Met Gly Leu Val Ser Lys Ala Val Tyr Glu Gly Gly Arg His Val
65                  70                  75                  80

ctc ggg gtc atc cct acc gcc ctc cta cct gaa gag gtg tca ggg gag 288
Leu Gly Val Ile Pro Thr Ala Leu Leu Pro Glu Glu Val Ser Gly Glu
                85                  90                  95

aca ttg gga gag gtg aaa gtg gtc agg gac atg cat cag cgc aag gcg 336
Thr Leu Gly Glu Val Lys Val Val Arg Asp Met His Gln Arg Lys Ala
            100                 105                 110

gaa atg gcg aaa cat gcc gac gct ttc atc gcc ctg cca ggt ggt tac 384
Glu Met Ala Lys His Ala Asp Ala Phe Ile Ala Leu Pro Gly Gly Tyr
        115                 120                 125

ggg aca atc gaa gaa ctg ctg gag atc ata gcg tgg gcg cag ctg ggc 432
Gly Thr Ile Glu Glu Leu Leu Glu Ile Ile Ala Trp Ala Gln Leu Gly
    130                 135                 140

atc cac agc aaa ccg gtg ggg ttg ctc aac gtg gac ggc tac tac aac 480
Ile His Ser Lys Pro Val Gly Leu Leu Asn Val Asp Gly Tyr Tyr Asn
145                 150                 155                 160

agc ctg ctc tcg ctg ttc gac aag gct gtc gag gag ggc ttc atc gac 528
Ser Leu Leu Ser Leu Phe Asp Lys Ala Val Glu Glu Gly Phe Ile Asp
                165                 170                 175

acc aag gca cgg aac atc ttc gtc ctc gct gac acc gcc gcc gac ctg 576
Thr Lys Ala Arg Asn Ile Phe Val Leu Ala Asp Thr Ala Ala Asp Leu
            180                 185                 190

ctg act agg ctc acc atg atg gcg cgc ctg gca gcc gac gac gac gat 624
Leu Thr Arg Leu Thr Met Met Ala Arg Leu Ala Ala Asp Asp Asp Asp
        195                 200                 205

gct act act acc ccc aga gga gac gga gac gga gac gga gac gaa cac 672
Ala Thr Thr Thr Pro Arg Gly Asp Gly Asp Gly Asp Gly Asp Glu His
    210                 215                 220

aag ggg gcc acc acc gct gca ggc gtc aaa agg aaa agg ggc taa 717
Lys Gly Ala Thr Thr Ala Ala Gly Val Lys Arg Lys Arg Gly
225                 230                 235

<210> 24
<211> 238
<212> PRT
<213> Zea mays

<400> 24

Met Glu Glu Asn Gln Glu Lys Phe Ala Pro Glu Ser Ser Gly Gly Asp
1               5                   10                  15

Gly Gly Gly Ser Val Arg Thr Ile Cys Val Phe Cys Gly Ser Arg Pro
            20                  25                  30

Gly Asn Arg Pro Ser Phe Ser Ala Ala Ala Leu Asp Leu Gly Lys Gln
        35                  40                  45

Leu Val Glu Arg Gln Met Asn Leu Val Tyr Gly Gly Gly Ser Gly Gly
    50                  55                  60

Leu Met Gly Leu Val Ser Lys Ala Val Tyr Glu Gly Gly Arg His Val
65                  70                  75                  80

Leu Gly Val Ile Pro Thr Ala Leu Leu Pro Glu Glu Val Ser Gly Glu
                85                  90                  95

Thr Leu Gly Glu Val Lys Val Val Arg Asp Met His Gln Arg Lys Ala
            100                 105                 110

Glu Met Ala Lys His Ala Asp Ala Phe Ile Ala Leu Pro Gly Gly Tyr
        115                 120                 125

Gly Thr Ile Glu Glu Leu Leu Glu Ile Ile Ala Trp Ala Gln Leu Gly
    130                 135                 140

Ile His Ser Lys Pro Val Gly Leu Leu Asn Val Asp Gly Tyr Tyr Asn
145                 150                 155                 160

Ser Leu Leu Ser Leu Phe Asp Lys Ala Val Glu Glu Gly Phe Ile Asp
                165                 170                 175

Thr Lys Ala Arg Asn Ile Phe Val Leu Ala Asp Thr Ala Ala Asp Leu
            180                 185                 190

Leu Thr Arg Leu Thr Met Met Ala Arg Leu Ala Ala Asp Asp Asp Asp
        195                 200                 205

Ala Thr Thr Thr Pro Arg Gly Asp Gly Asp Gly Asp Gly Asp Glu His
    210                 215                 220

Lys Gly Ala Thr Thr Ala Ala Gly Val Lys Arg Lys Arg Gly
225                 230                 235

<210> 25
<211> 672
<212> DNA
<213> Oryza sativa

<220>

<221> CDS

<222> (1)..(672)

<223> Lysin-Decarboxylase

<400> 25
atg ggc gac aac agc gcc gcc gcg gcg gcc gtg gcc gcg ccg cgc ggc 48
Met Gly Asp Asn Ser Ala Ala Ala Ala Ala Val Ala Ala Pro Arg Gly
1               5                   10                  15

agg ttc ggc agg atc tgc gtc ttc tgc ggc agc aac gcc ggc aac cgc 96
Arg Phe Gly Arg Ile Cys Val Phe Cys Gly Ser Asn Ala Gly Asn Arg
            20                  25                  30

gcg gtg ttc ggc gac gcg gcg ctc cag ctc ggg cag gag ctg gtg tcg 144
Ala Val Phe Gly Asp Ala Ala Leu Gln Leu Gly Gln Glu Leu Val Ser
        35                  40                  45

aga ggg atc gag ttg gtc tac ggt ggc ggc agc gtc ggg ttg atg ggc 192
Arg Gly Ile Glu Leu Val Tyr Gly Gly Gly Ser Val Gly Leu Met Gly
    50                  55                  60

ttg atc gcg cag acg gtt ctt gat ggc ggc tgc ggt gtt ctc ggg gtg 240
Leu Ile Ala Gln Thr Val Leu Asp Gly Gly Cys Gly Val Leu Gly Val
65                  70                  75                  80

att cca aaa gca ctt atg ccc acc gag ata tca ggt gca agt gtt gga 288
Ile Pro Lys Ala Leu Met Pro Thr Glu Ile Ser Gly Ala Ser Val Gly
                85                  90                  95

gaa gtg aaa att gtg tct gac atg cat gag agg aaa gct gag atg gca 336
Glu Val Lys Ile Val Ser Asp Met His Glu Arg Lys Ala Glu Met Ala
            100                 105                 110

cgc caa tcc gat gcc ttc atc gct ctt cct gga ggg tat gga aca atg 384
Arg Gln Ser Asp Ala Phe Ile Ala Leu Pro Gly Gly Tyr Gly Thr Met
        115                 120                 125

gag gag ttg tta gag atg ata act tgg tca caa ctt gga att cat gac 432
Glu Glu Leu Leu Glu Met Ile Thr Trp Ser Gln Leu Gly Ile His Asp
    130                 135                 140

aaa cca gtt ggg ttg ctg aat gtg gac ggt tac tat gat ccg ttg ctt 480
Lys Pro Val Gly Leu Leu Asn Val Asp Gly Tyr Tyr Asp Pro Leu Leu
145                 150                 155                 160

gcg cta ttt gat aag ggt gcg gca gaa gga ttt att aag gcc gat tgc 528
Ala Leu Phe Asp Lys Gly Ala Ala Glu Gly Phe Ile Lys Ala Asp Cys
                165                 170                 175

aga caa ata att gtt tcg gca ccg act gcg cat gag ctg ctg aga aag     576
Arg Gln Ile Ile Val Ser Ala Pro Thr Ala His Glu Leu Leu Arg Lys
        180                 185                 190

atg gag caa tac act cgt tca cac cag gag gta gcg cca cgt aca agc     624
Met Glu Gln Tyr Thr Arg Ser His Gln Glu Val Ala Pro Arg Thr Ser
    195                 200                 205

tgg gag atg tca gag ctt ggt tat gga aag aca cca gag gaa tcg taa     672
Trp Glu Met Ser Glu Leu Gly Tyr Gly Lys Thr Pro Glu Glu Ser
210                 215                 220

<210>  26
<211>  223
<212>  PRT
<213>  Oryza sativa

<400>  26

Met Gly Asp Asn Ser Ala Ala Ala Ala Ala Val Ala Ala Pro Arg Gly
1               5                   10                  15

Arg Phe Gly Arg Ile Cys Val Phe Cys Gly Ser Asn Ala Gly Asn Arg
            20                  25                  30

Ala Val Phe Gly Asp Ala Ala Leu Gln Leu Gly Gln Glu Leu Val Ser
        35                  40                  45

Arg Gly Ile Glu Leu Val Tyr Gly Gly Gly Ser Val Gly Leu Met Gly
    50                  55                  60

Leu Ile Ala Gln Thr Val Leu Asp Gly Gly Cys Gly Val Leu Gly Val
65                  70                  75                  80

Ile Pro Lys Ala Leu Met Pro Thr Glu Ile Ser Gly Ala Ser Val Gly
                85                  90                  95

Glu Val Lys Ile Val Ser Asp Met His Glu Arg Lys Ala Glu Met Ala
            100                 105                 110

Arg Gln Ser Asp Ala Phe Ile Ala Leu Pro Gly Gly Tyr Gly Thr Met
        115                 120                 125

Glu Glu Leu Leu Glu Met Ile Thr Trp Ser Gln Leu Gly Ile His Asp
    130                 135                 140

Lys Pro Val Gly Leu Leu Asn Val Asp Gly Tyr Tyr Asp Pro Leu Leu
145                 150                 155                 160

Ala Leu Phe Asp Lys Gly Ala Ala Glu Gly Phe Ile Lys Ala Asp Cys
                165                 170                 175

Arg Gln Ile Ile Val Ser Ala Pro Thr Ala His Glu Leu Leu Arg Lys
            180                 185                 190

Met Glu Gln Tyr Thr Arg Ser His Gln Glu Val Ala Pro Arg Thr Ser
        195                 200                 205

Trp Glu Met Ser Glu Leu Gly Tyr Gly Lys Thr Pro Glu Glu Ser
    210                 215                 220

## Beschreibung

Die vorliegende Erfindung betrifft ein Verfahren zur Herstellung von Aminosäuren in transgenen Organismen.

Weiterhin betrifft die Erfindung Nukleinsäurekonstrukte, Vektoren und transgene Organismen sowie deren Verwendung.

Aminosäuren bilden die grundlegende Struktureinheit sämtlicher Proteine und sind somit für die normale Zellfunktionen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nichtproteinogenen Aminosäuren (von denen Hunderte bekannt sind) gewöhnlich nicht In Proteinen vorkommen [siehe Ullmann's Encyclopedia of Industrial Chemistry, Bd. A2, S. 57-97 VCH: Weinheim (1985)]. Die Aminosäuren können in der D- oder L-Konflguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L. Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, da sie aufgrund der Komplexität ihrer Biosynthese mit der Ernährung aufgenommen werden müssen, werden durch einfache Biosynthesewege in die übrigen 11 "nichtessentiellen" Aminosäuren (Alanin, Arginin, Asparagin, Asparaginsäure, Cystein, Glutaminsäure, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Aminosäuren werden in vielen Industriezweigen verwendet, einschließlich der Nahrungsmittel-, Futtermittel-, Kosmetik-, pharmazeutischen und chemischen Industrie. So wird L-Glutaminsäure beispielsweise in infusionslösungen verwendet. Aminosäuren wie D;L-Methionln, L-Lysin oder L-Threonin werden in der Futtermittelindustrie verwendet. Von besonderer Wichtigkeit für die Ernährung von Menschen und vielen Nutztieren sind die essentiellen Aminosäuren Valin, Leucin, Isoleucin, Lysin, Threonin. Methionin, Tyrosin, Phenylalanin und Tryptophan. So ist Lysin beispielweise nicht nur für die Ernährung des Menschen eine wichtige Aminosäure, sondern auch für monogastrische Tiere, wie Geflügel und Schweine. In Pflanzen, wie Mais oder Weizen, ist L-Lysin die limitierende Aminosäure, das heißt um eine optimale Ausnutzung derartiger Pflanzennahrung zu ermöglichen, ist es sinnvoll die Nahrung oder das Futtermittel mit L-Lysin zu supplementieren. Glutamat wird am häufigsten als Geschmacksadditiv (Mononatriumglutamat, Seq MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze [Leuchtenberger, W. (1996) Amino acids - technical production and use, S. 466-502 in Rehm et al., (Hrsg.) Biotechnology Bd. 6, Kapitel 14a, VCH: Weinheim]. Außerdem eignen sich Aminosäuren für die chemischen Industrie als Vorstufen für die Synthese von synthetischen Aminosäuren und Proteinen, wie N-Acetylcystein, S-Carboxymethyl-L-cystein, (S)-5-Hydroxytryptophan und anderen, in Ullmann's Encyclopedia of Industrial Chemistry, Bd. A2, S. 57-97, VCH, Weinheim. 1985 beschriebenen Substanzen eignen.

Die Jahresproduktion von Aminosäuren beläuft sich derzeit auf Ober 1 Mio. t/a mit einem Marktwert von Ober 2 Mrd. US-$. Ihre Produktion erfolgt heutzutage durch vier konkurrierende Verfahren:
1. Extraktion aus Proteinhydrolysaten beispielsweise von L-Cystin, L-Leucin oder L-Tyrosin,
2. chemische Synthese beispielsweise von D,L-Methionin,
3. Umwandlung chemischer Vorstufen im Enzym- oder Zellreaktor beispielsweise L-Phenylalanin und
4. fermentative Herstellung mittels Anzucht von Bakterien im Großmaßstab, die entwickelt wurden, um große Mengen des jeweils gewünschten Moleküls zu produzieren und sezernieren. Ein für diesen Zweck besonders geeigneter Organismus ist Corynebacterium glutamicum, der beispielsweise für die Herstellung von L-Lysin oder L-Glutaminsäure verwendet wird. Weitere fermentativ hergestellte Aminosäuren sind beispielsweise L-Threonin, L-Tryptophan, L-Aspareginsäure und L-Phenylalanin.

Die Biosynthese der natürlichen Aminosäuren in Organismen, die sie produzieren können, bspw. Bakterien, ist gut charakterisiert worden [für einen Überblick der bakteriellen Aminosäure-Biosynthese und ihrer Regulation, s. Umbarger, H.E. (1978) Ann. Rev. Biochem. 47: 533 - 606]. Glutaminsäure wird durch reduktive Aminierung von α-Ketoglutarat, einem Zwischenprodukt im Citronensäure-Zyklus, synthetisiert. Glutamin, Prolin und Arginin werden jeweils nacheinander aus Glutamat erzeugt. Die Biosynthese von Serin erfolgt in einem Dreischritt-Verfahren und beginnt mit 3-Phosphoglycerat (einem Zwischenprodukt bei der Glykolyse), und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten-β-Kohlenstoffatoms auf Tetrahydrofolat, in einer durch Serintranshydroxymethylase katalysierten Reaktion. Phenylalanin und Tyrosin werden aus den Vorstufen des Glycolyse- und Pentosephosphatweges, Erythrose-4-phosphat und Phosphoenolpyruvat in einem 9-Schritt-Biosyntheseweg synthetisiert, der sich nur in den letzten beiden Schritten nach der Synthese von Prephenat unterscheidet Tryptophan wird ebenfalls aus diesen beiden Ausgangsmolekolen produziert, jedoch erfolgt dessen Synthese in einem 11-Schritt-Weg. Tyrosin lässt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Asparaginsäure wird aus Oxalacetat, einem Zwischenprodukt des Citronensäure-Zyklus, gebildet. Asparagin, Methionin, Threonin und Lysin werden jeweils durch Umwandlung von Asparaginsäure produziert. Isoleucin wird aus Threonin gebildet In einem komplexen 9-Schritt-Weg erfolgt die Bildung von Histidin aus 5-Phosphoribosyl-1-pyrophosphat, einem aktivierten Zucker.

Es ist bekannt, dass Aminosäuren durch Fermentation von Stammen coryneformer Bakterien, insbesondere Corynebacterium glutamicum, hergestellt werden. Wegen der großen Bedeutung wird ständig an der Verbesserung der bestehenden Herstellverfahren gearbeitet. Verfahrensverbesserungen können fermentationstechnische Maßnahmen, wie zum Beispiel Rührung und Versorgung mit Sauerstoff, oder die Zusammensetzung der Nährmedien, wie zum Beispiel die Zuckerkonzentration wahrend der Fermentation, oder die Aufarbeitung zum Produkt, beispielsweise durch Ionenaustauschchromatographie, oder die intrinsischen Leistungseigenschaften des Mikroorganismus selbst betreffen. Auch Bakterien anderer Gattungen wie Escherichia of Bacillus werden für die Herstellung von Aminosäuren verwendet.

Über Stammselektion sind eine Reihe von Mutantenstämmen entwickelt worden, die ein Sortiment wünschenswerter Verbindungen aus der Reihe der schwefelhaltigen Feinchemikalien produzieren. Zur Verbesserung der Leistungseigenschaften dieser Mikroorganismen hinsichtlich der Produktion eines bestimmten Moleküls werden Methoden der Mutagenese, Selektion und Mutantenauswahl angewendet. Dies ist jedoch ein zeitaufwendiges und schwieriges Verfahren. EP-A-0 066 129 beschreibt beispielhaft ein Verfahren zur Herstellung von Threonin mit Corynebakterien. Entsprechende Verfahren wurden auch zur Herstellung von Methionin bearbeitet Auf diese Weise erhält man z.B. Stämme, die resistent gegen Antimetabolite, wie z. B. die Methionin-Analoga α-Methyl-Methionin, Ethionin, Norleucin, N-Acetylnorleucin, S-Trifluoromethylhomocystein, 2-Amino-5-heprenoitsäure, Seleno-Methionin, Methioninsulfoximin, Methoxin, 1-Aminocyclopentan-Carboxylsaure oder auxotroph für regulatorisch bedeutsame Metabolite sind und schwefelhaltige Feinchemikalien, wie z. B. L-Methionin, produzieren. Derartige zur Herstellung von Methionin entwickelte Verfahren haben den Nachteil, dass sie für eine wirtschaftliche Nutzung zu geringe Ausbeuten aufweisen und deshalb gegenüber der chemischen Synthese nicht konkurrenzfähig sind.

Von Zeh et al.(Plant Physiol., Vol. 127, 2001:792-602) wird eine Erhöhung des Methioningehalts in Kartoffelpflanzen durch die Hemmung der Threoninsynthase über die sogenannte Antisense-Technologie beschrieben. Dies führt zu einer verringerten Aktivität der Threoninsynthase, ohne dass der Threoningehalt in den Pflanzen reduziert wird. Von Nachteil ist, dass diese Technologie sehr komplex ist und in einem technischen Maßstab nicht oder nur sehr schlecht verwendet werden kann. Außerdem muss die Hemmung des Enzymaktivität sehr differenziert erfolgen, da sonst eine Auxotrophie für die Aminosäure auftritt und die Pflanze nicht mehr wächst.

Seit einigen Jahren werden ebenfalls Methoden der rekombinanten DNA-Technik zur Stammverbesserung von L-Aminosäure produzierender Stämme von Corynebacterium eingesetzt, indem man einzelne Aminosäure-Biosynthesegene amplifiziert und die Auswirkung auf die Aminosäure-Produktion untersucht.

Aminosäuren, deren Menge den Proteinbiosynthesebedarf der Zelle übersteigt, können nicht gespeichert werden, und werden stattdessen abgebaut, so dass Zwischenprodukte für die Haupt-Stoffwechselwege der Zelle bereitgestellt werden [für einen Überblick siehe Stryer, L., Biochemistry, 3. Aufl. Kap. 21 "Amino Acid Degradation and the Urea Cycle"; S 495-516 (1988)]. Die Zelle ist zwar in der Lage, unerwünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, dass die Aminosäure-Biosynthese durch Feedback-Hemmung reguliert wird, wobei das Vorliegen einer bestimmten Aminosäure ihre eigene Produktion verlangsamt oder ganz beendet [für einen Überblick über den Rückkopplungs-Mechanismus bei Aminosäure-Biosynthesewegen, siehe Stryer, L., Biochemistry, 3. Aufl., Kap. 24, "Biosynthesis of Amino Acids and Heme", S. 575-600 (1988)]. Der Ausstoß einer bestimmten Aminosäure wird daher durch die Menge dieser Aminosäure in der Zelle eingeschränkt.

Verbesserungen der fermentativen Herstellung von Feinchemikalien korrelieren in der Regel mit Verbesserungen von Stoffflüssen und Ausbeuten. Wichtig dabei ist es, Zwischen- oder Endprodukthemmungen wichtiger Syntheseenzyme zu verhindern oder zu verringern. Ebenso ist es von Vorteil, Abflüsse des Kohlenstoffflusses in ungewünschte Produkte oder Seitenprodukte zu verhindern oder zu verringern.

Die essentiellen Aminosäuren sind wie oben beschrieben für den Menschen und viele Säugetiere beispielsweise für Nutztiere notwendig. L-Methionin ist dabei wichtig als Methylgruppen-Lieferant für die Biosynthese z.B. von Cholin, Creatin, Adrenalin, Basen u. RNS u. DNS, Histidin sowie für die Transmethylierung nach Bildung von S-Adenosylmethionin oder als Sulfhydrylgruppen-Lieferant für die Cysten-Bildung.

L-Methionin scheint außerdem einen positiven Einfluss bei Depressionen zu haben

Die Verbesserung der Qualität von Nahrungs- und Futtermitteln ist daher eine wichtige Aufgabe der Nahrungs- und Futtermittelindustrie. Diese ist notwendig, da beispielsweise in Pflanzen Aminosäuren wie L-Lysin und L-Tryptophan für die Versorgung von Säugetieren limitierend sind. Besonders vorteilhaft für die Qualität von Nahrungs- und Futtermittel ist eine möglichst ausgewogene Aminosäurebalance, da ein hoher Überschuss an einer Aminosäure wie beispielsweise L-Lysin ab einer bestimmten Konzentration im Lebensmittel keinen weiteren positiven Effekt auf die Verwertung des Lebensmittels hat, da andere Aminosäuren plötzlich limitierend werden. Eine weitere Steigerung der Qualität ist nur über Zugabe weiterer unter diesen Bedingungen limitierenden Aminosäuren möglich. So ist in wachsenden Schweinen zunächst Lysin limitierend. Ist ausreichend Lysin im Futtermittel enthalten, so wird Threonin zur limitierenden Aminosäure. Wird auch Threonin ausreichend zum Futtermittel gegeben, so ist als nächste Aminosäure Tryptophan limitiert Für Hühner ist die Reihenfolge der ersten drei limitierenden Aminosäuren wie folgt Methionin, Lysin und dann Threonin. Dies zeigt, dass diese Aminosäuren für eine optimale Ernährung wichtige Funktion haben und in einem ausgewogenen Verhältnis in der Nahrung vorliegen müssen.

Eine gezielte Gabe der limitierenden Aminosäure in Form synthetischer Produkte muss deshalb sehr vorsichtig vorgenommen werden, um Aminosäure-Imbalanzen zu vermeiden. Durch Zugabe einer essentiellen Aminosäure wird nämlich die Proteinverdauung angeregt, was besonders Mangelsituationen bei der in zweiter oder dritter Stelle limitierenden Aminosäure hervorrufen kann.

So hat man bei Fütterungsversuchen beispielsweise von Casein durch zusätzliche Gaben von Methionin, das im Casein limitiert ist, Leberverfettungen festgestellt, die erst nach zusätzlicher Gabe von Tryptophan behoben werden konnten.

Für eine hohe Nahrungs- und Futtermittelqualität ist deshalb die je nach Organismus ausgewogene Zugabe mehrerer Aminosäuren notwendig. Die vorgenannten fermentativen sowie anderen Syntheseverfahren ermöglichen in der Regel nur den Zugang zu einer einzelnen Aminosäure.

Es bestand daher die Aufgabe für die vorliegende Erfindung ein kostengünstiges Verfahren zur Synthese von Aminosäuren vorteilhaft der essentiellen Aminosäuren L-Lysin und L-Methionin bevorzugt von L-Methionin zu entwickeln, die zu den zwei häufigsten limitierenden Aminosäuren gehören.

Diese Aufgabe wurde durch das erfindungsgemäße Verfahren zur Herstellung von Aminosäuren vorteilhaft von L-Methionin in transgenen Organismen gelöst, wobei das Verfahren dadurch gekennzeichnet ist, dass das Verfahren folgende Schritte umfasst:
a) Einbringen einer Nukleinsäuresequenz, die für ein Threonin-abbauendes Protein und/oder Lysin-abbauendes Protein codiert, oder
b) Einbringen einer Nukleinsäuresequenz, die den Threoninabbau und/oder Lysinabbau in den transgenen Organismen erhöht und
c) Expression einer unter (a) oder (b) genannten Nukleinsäuresequenz im transgenen Organismus.

Unter Threonin-abbauenden Proteinen sind vorteilhaft Proteine zu verstehen wie die Threonin-Aldolase (EC 4.1.2.5) oder die Serin-Hydroxymethyltransferase (EC 2.1.2.1), die Threonin zu Acetaldehyd und Glycin umsetzen, die Threonindehydrogenase, die unter Bildung von NADH + H⁺ Threonin zu L-2-Amino-Acetoacetat umsetzt, oder die Threonindehydratase, die Threonin unter NH₃ und Wasserabspaltung zu Oxobutyrate umsetzt. Vorteilhaft wird als Throninabbauende Aktivität die Threonin-Aldolase im erfindungsgemäßen Verfahren verwendet Die Aktivität der vorgenannten Proteine und/oder der für sie codierenden Nukleinsäuresequenzen kann auf verschiedenen Wegen erhöht werden. Vorteilhaft werden die Nukleinsäuresequenzen in einem Organismus exprimiert und so die Aktivität in einem Organismus über die Genkopienzahl erhöht und/oder aber es wird die Stabilität der exprimierten mRNA erhöht und/oder es wird die Stabilität des Genprodukts erhöht Weiterhin kann die Regulation der vorgenannten Nukleinsauresequenzen verändert werden, so dass die Expression der Gene erhöht wird. Dies kann vorteilhaft durch heterologe Regulationssequenzen oder durch Veränderung z.B. über Mutation der vorhandenen natürlichen Regulationssequenzen erreicht werden. Beide vorteilhaften Methoden können auch miteinander kombiniert werden.

Eine vorteilhafte Ausführungsform des erfindungsgemäßen Verfahrens ist ein Verfahren zur Herstellung von Aminosäuren vorteilhaft von L-Methionin in transgenen Organismen dadurch gekennzeichnet, dass das Verfahren folgende Schritte umfasst:
a) Einbringen einer Nukleinsäuresequenz, die für ein Threonin-abbauendes Protein codiert, das folgende Consensus-Sequenz enthält
   H[-x]₂G[X]R[X]₁₉D[X]K[X]₂₇G, oder
   HXDGAR[X]₃A[X]₁₅D[X]₄CXSK[X]₄PXGS[X]₃G[X]₇A[X]₄K[X]₂GGGXRQXG
b) Einbringen einer Nukleinsäuresequenz, die den Threoninabbau In den transgenen Organismen erhöht und
c) Expression einer unter (a) oder (b) genannten Nukleinsäuresequenz im transgenen Organismus.

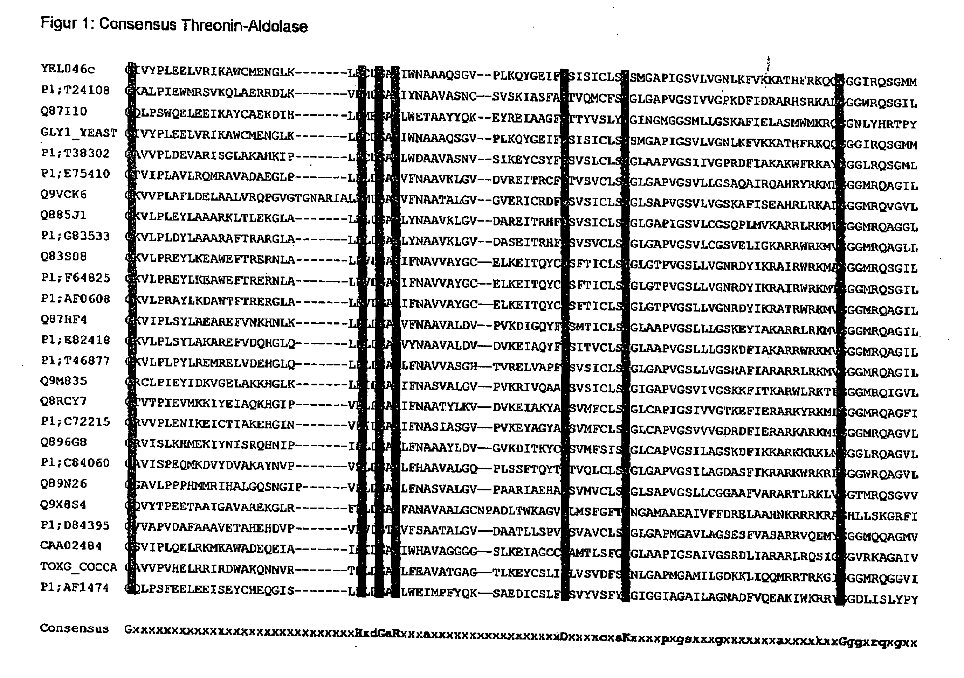

Wobei in der Consensus-Sequenz der ein Buchstaben Aminosäurecode verwendet wurde. An Stellen an denen ein X steht, kann eine beliebige Aminosäure stehen. Figur 1 gibt den Consensus der Threonin-Aldolase, die im erfindungsgemäßen Verfahren vorteilhaft verwendet werden können, wieder. Die in Figur 1 genannten Abkürzungen der Proteine bzw. deren Accession-Nummern bedeuten das folgende: P1:T24108 ist ein hypothetisches Protein R102.4b aus Caenorhabditis elegans, Q87I10 ist eine putative L-allo-Threonin Aldolase aus Vibrio parahaemolyticus, GLY1_YEAST ist eine low-specificity L-Threonin Aldolase aus Saccharomyces cerevisiae (Baker's yeast), P1;T38302 ist eine mögliche Threonin Aldolase aus Schizosaccharomyces pombe, P1;E75410 ist eine L-allo-Threonin Aldolase aus Deinococcus radiodurans. Q9VCK6 wird als CG10184 Protein bezeichnet und stammt aus Drosophila melanogaster (Fruit fly), Q885J1 ist eine Threonin Aldolase mit geringer Spezifität aus Pseudomonas syringae. P1;G83533 ist ein hypothetisches Protein PA0902 aus Pseudomonas aeruginosa, Q83S08 ist eine putative Arylsulfatase aus Shigella flexneri, P1;F64825 ist eine L-allo-Threonin Aldolase aus Escherichia coli, ebenso wie P1;AF0608, das aus Salmonella enterica stammi. Q87HF4 ist eine L-allo-Threonin Aldolase aus Vibrio parahaemolyticus. Auch bei den folgenden Proteinen handelt es sich um L-allo-Threonin Aldolasen: P1;E82418 (Vibrio cholerae), P1;T46877 (Aeromonas jandaei), O9M835 (Arabidopsis thaliana; Mouse-ear cress), Q8RCY7 (Thermoanaerobacter tengcongensis), P1;C72215 (Thermotoga maritima), Q896G8 (Clostridium tetani), P1;C84080 (Bacillus halodurans. Q89N26 ist ein Bil4016 Protein aus Bradyrhizobium japonicum.Q9X8S4 ist eine L-Threonin Aldolase mit geringer Spezifität aus Zymomonas mobilis, P1;D84395 ist eine L-allo-Threonin Aldolase aus Halobacterium sp. NRC-1. Bei CAA02484 handelt es sich um Sequenz 33 aus der PCT-Anmeldung WO 94/25606. Die Sequenz stammt aus Tolypocladium inflatum. TOXG_COCCA ist eine Alanin Racemase TOXG aus Cochliobolus carbonum (Bipolaris zeicola), P1;AF1474 stammt aus Listeria innocua und ist eine L-allo-Threonin Aldolase mit geringer Spezifität.

Unter Lysin-abbauenden Proteinen sind vorteilhaft Proteine zu verstehen wie die Lysin-Decarboxylase (EC 4.1.1.18), die L-lysine 6-monooxygenase (EC 1.14.13.59), die L-Lysine 2-monooxygenase (EC 1.13.12.2), Lysin-Ketoglutaratreductase (EC 1.5.1.7) oder die Lysine 2,3-aminomutase (EC 5.4.3.2), die L-Lysin zu Cadaverin, N6-hydroxy-L-lysine, 5-Aminopentanamid, Saccharopin oder (3S)-3,6-Aminohexanoat umsetzen, Vorteilhaft wird als Lysin-abbauende Aktivität die Lysin-Decarboxylase allein oder in Kombination mit einer Threonin-abbauenden Aktivität vorteilhaft der Threonin-Aldolase im erfindungsgemäßen Verfahren verwendet Die Aktivität der vorgenannten Proteine und/oder der für sie codierenden Nukleinsäuresequenzen kann auf verschiedenen Wegen erhöht werden. Vorteilhaft werden die Nukleinsäuresequenzen in einem Organismus exprimiert und so die Aktivität in einem Organismus über die Genkopienzahl erhöht und/oder aber es wird die Stabilität der exprimierten mRNA erhöht und/oder es wird die Stabilität des Genprodukts erhöht. Weiterhin kann die Regulation der vorgenannten Nukleinsäuresequenzen verändert werden, so dass die Expression der Gene zen verändert werden, so dass die Expression der Gene erhöht wird. Dies kann vorteilhaft durch heterologe Regulationssequenzen oder durch Veränderung z.B. über Mutation der vorhandenen natürlichen Regulationssequenzen erreicht werden. Beide vorteilhaften Methoden können auch miteinander kombiniert werden.

Eine vorteilhafte Ausführungsform des erfindungsgemäßen Verfahrens ist ein Verfahren zur Herstellung von Aminosäuren vorteilhaft von L-Methionin in transgenen Organismen dadurch gekennzeichnet, dass das Verfahren folgende Schritte umfasst, gelöst
a) Einbringen einer Nukleinsäuresequenz, die für ein Lysin-abbauendes Protein codiert, das folgende Consensus-Sequenz enthält
   G[X]₄GIM[X]₄₅**M[X]₂RK[X]₂M[X]₁₁GGXG[X]₃E[X]₂E[X]₃**W, oder
   LG[X]₉ LVYGG[X]₃GIMGXVA[X]₉G[X]₃GXIP[X]₂₄**MHXRK[X]₂M[X]₅F[X]₃PGGXGTXEE[X]₂ E**[X]₂TW[X]₂IG[X]₃KP[X]₄N[X]₃FY[X]₁₄F
b) Einbringen einer Nukleinsäuresequenz, die den Lysinabbau in den transgenen Organismen erhöht und
c) Expression einer unter (a) oder (b) genannten Nukleinsäuresequenz im transgenen Organismus.

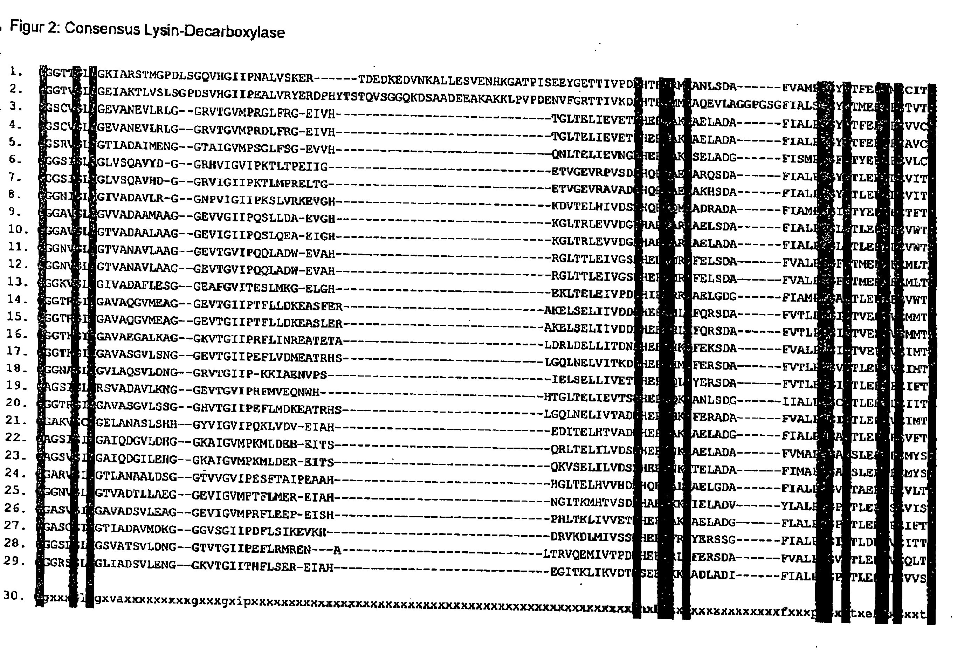

Wobei in der Consensus-Sequenz der ein Buchstaben Aminosäurecode verwendet wurde. An Stellen an denen ein X steht, kann eine beliebige Aminosäure stehen. Figur 2 gibt den Consensus der Lysin-abbauendes Protein , die im erfindungsgemäßen Verfahren vorteilhaft verwendet werden können, wieder. Die in Figur 2 aufgeführten Aminosäuresequenzen sind nummeriert (1., 2., 3., usw) und bedeuten folgendeAbkürzungen der Proteine bzw. deren Accession-Nummem: Q871Q6 [2] ist ein hypothetisches Protein aus Neurospa crassa. Bei Q815T3 [3] handelt es sich um eine Lysin-Decarboxylase aus Bacillus cereus. Q81XE4 [4] codiert für ein hypothetisches Protein aus Bacillus anthracis, ebenso wie P1;D70033 [5] für ein hypothetisches Protein aus Bacillus subtilis codiert Q8H7U8 [6] ist eine putative Lysin-Decarboxylase aus Oryza sativa. Q8L8B8 [7] codiert für ein hypothetisches Protein aus Arabidopsis thaliana. Auch bei Q8XXM6 [8] handelts es sich um ein hypothetisches Protein aus Ralstonia solanacearum. Auch bei den folgenden Proteinen handelt es sich um hypothetische Proteine: Q88DF4 [9] (Pseudomonas putida), P1;A83031 [10] (Pseudomonas aeruginosa),Q8PAJ9 [11] (Xanthomonas campestris) und Q8PMA0 [12] (Xanthomonas axonopodis). Q8NN34 [13] codiert für ein vorhergesagtes Rossmann Fold Nucleotide Binding-Protein (1 segment) aus Corynebacterium glutamicum. P1;Al3438 [14] codiert für eine Lysin-Decarboxylase aus Brucella melitensis, Q8G289 [15] codiert für ein hypothetisches Protein aus Brucella suis, ebenso wie Q984W8 [16] (Rhizobium loti). P1;B97490 [17] ist eine Lysin-Decarboxylase aus Agrobacterium tumefaciens. Auch P1;B83993 [18] codiert für eine Lysin-Decarboxylase aus Bacillus halodurans Von Q8A2T1 [19] wird angenommen, dass es sich um eine putative Lysin-Decarboxylase aus Bacteroides thetaiotaomicron handelt. Die folgenden Proteine codieren für hypothetische Proteien: Q92R13 [20] (Rhizobium meliloti), Q8ETC2 [21] (Oceanobacillus iheyensis), Q8NXQ6 [22] (Staphylococcus aureus), Q8CTK0 [23] (Staphylococcus epidermidis) und P1;F55578 [24] (Rhadococous fascians). Q839D0 [25] codiert für ein Protein der Decarboxylase-Familie aus Enterococcus faecalis. P1;D84035 [26] ist ein hypothetisches Protein aus Bacillus halodurans. Q8EZ03 [27] ist eine Lysin-Decarboxylase aus Leptospira interrogans. Q89NP4 [28] Ist ein hypothetisches Protein aus Bradyrhizobium japonicum ebenso wie Q8RFZ1 [29] (Fusobacterium nucleotum). Die in den eckigen Klammern wiedergegebenen Nummern geben die in Figur 2 gezeigte Nummerierung und damit die Reihenfolge der Proteine an. Der Klon YJL055w, der vorteilhaft im erfindungsgemäßen Verfahren verwendet wird, trägt die Nummer 1. Die Consensussequenz ist in Nummer 30 wiedergegeben.

In einer weiteren Ausführungsform des Verfahrens handelt es sich um ein Verfahren zur Herstellung von Aminosäuren vorteilhaft von L-Methionin in transgenen Organismen, dadurch gekennzeichnet, dass das Verfahren folgende Schritte umfasst:
a) Einbringen einer Nukleinsäuresequenz, die für ein Threonin-abbauendes Protein codiert, das folgende Consensus-Sequenz enthält
   H[x]₂G[X]R[X]₁₉D[X]₇K[X]₂₇G, oder
   HXDGAR[X]₃A[X]₁₅D[X]₄CXSK[X]₄PXGS[X]₃G[X]₇A[X]₄K[X]₂GGGXRQXG
   und Einbringen einer Nukleinsäuresequenz, die für ein Lysin-abbauendes Protein codiert, das folgende Consensus-Sequenz enthält
   G[X]₄GIM[X]₄₅**M[X]₂RK[X]₂M[X]₁₁,GGXG[X]₃E[X]₂E**[X]₃W, oder
   LG[X]₉ LVYGG[X]₃GIMGXVA[X]₉G[X]₃GXIP[X]₂₄**MHXRK[X]₂M[X]₆F[X]₃PGGXGTXEE[X]₂** E[X]₂TW[X]₂IG[X]₃KP[X]₄N[X]₃FY[X]₁₄F, oder
b) Einbringen einer Nukleinsäuresequenz, die für Proteine codiert, die den Threoninabbau und Lysinabbau in den transgenen Organismen erhöhen, und
c) Expression einer unter (a) oder (b) genannten Nukleinsäuresequenz im transgenen Organismus.

In einer vorteilhaften Ausführungsform des Verfahrens zur Herstellung von Aminosäuren Vorteilhaft von L-Methionin in transgenen Organismen ist das Verfahren dadurch gekennzeichnet, dass im oben genannten Verfahrensschritt (a) eine Nukleinsäuresequenz, eingebracht wird, die ausgewählt ist aus der Gruppe der Nukleinsäuresequenzen
i) einer Nukleinsäuresequenz mit der in SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 dargestellten Sequenz;
ii) einer Nukleinsäuresequenz, die aufgrund des degenerierten genetischen Codes durch Rückübersetzung der in SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 dargestellten Aminosäuresequenz erhalten wird und
iii) eines Derivats der in SEQ ID NO: 1, SEQ ID NO: 11, SEQ ID NO:13, SEQ ID NO: 15, SEQ ID NO: 17, SEQ ID NO: 19, SEQ ID NO: 21, SEQ ID NO: 23 oder SEQ ID NO: 25 dargestellten Nukleinsäuresequenz, die für ein Polypeptid codiert, welches mindestens 50 % Homologie auf Aminosäureebene zu der in SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 dargestellten Aminosäuresequenz aufweist, ohne dass die biologische Aktivität der Polypeptide wesentlich reduziert ist; und
diese Nukleinsäuresequenzen anschließend in einem transgenen Organismus exprimiert werden.

Eine weitere vorteilhafte Ausführungsform des Verfahrens zur Herstellung von Aminosäuren vorteilhaft von L-Methionin in transgenen Organismen ist das Verfahren dadurch gekennzeichnet, dass im oben genannten Verfahrensschritt (a) oder (b) Nukleinsäuresequenzen in Kombination miteinander oder in Kombination mit anderen Nukleinsäuresequenzen, die die Synthese von L-Lysin und/oder L-Threonin in einem transgenen Organismus steigern können. Hierzu gehören neben Genen, die für den Zentralstoffwechsel wie der Verwertung von Zuckern wie Glucose innerhalb der Glykolyse oder des Citratzykluses codieren, auch Gene, die ausgehend von Aspartat an der Synthese der Aminosäuren beteiligt sind.

Diese vorteilhaften Ausführungsformen des Verfahrens zur Herstellung von Aminosäuren vorteilhaft von L-Methionin in transgenen Organismen stellt sich damit wie folgt dar:
a) Einbringen einer Nukleinsäuresequenz ausgewählt aus der Gruppe
   i) einer Nukleinsäuresequenz mit der in SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 und/oder SEQ ID NO: 25 dargestellten Sequenz;
   ii) einer Nukleinsäuresequenz, die aufgrund des degenerierten genetischen Codes durch Rückübersetzung der in SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24 und/oder SEQ ID NO: 26 dargestellten Aminosäuresequenz erhalten wird und
   iii) eines Derivats der in SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 und/oder SEQ ID NO: 25 dargestellten Nukleinsäuresequenz, die für ein Polypeptid codiert, welches mindestens 50 % Homologie auf Aminosäureebene zu der in SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24 und/oder SEQ ID NO: 26 dargestellten Aminosäuresequenz aufweist, ohne dass die biologische Aktivität der Polypeptide wesentlich reduziert ist; und
b) Expression einer unter (a) genannten Nukleinsäuresequenz in einem transgenen Organismus.

In einer weiteren vorteilhaften Ausführungsform des Verfahrens zur Herstellung von Aminosäuren vorteilhaft von L-Methionin in transgenen Organismen ist das Verfahren dadurch gekennzeichnet, dass im oben genannten Verfahrensschritt (a) eine oder mehrere Nukleinsäuresequenz(en) eingebracht wird/werden, die ausgewählt sind aus der Gruppe der Nukleinsäuresequenzen
i) einer Nukleinsäuresequenz, die sich aufgrund des degenerierten genetischen Codes durch Rückübersetzung der in SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO: 10 dargestellten Aminosäuresequenz erhalten wird;
ii) eines Derivats der Nukleinsäuresequenz, die durch Rückübersetzung der in SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO:10 dargestellten Aminosäuresequenz erhalten wird und welche mindestens 70 % Homologie auf Aminosäureebene zu den vorgenannten Aminosäuresequenzen aufweist, ohne dass die biologische Aktivität der Polypeptide wesentlich reduziert ist; eingebracht wird und
anschließend diese Nukleinsäuresequenzen in einem transgenen Organismus exprimiert werden.

Nach dem Einbringen und der Expression der in den erfindungsgemäßen Verfahren verwendeten Nukleinsäuresequenzen wird der transgene Organismus vorteilhaft kultiviert und anschließend geerntet. Im Falle, dass es sich bei dem transgenen Organismus um einen Mikroorganismus wie einem eukaryotischen Organismus wie einem Pilz, einer Alge oder einer Hefe oder einem prokaryotischen Organismus wie einem Bakterium wie einem Bakterium der Gattungen Escherichia, Bacillus, Serratia, Salmonella, Klebsiella, Enterobacter, Corynebacterium oder Brevibacterium handelt wird dieser in einem dem Fachmann bekannten und je nach Organismus üblichen Fest- oder Flüssigmedium kultiviert Nach Anzucht werden die Organismen gegebenenfalls geerntet. Die Aminosäuren können dann direkt in Nahrungsmittel oder Futtermittel oder für sonstige Anwendungen beispielsweise gemäß den in EP-B-0 533 039 oder EP-A-0 615 693 gemachten Offenbarungen, auf die hier ausdrücklich verwiesen wird, weiter verarbeitet werden oder aber weiter in üblicherweise über Extraktion und Fällung oder Kristallisation oder über Ionenaustauscher oder Kombinationen dieser Methoden weiter aufgereinigt werden. Produkte dieser unterschiedlichen Aufarbeitungen sind Aminosäuren oder Aminosäurezusammensetzungen, die noch Anteile der Fermentationsbrühe und der Zellen in unterschiedlichen Mengen vorteilhaft im Bereich von 0 bis 100 Gew.-%, bevorzugt von 1 bis 80 % Gew.-%, besonders bevorzugt zwischen 5 und 50 Gew.-%, ganz besonders bevorzugt zwischen 5 und 40 Gew.-% enthalten.

In einer vorteilhaften Ausführungsform der Erfindung handelt es sich bei dem Organismus um eine Pflanze, deren Aminosäuregehalt durch die eingebrachte Nukleinsäuresequenz vorteilhaft verändert wird. Dies ist für Pflanzenzüchter wichtig, da beispielsweise für monogastrische Tiere der Nährwert von Pflanzen durch einige essentielle Aminosäuren wie Lysin oder Methionin limitiert ist. Auch Threonin spielt in diesem Zusammenhang eine wichtige Rolle. Diese so hergestellte transgene Pflanze wird nach dem Einbringen der im erfindungsgemäßen Verfahren vewendeten Nukleinsäure bzw. Nukleinsäurekombination auf oder in einem Nährmedium oder aber in Festkultur z.B. einer Erdkultur angezogen und anschließend geerntet. Die Pflanzen können dann direkt als Lebens- oder Futtermittel verwendet werden oder aber weiter verarbeitet werden. Auch in diesem Fall können die Aminosäuren in üblicher Weise über Extraktion, Kristallisation und/oder Fällung oder über Ionenaustauscher oder Kombinationen dieser Methoden weiter aufgereinigt werden. Produkte dieser unterschiedlichen Aufarbeitungen sind Aminosäuren oder Aminosäurezusammensetzungen, die noch Anteile der Pflanze in unterschiedlichen Mengen vorteilhaft im Bereich von 0 bis 100 Gew.-%, bevorzugt von 20 bis 80 % Gew.-%, besonders bevorzugt zwischen 50 und 90 Gew.-%, ganz besonders bevorzugt zwischen 80 und 99 Gew.-% enthalten. Vorteilhaft werden die Pflanzen direkt ohne weitere Aufarbeitung verwendet.

In einer weiteren Ausführungsform der Erfindung handelt es sich bei dem Organismus um einen Mikroorganismus wie Bakterien der Gattungen Corynebacterium, Brevibacterium, Escherichia, Serratia, Salmonella, Klebsiella, Enterobacter, oder Bacillus. Bevorzugt werden Mikroorganismen der Gattungen Corynebacterium, Brevibacterium, Escherichia oder Bacillus verwendet. Diese Mikroorganismen werden vorteilhaft in einem fermentativen Verfahren verwendet Vorteilhaft werden in den Organismen neben der In SEQ ID NO: 1, SEQ ID NO: 11, SEQ ID NO: 13, SEQ ID NO: 15, SEQ ID NO: 17, SEQ ID NO: 19, SEQ ID NO: 21, SEC ID NO: 23 und/oder SEQ ID NO: 25 genannten Sequenz, den Nukleinsäuresequenzen, die sich von den Sequenzen SEC ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO: 10 ableiten lassen, oder deren Derivaten noch weitere Gene exprimiert und/oder mutiert. Besonders vorteilhaft wird in den Organismen wie Pflanzen oder Mikroorganismen zusätzlich wenigstens ein weiteres Gen des Biosyntheseweges des L-Lysins, L-Threonins und/oder L-Methionins exprimiert und/oder es werden Gene exprimiert, deren Regulation verändert wurden. Vorteilhaft kann auch die Regulation der natürlichen Gene verändert worden sein, so dass das Gen und/oder dessen Genprodukt nicht mehr der den in Organismen vorhandenen Regelkreisläufen unterliegt. Dadurch kommt es zu einer verstärkten Synthese der gewünschten Aminosäuren, da z.B. Feedback-Regulationen nicht mehr oder nicht mehr in dem Maße vorhanden sind. Vorteilhaft werden im erfindungsgemäßen Verfahren Aminosäuren wie L-Lysin, L-Threonin und/oder L-Methionin, bevorzugt L-Methionin produziert

Gemäß einer weiteren Ausführungsform des erfindungsgemäßen Verfahrens werden deshalb Organismen vorteilhaft Bakterien der Gattungen Corynebacterium, Brevibacterium. Bacillus oder Escherichia oder Pflanzen angezogen, in denen gleichzeitig wenigstens eine Nukleinsäure bzw. eines der Gene, die für Proteine kodieren, ausgewählt aus der Gruppe der Genprodukte bestehend aus der Aspartatkinase (lysC), der Aspartat-Semialdehyd-Dehydrogenase (asd), der Dihydrodipicolinat Synthase, der Dihydrodipicolinat Reductase, der Tetrahydrodipiconat Succinyltransferase, der N-Succinyl-L-Diaminopimelinsäure-Glutaminsäure Transaminase, der Succinyl-diaminopimelat Desuccinylase, der Diaminopimelat Epimerase, der Diaminopimelat Decarboxylase, der Glycerinaldehyd-3-Phosphat Dehydrogenase (gap), der 3-Phosphoglycerat Kinase (pgk), der Pyruvat Carboxylase (pyc), der Triosephosphat Isomerase (tpl), der Homoserin O-Acetyltransferase (metA), der Cystahionin-γ-Synthase (metB), der Cystahionin-gamma-Lyase (metC), Cystahionin-β-Lyase, der Methionin-Synthase (metH), der Serin-Hydroxymethyltransferase (glyA), der O-Acetylhomoserin-Sulfhydrylase (metY), der Methylen-Tetrahydrofolat-Reduktase (metF), der Phosphoserin-Aminotransferase (serC), der Phosphoserin-Phosphatase (serB), der Serine Acetyl-Transferase (cysE), der Cystein-Synthase (cysK), der Homoserin-Dehydrogenase (horn), Homoserin-Kinase, Homocysteine S-Methyltransferase und S-Adenosylmethionin Synthase (metX) überexprimiert ist

In einer weiteren vorteilhaften Ausführungsform des erfindungsgemäßen Verfahrens werden im Verfahren Organismen verwendet, in denen gleichzeitig wenigstens eines der vorgenannten Gene bzw. eine der vorgenannten Nukleinsäuren mutiert ist, so dass die korrespondierenden Proteine, verglichen mit den nicht mutierten Proteinen, in geringerem Maße oder gar nicht durch Stoffwechselmetabolite in ihrer Aktivität beeinflusst werden und dass insbesondere die erfindungsgemäße Produktion der Aminosäuren nicht beeinträchtigt wird, oder so dass ihre spezifische enzymatische Aktivität gesteigert wird. Unter geringer Beeinflussung ist dabei eine gegenüber dem Ausgangsorganismus um mindestens 10 %, vorteilhaft mindestens 20, 30 oder 40 %, besonders vorteilhaft um mindestens 50, 60 oder 70 % geringere Regulierung der Enzymaktivitat, das heißt Beeinflussung ihrer enzymatischen Aktivität durch Stoftwechselmetabolite und damit um diese genanten Zahlen erhöhte Aktivität des Enzyms gegenüber dem Ausgangsorganismus zu verstehen. Unter Steigerung der enzymatischen Aktivität ist eine gegenüber dem Ausgangsorganismus um mindestens 10 %, vorteilhaft mindestens 20, 30 oder 40 %, besonders vorteilhaft um mindestens 50, 60 oder 70 % gesteigerte enzymatische Aktivität zu verstehen. Dies führt zu einer erhöhten Produktivität der gewünschten Aminosäure oder der gewünschten Aminosäuren.

In einer weiteren vorteilhaften Ausführungsform des erfindungsgemäßen Verfahrens werden im Verfahren Organismen verwendet, in denen gleichzeitig wenigstens eines der Gene, die für eine enzymatische Aktivität codiert ausgewählt unter der Homoserine-Kinase (thrB), der Threonin Dehydratase (ilvA), der Threonin Synthese (thrC), der Meso-Diaminopimelat D-Dehydrogenase (ddh), der Phosphoenolpyruvat-Carboxykinase (pck), der Glucose-6-Phosphat-6-isomerase (pgi), der Pyruvat-Oxidase (poxB), der Dihydrodipicolinat Synthase (dapA), der Dihydrodipicolinat Reduktase (dapB) und der Diaminopicolinat Decarboxylase (lysA) abschwächt ist, insbesondere durch Verringerung der Expressionsrate des korrespondierenden Gens.

Gemäß einer anderen Ausführungsform des erfindungsgemäßen Verfahrens werden im Verfahren Organismen verwendet, in denen gleichzeitig wenigstens eine der vorgenannten Nukleinsäuren bzw. der vorgenannten Gene so mutiert ist, dass die enzymatische Aktivität des korespondierenden Proteins teilweise verringert wird oder vollständig blockiert ist. Unter Verringerung der enzymatischen Aktivität ist eine gegenüber dem Ausgangsorganismus um mindestens 10 %, vorteilhaft mindestens 20, 30 oder 40 %, besonders vorteilhaft um mindestens 50, 60 oder 70 % verringerte enzymatische Aktivität zu verstehen.

Enzyme können derart in ihrer Aktivität beeinflusst werden, dass es zu einer Verringerung oder Erhöhung der Reaktionsgeschwindigkeit, oder zu einer Veränderung (Verringerung oder Erhöhung) der Affinität gegenüber dem Substrat kommt.

Vorzugsweise werden in dem erfindungsgemäßen Verfahren Mikroorganismen der Gattungen Corynebacterium oder Brevibacterium oder Pflanzen eingesetzt

Auch chemisch reine Aminosäuren oder Aminosäurezusammensetzungen sind nach den vorbeschriebenen Verfahren darstellbar. Dazu werden die Aminosäuren oder die Aminosäurezusammensetzungen aus dem Organismus wie den Mikroorganismen oder den Pflanzen oder dem Kulturmedium, in dem oder auf dem die Organismen angezogen wurden, oder aus dem Organismus und dem Kulturmedium in bekannter Weise isoliert. Diese chemisch reinen Aminosäuren oder Aminosäurezusammensetzungen sind für Anwendungen im Bereich der Lebensmittelindustrie, der Kosmetikindustrie oder der Pharmaindustrie vorteilhaft.

Vorteilhaft werden nach dem erfindungsgemäßen Verfahren Aminosäuren wie Methionin, Lysin oder deren Mischungen bevorzugt Methionin hergestellt.

Dabei können im erfindungsgemäßen Verfahren die vorgenannten Aminosäuren mindestens um den Faktor 3, bevorzugt mindestens um den Faktor 5, besonders bevorzugt mindestens um den Faktor 10, ganz besonders bevorzugt um mindestens den Faktor 50 gegenüber dem Wildtyp der Organismen erhöht werden. Besonders vorteilhaft wirkt es sich im erfindungsgemäßen Verfahren auf die Aminosäureproduktivität aus, wenn eine Kombination von Genen verwendet wird, die für eine Threonin-Aldolase oder Threonin-Aldolase ähnliches Protein oder eine Lysin-Decarboxylase oder ein Lysin-Decarboxylase ähnliches Protein codieren.

Durch das erfindungsgemäße Verfahren können die hergestellten Aminosäuren in den im Verfahren verwendeten Organismen prinzipiell auf zwei Arten erhöht werden. Es kann vorteilhaft der Pool an freien Aminosäuren und/oder der Anteil der über das Verfahren hergestellten Aminosäuren in den Proteinen erhöht werden. Vorteilhaft wird durch das erfindungsgemäße Verfahren der Pool an freien Aminosäuren in den transgenen Organismen erhöht. Im vorteilhaften Falle der Fermentation von Mikroorganismen werden die Aminosäuren im Medium angereichert.

Prinzipiell sind für das erfindungsgemäße Verfahren alle eukaryotischen oder prokaryotischen Organismen geeignet, die in der Lage sind, Methionin und/oder Lysin zu synthetisieren. Vorteilhaft handelt es sich bei den im Verfahren verwendeten Organismen um Mikroorganismen wie Bakterien, Pilze, Hefen oder Algen oder Pflanzen wie dicotyledone oder monocotyledone Pflanzen wie Pflanzen der Familien Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Caprifolaceae, Rubiaceae, Scrophulariaceae, Caryophyllaceae, Ericaceae, Polyganaceae, Violaceae, Juncaceae oder Poaceae bevorzugt einer Pflanze ausgewählt aus den Gruppe der Familien Apiaceae, Asteraceae, Brassicaceae, Cucurbitaceae, Fabaceae, Papaverareae, Rosaceae, Solanaceae, Liliaceae oder Poaceae.

Vorteilhaft werden im erfindungsgemäßen Verfahren transgene Mikroorganismen wie Pilze wie die Gattung Claviceps oder Aspergillus oder gram-positive Bakterien wie die Gattungen Bacillus, Corynebacterium, Micrococcus, Brevibacterium, Rhodococcus, Nocardia, Caseobacter oder Arthrobacter oder gram-negative Bakterien wie die Gattungen Escherichia, Flavobacterium oder Salmonella oder Hefen wie die Gattungen Rhodotorula, Hansenula oder Candida. Besonders vorteilhaft sind Organismen ausgewählt aus der Gruppe der Gattungen Corynebacterium, Brevibacterium, Escherichia, Bacillus, Serratia, Salmonella, Klebsiella, Enterobacter, Rhodotorula, Hansenula, Candida, Claviceps oder Flavobacterium. Gans besonders vorteilhaft werden im erfindungsgemäßen Verfahren Mikroorganismen ausgewählt aus der Gruppe der Gattungen und Arten bestehend aus Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. oder Escherichia coli speziell Escherichia coli K12 und dessen beschriebene Stämme verwendet.

Vorteilhaft werden im erfindungsgemäßen Verfahren transgene Pflanze ausgewählt aus der Gruppe der Nutzpflanzen verwendet. Wie Pflanzen ausgewählt aus der Gruppe der Erdnuss, Raps, Canola, Sonnenblume, Safflor (Färberdistel), Olive, Sesam, Haselnuss, Mandel, Avocado, Lorbeer, Kürbis, Lein, Soja, Pistazlen, Borretsch, Mais, Weizen, Roggen, Hafer, Hirse, Triticale, Reis, Gerste, Maniok, Kartoffel, Zuckerrübe, Futterrübe, Aubergine, sowie ausdauernde Gräser und Futterfeldfrüchte, Ölpalme. Gemüse (Kohlgemüse, Wurzelgemüse, Knollengernüse, Hülsengernüse. Fruchtgemüse. Zwiebelgemüse, Blatt- und Stielgemüse). Buchweizen, Topinambur, Ackerbohne, Wicken, Linse, Buschbohne, Alfaalfa, Lupine, Klee und Luzeme.

Die im Verfahren zur Herstellung von Aminosäuren in transgenen Organismen verwendete(n) Nukleinsäuresequenz(en) stammen vorteilhaft aus einem Eukaryont (für die Erfindung soll der Plural den Singular und umgekehrt umfassen), können aber auch aus einem Prokaryont wie Bakterien ausgewählt aus den Gattungen Brevibacterium, Escherichia, Salmonella, Bacillus, Corynebacterium, Serratia, Klebsiella oder Enterobacter stammen. Vorteilhaft stammen die Nukleinsäuresequenzen aus einer Pflanze wie einer Pflanze ausgewählt aus den Familien Aceraceae, Anacardiaceae, Apiaceae, Asteraceae, Brassicaceae, Cactaceae, Cucurbitaceae. Euphorbiaceae, Fabaceae, Malvaceae, Nymphaeaceae, Papaveraceae, Rosaceae, Salicaceae, Solanaceae, Arecaceae, Bromeliaceae, Cyperaceaa, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae oder Poaceae bevorzugt einer Pflanze ausgewählt aus den Gruppe der Familien Apiaceae, Asteraceae, Brassicaceae, Cucurbitaceae, Fabaceae, Papaveraceae, Rosaceae, Solanaceae, Lillaceae oder Poaceae; einem Pilz wie den Gattungen Aspergillus, Penicillum oder Claviceps oder einer Hefe wie den Gattungen Pichia, Torulopsis, Hansenula, Schizosaccharomyces, Candida, Rhodotorula oder Saccharomyces. Besonders vorteilhaft stammen die Sequenzen aus Hefen wie den Gattungen Pichia, Torulopsis, Kansenula, Schizosaccharomyces, Candida, Rhodotorula oder Saccharomyces, ganz besonders vorteilhaft aus der Hefe der Familie Saccharomycetaceae wie der vorteilhaften Gattung Saccharomyces und der besonders vorteilhaften Gattung und Art Saccharomyces cerevisiae.

Die im erfindungsgemäßen Verfahren verwendeten Nukleinsauresequenzen mit der Sequenz SEQ ID NO: 1, SEQ ID NO: 13 und/oder SEQ ID NO: 15 kodieren für eine Threonin-Aldolase. Diese Aldolase (SEQ ID NO: 1) zeigt die höchste Homologie zum GLY1 Protein von A. gossypii [Eremothecium ashbii, Eremothecium gossypii] (EMBL-Datenbank. Accession Nr. AJ005442, CAA06545.1_{,} GENSEQ_PROT: AAY25338, Identität auf Aminosäureebene zu SEQ ID NO: 1 von 76 %). Auch zu Threonin-Aldolasen, die aus Reis, Soja, Weizen stammen und die in US 2002123118 A1 offenbart wurde, besteht eine hohe Homologie, Darüber hinaus lassen sich Homologien zu einer Vielzahl von Nukleinsäuren finden. Die Threonin-Aldolase von Hefen wie Candida albicans (EMBL-Datenbank. Accession Nr. AF009967, AAB64198.1, Identität auf Aminosaureebene zu SEQ ID NO: 1 von 56 %). von Schizosaccharomyces pombe (EMBL-Datenbank. Accession Nr. Z99163, CAB16235.1, identität auf Aminosäureebene zu SEQ ID NO: 1 von 49 %), oder von Bakterien wie Aeromonas jandaei (EMBL-Datenbank. Accession Nr. AF169478, AAD47837.1, Identität auf Aminosäureebene zu SEQ ID NO: 1 von 41 %), Pseudomonas aeruginosa (EMBL-Datenbank, Accession Nr. AF011922, AAC46016.1, Identität auf Aminosäureebene zu SEQ ID NO: 1 von 38 %), Vibrio cholerae (EMBL-Datenbank. Accession Nr. AE004405, AAF96663.1, Identität auf Aminosäureebene zu SEQ ID NO: 1 von 38 %), Escherichia coli (EMBL-Datenbank. Accession Nr. AB005050, BAA20882.1, Identität auf Aminosäureebene zu SEQ ID NO: 1 von 38 %), Deinococcus radiodurans (EMBL-Datenbank. Accession Nr. AE001978, AAF10885.1, Identität auf Aminosäureebene zu SEQ ID NO: 1 von 38 %), Bacillus halodurans (EMBL-Datenbank. Accession Nr. AP001518, BAB07002.1, Identitat auf Aminosäureebene zu SEQ ID NO: 1 von 34 %), Halobacterium sp. (EMBL-Datenbank. Accession Nr. AE005124, AAG20528.1), Thermologa maritima (EMBL-Datenbank. Accession Nr. AE001813, AAD36809.1, Identität auf Aminosäureebene zu SEQ ID NO: 1 von 40 %) oder den Pflanzen Arabidopsis thaliana (EMBL-Datenbank. Accession Nr. AF325033, AAG40385.1, AC022287, AAF63783.1, AC003981, AAC14037.1, Identität auf Aminosäureebene zu SEQ ID NO: 1 von jeweils 40, 42 bzw. 37 %) oder von nicht-humanen Tieren wie Caenorhabditis elegans (EMBL-Datenbank. Accession Nr. Z70309, CAA94358.1, Identität auf Aminosaureebene zu SEQ ID NO: 1 von 41 %) oder Drosophila melanogaster (EMBL-Datenbank Accession Nr. AE003744, AAF56152.1, Identität auf Aminosäureebene zu SEQ ID NO: 1 von 39 %) oder die Alaninracemase von Pilzen wie Cochliobolus carbonum/Bipolaris zeicola (EMBL-Datenbank. Accession Nr. AF169478, AAD47837.1, Identität auf Aminosäureebene zu SEQ ID NO: 1 von 38 %). Vorteilhaft werden im erfindungsgemäßen Verfahren Nukleinsäuresequenzen und durch diese codierte Proteine verwendet, die aus Hefen der Gattungen Candida, Hansenula, Rhodotorula, Schizosaccharomyces oder Saccharomyces stammen. Weiterhin zeigt die vorteilhaft im erfindungsgemäßen Verfahren verwendete Aldolase eine hohe Homologie zu den unter SEQ ID NO: 3 (Identität auf Aminosäureebene zu SEQ ID NO: 1 von 35 %), SEQ ID NO: 4 (Identität auf Aminosäureebene zu SEQ ID NO: 1 von 35 %), SEQ ID NO: 5 (Identität auf Aminosäureebene zu SEQ ID NO: 1 von 27 %), SEQ ID NO: 6 (Identität auf Aminosäureebene zu SEQ ID NO: 1 von 43 %), SEQ ID NO: 7 (Identität auf Aminosäureebene zu SEQ ID NO: 1 von 39 %), SEQ ID NO: 8 (Identität auf Aminosäureebene zu SEQ ID NO: 1 von 32 %), SEQ ID NO: 9 (Identität auf Aminosäureebene zu SEQ ID NO: 1 von 35 %) oder SEQ ID NO: 10 (Identität auf Aminosäureebene zu SEQ ID NO: 1 von 36 %) genannten Sequenzen, die aus Soja (SEQ ID NO: 3 - 5), Reis (SEQ ID NO: 6 und 7) bzw. aus Canola (SEQ ID NO: 8 -10) stammen. Vorteilhaft können im Verfahren Nukleinsauresequenzen verwendet werden, die sich von den Aminosäuresequenzen SEQ ID NO: 3, SEQ ID NO: 4, SEQ ID NO: 5, SEQ ID NO: 6, SEQ ID NO: 7, SEQ 10 NO: 8, SEQ ID NO: 9 oder SEQ ID NO: 10 ableiten.

Die im erfindungsgemäßen Verfahren verwendeten Nukleinsäuresequenzen mit der Sequenz SEQ ID NO: 11, SEQ ID NO: 17, SEQ ID NO: 19, SEQ ID NO: 21, SEQ ID NO: 23 oder SEQ ID NO: 25 sowie deren Derivate kodieren für eine Lysin-Decarboxylase bzw. für ein Lysin-Decarboxylase ähnliches Protein. Diese vorteilhafte Lysin-Decarboxylase (SEQ ID NO: 11) zeigt die höchste Homologie zu einem Protein von Neurospora crasa (EMBL-Datenbank. Accession Nr. TREMBL_NEW:CAD70937, Identität auf Aminosäureebene zu SEQ ID NO: 11 von 45 °C über ein maximale Länge von 231 Aminosäuren = AS). Auch die Lysin-Decarboxylasen von Oryza sative (EMBL-Datenbank, Accession Nr. SPTREMBL:Q9FWG6, TREMBL_NEW:AA019380, zeigen Identität auf Aminosäureebene zu SEQ ID NO: 11 von jeweils 41 % bzw. 37 %) bzw. die Proteine von Arabidopsis thaliana (EMBL-Datenbank. Accession Nr. SPTREMBL:Q9ASW6, PIR:T45885, SPTREMBL:Q9FNH8, PIR:H84789, PIR:T48348, SPTREMBL:Q9FYM7, PIR:T48554, PIR:T04966 und PIR:E84775, Identität auf Aminosäureebene zu SEQ ID NO: 11 jeweils von 42 %, 42 %, 41 %, 39 %, 39 %, 39 %, 39 %, 36 %, und 35 %,) oder von Bakterien wie Ralstonia solanacearum [= Pseudomonas solanacearum] (EMBL-Datenbank. Accession Nr. SPTREMBL:Q8XXM6, Identität auf Aminosäureebene zu SEQ ID NO: 11 von 43 %), Pseudomonas putida (EMBL-Datenbank. Accession Nr. TREMBL_NEW:AAN70440. Identität auf Aminosäureebene zu SEQ ID NO: 11 von 40 %), Pseudomonas aeruginosa (EMBL-Datenbank. Accession Nr. PIR:A83031, Identität auf Aminosäureebene zu SEQ ID NO: 11 von 40 %), Bacteroides thetalotaomicron (EMBL-Datenbank Accession Nr. TREMBL_NEW:AA078330, Identität auf Aminosäureebene zu SEQ ID NO: 11 von 39 %), Brucella melitensis (EMBL-Datenbank. Accession Nr. PIR:Al3438, Identität auf Aminosäureebene zu SEQ ID NO: 11 von 43 %), Bacillus subtilis (EMBL-Datenbank. Accession Nr. PIR:D70033, Identität auf Aminosäureebene zu SEQ ID NO: 11 von 36 %), Rhizobium loti bzw. Rhisobium meliloti (EMBL-Datenbank. Accession Nr. STREMBL:Q984W8 bzw. STREMBL:Q92R13, Identität aufAminosaureebene zu SEQ ID NO: 11 von 39 % bzw. 36 %). Bacillus halodurans. (EMBL-Datenbank. Accession Nr. PIR:B83993, Identität auf Aminosäureebene zu SEQ ID NO: 11 von 37 %), Agrobacterium tumefaciens (EMBL-Datenbank. Accession Nr. PIR:Al2707 bzw. PIR:B97490, Identität auf Aminosäureebene zu SEQ ID NO: 11 von jeweils 41 %), Staphylococcus aureus (EMBL-Datenbank. Accession Nr. PIR:A89839, Identität auf Aminosaureebene zu SEQ ID NO: 11 von jeweils 34 %), zeigen Homologien zur im erfindungsgemäß verwendeten Lysin-Decarboxylase-Sequenz SEQ ID NO: 11. Vorteilhaft werden im erfindungsgemäßen Verfahren Nukleinsäuresequenzen und durch diese codierte Proteine verwendet, die aus Hefen der Gattungen Candida, Hansenula, Rhodotorula, Schizosaccharomyces oder Saccharomyces stammen. Weiterhin zeigt die vorteilhaft im erfindungsgemäßen Verfahren verwendete Lysin-Decarboxylase eine hohe Homologie zu den unter SEQ ID NO: 21 (Identität auf Aminosäureebene zu SEQ ID NO: 11 von 42 %), SEQ ID NO: 23 (Identität auf Aminosäureebene zu SEQ ID NO: 11 von 43 %) oder SEQ ID NO: 25 (Identität auf Aminosäureebene zu SEQ ID NO: 11 von 37%), die aus Raps, Reis und Maiz stammen. Vorteilhaft können im Verfahren Nukleinsäuresequenzen verwendet werden, die sich von den Aminosäuresequenzen SEQ ID NO: 11, SEQ ID NO: 17, SEQ ID NO: 19, SEQ ID NO: 21, SEQ ID NO: 23 oder SEQ ID NO: 25 ableiten und eine Lysin-Decarboxylase-Aktivität aufweisen.

Für das erfindungsgemäße Verfahren vorteilhafte Nukleinsäuresequenzen, die für Polypeptide mit Threonin-Aldolase-Aktivität oder Lysin-Decarboxylase-Aktivität codieren, lassen sich aus allgemein zugänglich Datenbanken ermitteln. Insbesondere sind hier allgemeine Gen-Datenbanken zu nennen, wie die EMBL-Datenbank (Stoesser G. et al., Nucleic Acids Res 2001, Vol. 29, 17-21), die GenBank-Datenbank (Benson D.A. et al., Nucleic Acids Res 2000, Vol. 28,15-18), oder die PIR-Datenbank (Barker W. C. et al., Nucleic Acids Res. 1999, Vol. 27, 39-43).

Weiterhin können Organismen-spezifische Gen-Datenbanken zur Ermittlung vorteilhafter Sequenzen verwendet werden, so z.B. vorteilhaft für Hefe die SGD-Datenbank (Cherry J. M. et al., Nucleic Acids Res. 1998, Vol. 26, 73-80) oder die MIPS-Datenbank (Mewes H.W. et al., Nucleic Acids Res. 1999, Vol. 27, 44-48), für *E. coli* die GenProtEC-Datenbank (http://web.bham.ac.uk/bcm4ght6/res.html), für Arabidopsis die TAIR-Datenbank (Huala, E. et al., Nucleic Acids Res. 2001 Vol. 29(1), 102-5) oder die MIPS-Datenbank.

Um das Einbringen der Nukleinsäuresequenzen und die Expression der Sequenzen in den transgenen Organismen, die im Verfahren verwendet werden, zu verbessern, werden die Nukleinsäuresequenzen in ein Nukleinsäurekonstrukt und/oder einen Vektor eingebaut Zusätzlich zu den im erfindungsgemäßen Verfahren verwendeten oben beschriebenen Sequenzen können im Nukleinsäurekonstrukt bzw. im Vektor noch weitere Nukleinsäuresequenzen vorteilhaft von Biosynthesegene der im Verfahren hergestellten Aminosäure vorhanden sein, die zusammen in den Organismus eingebracht werden. Diese zusätzlichen Sequenzen können aber auch direkt oder über andere getrennte Nukleinsäurekonstrukte oder Vektoren in die Organismen eingebracht werden. Vorteilhaft werden Gene, die für Threonin-Aldolasen oder Lysin-Decarboxylase codieren, allein oder in Kombination in einen Organismus vorteilhaft einen Mikroorganismus oder einer Pflanze eingebracht

Bei den im erfindungsgemäßen Verfahren verwendeten Nukleinsäuresequenzen handelt es sich um isolierte Nukleinsäuresequenzen, die für Polypeptide mit Threonin-Aldolase-Aktivität oder Lysin-Decarboxylase-Aktivität codieren.

Unter Nukleinsäuren sind im erfindungsgemäßen Verfahren DNA oder RNA-Sequenzen, die einzel- oder doppelsträngig sein oder gegebenenfalls synthetische, nicht natürliche oder veränderte, in DNA oder RNA einbaubare Nukleotidbasen aufweisen können, zu verstehen.

Der Begriff "Expression" meint die Transkription und/oder Translation eines kodogenen Genabschnitts bzw. Gens. In der Regel ist das resultierende Produkt ein Protein. Zu den Produkten gehören aber auch funktionelle RNAs wie z.B. Ribozyme. Die Expression kann systemisch oder lokal, z.B. auf bestimmte Zelltypen, Gewebe oder Organe beschränkt, erfolgen.

Die Expressionsprodukte der im erfindungsgemaßen Verfahren verwendeten Nukleinsäuren z.B. der kodogenen Genabschnitte (ORFs) und ihrer regulatorischen Elemente lassen sich durch ihre Funktion kennzeichnen. Hierzu gehören beispielsweise Funktionen in den Bereichen Metabolismus, Energie, Transkription. Proteinsynthese, Proteinprozessierung, zellulärer Trans, port und Transportmechanismen, zelluläre Kommunikation und Signaltransduktion, Zellrettung, Zellverteidigung und Zellvirulenz, Regulation der zellulären Umgebung und Interaktion der Zelle mit ihrer Umgebung, Zeilschicksal, transponierbare Elemente, virale Proteine und Plasmidproteine, zelluläre Organisationskontrolle, subzelluläre Lokalisation, Regulation der Proteinaktivität, Proteine mit Bindungsfunktion oder Cofaktor-Erfordernis und Transporterleichterung. Gene gleicher Funktion werden zu sogenannten funktionellen Genfamilien zusammengefasst

Durch die biologische Aktivität der im erfindungsgemäßen Verfahren verwendeten Nukleinsäuren, die für Polypeptide mit Threonin-Aldolase-Aktivität oder Lysin-Decarboxylase-Aktivität codieren, können unterschiedliche Aminosäuren hergestellt bzw. deren Herstellung verbessert und/oder gesteigert werden. Je nach Auswahl des für das erfindungsgemäße Verfahren verwendeten Organismus beispielsweise eines Mikroorganismus oder einer Pflanze lassen sich Mischungen der verschiedenen Aminosäuren herstellen. Vorteilhaft wird im erfindungsgemäßen Verfahren L-Lysin und/oder L-Methionin als Aminosäure oder Aminosäuregemisch hergestellt. Besonders bevorzugt wird im Verfahren L-Methionin hergestellt Diese hergestellten Aminosäuren können in den Zellen der transgenen Organismen als freie Aminosäuren und/oder gebunden in Proteinen vorliegen.

Im erfindungsgemäßen Verfahren sind unter transgenen Organismen wenn es sich um Pflanzen handelt auch Pflanzenzellen, -gewebe, -organe wie Wurzel, Spross, Stängel, Same, Blüte, Knolle oder Blatt oder ganze Pflanzen zu verstehen, die zur Herstellung von Aminosäuren angezüchtet werden. Unter Anzucht ist beispielsweise die Kultivierung der transgenen Pflanzenzellen, -gewebe oder -organe auf oder in einem Nahrmedium oder der ganzen Pflanze auf bzw. in einem Substrat beispielsweise in Hydrokultur, Blumentopferde oder auf einem Ackerboden zu verstehen.

Werden Pflanzen im erfindungsgemäßen Verfahren als Spenderorganismus gewählt, so kann diese Pflanze im Prinzip eine beliebige phylogenetische Verwandtschaft mit der Empfängerpflanze aufweisen. So können Spender- und Empfängerpflanze derselben Familie, Gattung, Spezies, Sorte oder Linie angehören, wobei sich eine zunehmende Homologie zwischen den zu integrierenden Nukleinsäuren und entsprechenden Teilen des Genoms der Empfängerpflanze ergibt Gleiches gilt auch für Mikroorganismen als Spender- und Empfängerorganismus.

Vorteilhaft wird im erfindungsgemäßen Verfahren eine Nukleinsäuresequenz mit der in SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 und/oder SEQ ID NO: 25 dargestellten Sequenz, Nukleinsäuresequenzen, die sich von den Aminosäuresequenzen SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO: 10 ableiten, oder deren Derivat oder Homologe, die für Polypeptide codieren, die noch die enzymatische Aktivität bzw. biologische Aktivität besitzen, verwendet Diese Sequenzen werden einzeln oder in Kombination in Expressionskonstrukte cloniert. Diese Expressionskonstrukte ermöglichen eine optimale Synthese der im erfindungsgemäßen Verfahren produzierten Aminosäuren.

Bei einer bevorzugten Ausführungsform umfasst das Verfahren ferner den Schritt des Gewinnens einer Zelle, die die im Verfahren verwendeten Nukleinsäuresequenzen, die für ein Enzym mit Threonin-Aldolase-Aktivität oder Lysin-Decarboxylase-Aktivität codieren, enthält, wobei eine Zelle mit den Nukleinsäuresequenzen, einem Genkonstrukt (= Nukleinsäurekonstrukt) oder einem Vektor, weiche die Expression der Aldolase- oder Decarboxylase-Nukleinsäure allein oder in Kombination mit anderen Genen bzw. Sequenzen herbeiführen, transformiert wird. Bei einer weiteren bevorzugten Ausführungsform umfasst dieses Verfahren ferner den Schritt des Gewinnens der Aminosäure(n) bzw. des Aminosäuregemisches aus der Kultur und/oder dem Organismus. Die so hergestellte Zelle ist vorteilhaft eine Zelle einer wie oben als vorteilhaft beschriebenen Pflanze oder eines Mikroorganismus.

Unter transgenem Organismus wie einer Pflanze oder eines transgenen Mikroorganismus im Sinne der Erfindung ist zu verstehen, dass die im Verfahren verwendeten Nukleinsäuren nicht an ihrer natürliche Stelle im Genom eines Organismus sind, dabei können die Nukleinsäuren homolog oder heterolog exprimiert werden. Transgen bedeutet aber auch, dass die erfindungsgemäßen Nukleinsäuren an ihrem natürlichen Platz im Genom eines Organismus sind, dass jedoch die Sequenz gegenüber der natürlichen Sequenz verändert wurde und/oder das die Regulationssequenzen, der natürlichen Sequenzen verändert wurden. Bevorzugt ist unter transgen die Expression der im erfindungsgemäßen Verfahren verwendeten Nukleinsäuren an nicht natürlicher Stelle im Genom zu verstehen, das heißt eine homologe oder bevorzugt heterologe Expression der Nukleinsäuren liegt vor. Die Expression kann dabei transient oder von einer stabil im Genom integrieten Sequenz aus erfolgen. Bevorzugte transgene Pflanzen sind beispielhaft folgende Pflanzen, ausgewählt aus den Familien Aceraceae, Anacardiaoeae, Apiaceae, Asteraceae, Brassicaceae, Cactaceae, Cucurbitaceae, Euphorbiaceae, Fabaceae, Malvaceae. Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae oder Poaceae bevorzugt einer Pflanze ausgewählt aus den Gruppe der Familien Apiaceae, Asteraceae, Brassicaceae, Cucurbitaceae, Fabaceae, Papaveraceae, Rosaceae, Solanaceae, Liliaceae oder Poaceae. Weitere vorteilhafte bevorzugte Pflanzen sind Nutzpflanzen vorteilhaft ausgewählt aus der Gruppe der Gattung der Erdnuss, Raps, Canola, Sonnenblume, Safflor (Färberdistel), Olive, Sesam, Haselnuss, Mandel, Avocado, Lorbeer, Kürbis, Lein, Soja, Pistazien, Borretsch, Mais, Weizen, Roggen, Hafer, Hirse, Triticale, Reis, Gerste, Maniok, Kartoffel, Zuckerrübe, Aubergine, Alfaalfa sowie ausdauemde Gräser und Futterfeldfrüchte, Ölpalme, Gemüse (Kohlgemüse, Wurzelgemüse, Knollengemüse, Hülsengemüse, Fruchtgemüse, Zwiebeigemüse, Blatt- und Stielgemüse), Buchweizen, Topinambur, Ackerbohne, Wicken, Linse, Buschbohne, Lupine, Klee und Luzerne.

Der erfindungsgemäß verwendete Begriff "transgene Pflanze" bezieht sich auch auf die Nachkommenschaft einer transgenen Pflanze, z.B. die T₁-, T₂-, T₃- und nachfolgende Pflanzengenerationen oder die BC₁-, BC₂-, BC₃- und nachfolgende Pflanzengenerationen. So können die erfindungsgemäßen transgenen Pflanzen aufgezogen und mit sich selbst oder anderen Individuen gekreuzt werden, um weitere erfindungsgemäße transgene Pflanzen zu erhalten. Auch können transgene Pflanzen durch vegetative Propagation transgener Pflanzenzellen erhalten werden. Gegenstand der vorliegenden Erfindung ist auch von einer erfindungsgemäßen Population transgener Pflanzen ableitbares transgenes pflanzliches Material. Hierzu gehören Pflanzenzellen und bestimmte Gewebe, Organe und Teile von Pflanzen in all ihren Erscheinungsformen, wie Samen, Blätter, Antheren, Fasern, Wurzeln, Wurzelhaare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen.

Transgene Pflanzen, die die im erfindungsgemäßen Verfahren synthetisierten Aminosäuren enthalten, können direkt vermarktet werden ohne die synthetisierten Verbindungen zu isolieren. Unter Pflanzen im erfindungsgemäßen Verfahren sind alle Pflanzenteile, Pflanzenorgane wie Blatt, Stiel, Wurzel, Knollen oder Samen oder die gesamte Pflanze zu verstehen. Der Samen umfasst dabei alle Samenteile wie die Samenhüllen, Epidermis- und Samenzellen, Endosperm oder Embyrogewebe. Die im erfindungsgemäßen Verfahren hergestellten Aminosäuren bzw. die vorteilhaft hergestellte Aminosäure L-Methionin können aber auch aus den Pflanzen in Form ihrer freien Aminosäuren oder gebunden in Proteinen isoliert werden. Durch dieses Verfahren hergestellte Aminosäuren lassen sich durch Ernten der Organismen entweder aus der Kultur, in der sie wachsen, oder vom Feld ernten. Dies kann über Pressen, Mahlen und/oder Extraktion, Salzfällung und/oder Ionenaustauscherchromatographie der Pflanzenteile bevorzugt der Pflanzensamen, -früchte, -knollen etc. erfolgen.

Auf diese Weise können mehr als 50 Gew.-%, vorteilhaft mehr als 60 Gew.-%, bevorzugt mehr als 70 Gew.-%, besonders bevorzugt mehr als 80 Gew.-%, ganz besonders bevorzugt mehr als 90 Gew.-% der im Verfahren hergestellten Aminosäuren isoliert werden. Anschließend können die so erhaltenen Aminosäuren ggf. weiter gereinigt, falls gewünscht mit anderen Wirkstoffen wie Vitaminen, Aminosäuren, Kohlenhydraten, Antibiotika etc. abgemischt werden und gegebenenfalls formuliert werden.

Eine weitere erfindungsgemäße Ausführungsform ist die Verwendung der im Verfahren hergestellten Aminosäuren oder der transgenen Organismen in Futtermitteln. Nahrungsmitteln, Kosmetika oder Pharmazeutika.

Die im Verfahren verwendeten Nukleinsäuren können nach Einbringung in eine Pflanzenzelle bzw. Pflanze entweder im Plastidengenom oder bevorzugt in das Genom der Wirtszelle integriert sein auch eine transiente Expression ist möglich und kann vorteilhaft verwendet werden. Auch eine Produktion durch beispielsweise eine Virusinfektion mit rekombinanten Virus ist prinzipiell möglich, dabei wird vorteilhaft die Expression des Gens bzw. der Gene erhöht. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression von Genen in die Pflanzen gebracht. In einer weiteren vorteilhaften Ausführungsform wird die Nukleinsäuresequenz in einer einfachen Expressionskassette oder einem einfachen Konstrukt, das heißt ohne weitere andere Nukleinsäuresequenzen in die Pflanze eingebracht. Bevorzugt werden heterologe Nukleinsäuresequenzen eingebracht

Unter der Verwendung von Klonierungsvektoren in Pflanzen und bei der Pflanzentransformation, wie denjenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utitization, Hrsgb.: Kung und R. Wu, Academic Press (1993), 128-143; Potrykus, Annu. Rev. Plant Physiol. Plant Molec. Biol. 42 (1991), 205-225)), lassen sich die Nukleinsäuren zurgentechnologischen Veränderung eines breiten Spektrums an Pflanzen verwenden, so dass diese ein besserer oder effizienterer Produzent von den im erfindungsgemäßen Verfahren hergestellten Aminosäuren wird. Diese verbesserte Produktion oder Effizienz der Produktion der Aminosäuren oder davon abgeleitete Produkte, wie veränderte Proteine, kann durch direkte Wirkung der Manipulation oder eine indirekte Wirkung dieser Manipulation hervorgerufen werden.

Es gibt eine Reihe von Mechanismen, durch die die Veränderung des im erfindungsgemäßen Verfahren verwendeten Threonin-Aldolase- oder Lysin-Decarboxylase-Proteins die Ausbeute, Produktion und/oder Effizienz der Produktion der Aminosäuren aus einer den transgenen Pflanzen oder den Mikroorganismen wie einer Hefe, einem Pilz oder einem Bakterium aufgrund eines veränderten Proteins direkt beeinflussen kann. Die Anzahl oder Aktivität des Threonin-Aldolase-oder Lysin-Decarboxylase-Proteins oder -Gens kann erhöht sein, so dass durch diese Enzymaktivität größere Mengen des gewünschtes Produkts de novo hergestellt werden, weil den Organismen beispielsweise die eingebrachte enzymatische Aktivität und damit die Fähigkeit zur Erhöhung der Biosynthese vor dem Einbringen des entsprechenden Gens fehlte. Es kann aber auch die Expression des natürlich in den Organismen vorhandenen Gens beispielsweise durch eine veränderte Regulation des Gens erhöht werden oder es kann die Stabilität der mRNA oder des Genproduktes, das heißt der Aldolase oder der Carboxylase erhöht werden. Entsprechendes gilt für die Kombination mit weiteren für die Synthese der Aminosäuren nützlichen Enzymen aus dem Biosynthesestoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression ermöglicht.

Durch das Einbringen eines Threonin-Aldolase- oder Lysin-Decarboxylase-Gens oder mehrerer Aldolase- und/oder Decarboxylase-Gene in einen Organismus allein oder in Kombination mit anderen Genen kann nicht nur der Biosynthesefluss zum Endprodukt erhöht werden, sondern auch eine im Organismus vorhandene Produkt-Zusammensetzung erhöht, verändert oder de novo geschaffen werden. Ebenso kann die Anzahl oder Aktivität anderer Gene, die am Im- oder Export von Nährstoffen der Zelle(n), die zur Biosynthese der Aminosäuren nötig sind, erhöht sein, so dass die Konzentration dieser vorläufer, Cofaktoren oder Zwischenverbindungen innerhalb der Zelle(n) oder innerhalb des Speicherkompartiments erhöht ist, wodurch die Fähigkeit der Zellen zur Produktion von Aminosäuren, wie im folgenden beschrieben, weiter gesteigert wird. Durch die Optimierung der Aktivität oder Erhöhung der Anzahl von Threonin-Aldolase-und/oder Lysin-Decarboxylasenukleinsäuresequenzen und/oder weiteren Genen, die an der Biosynthese der Aminosäuren beteiligt sind, oder durch Zerstören der Aktivität einer oder mehrerer Gene, die am Abbau der Aminosäuren beteiligt sind, kann die Ausbeute, Produktion und/oder Effizienz der Produktion von Aminosäuren im Wirtsorganismus wie der Pflanzen oder der Mikroorganismen gesteigert werden.

Durch diese Beeinflussung des Stoffwechsels lassen sich im erfindungsgemäßen Verfahren weitere vorteilhafte schwefelhaltige Verbindungen herstellen, die wenigstens ein Schwefelatom kovalent gebunden enthalten. Beispiele für derartige Verbindungen sind neben Methionin, Homocystein, S-Adenosylmethionin, Cystein, vorteilhaft Methionin und S-Adenosylmethionin.

Im Rahmen der vorliegenden Erfindung umfassen die Begriffe "L-Methionin", "Methionin", "Homocystein" und "S-Adenosylmethionin" auch die korrespondierenden Salze, wie z. B. Methionin-Hydrochlorid oder Methionin-Sulfat. Die Bezeichnungen Methionin oder Threonin sollen auch die Bezeichnungen L-Methionin oder L-Threonin mit umfassen. Weiterhin sind Proteine umfasst, in die das im Verfahren hergestellte Methionin gebunden sind.

Die im erfindungsgemäßen Verfahren verwendeten isolierten Nukleinsäuremoleküle codieren für Proteine oder Teilen von diesen, wobei die Proteine oder das einzelne Protein oder Teilen davon eine Aminosäuresequenz enthält, die ausreichend homolog zu einer Aminosäuresequenz der Sequenz SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 ist, so dass das Protein oder der Teil davon eine Threonin-Aldolase- oder Lysin-Decarboxylase-Aktivität beibehält Vorzugsweise hat das Protein oder der Teil davon, das/der von dem Nukleinsäuremolekül kodiert wird, seine wesentliche enzymatische bzw. biologische Aktivität und die Fähigkeit, am Stoffwechsel von Aminosäuren in Pflanzen oder Mikroorganismen bzw. allgemein am Pflanzen- oder Mikroorganismenstoffwechsel oder am Transport von Molekülen Ober Membranen teilzunehmen. Vorteilhaft ist das von den Nukleinsäuremolekülen kodierte Protein zu mindestens etwa 30 %, 35 %, 40 %, 45 % oder 50 %, vorzugsweise mindestens etwa 60 % und stärker bevorzugt mindestens etwa 70 %, 80 % oder 90 % und am stärksten bevorzugt mindestens etwa 95 %, 96 %, 97 %, 98 %, 99 % oder mehr homolog zu einer Aminosäuresequenz der Sequenz SEQ ID NO: 2. Bevorzugt ist das Protein ein Vollängen-Protein, das im wesentlichen in Teilen homolog zu einer gesamten Aminosäuresequenz der SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 (die von dem in SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 gezeigten offenen Leserahmen herrührt) ist Weitere vorteilhafte weitere im erfindungsgemäßen Verfahren verwendete Nukleinsäuresequenzen leiten sich von den Aminosäuresequenzen SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO: 10 ab. Vorteilhaft sind die von diesen abgeleiteten Nukleinsäuremoleküle codierten Proteine zu mindestens etwa 70 % oder 75 %, vorzugsweise etwa mindestens 80 % oder 85 %, stärker bevorzugt mindestens etwa 90 %, 91 %, 92 % oder 94 % und am stärksten bevorzugt mindestens etwa 95 %, 96 %, 97 %, 98 %, 99 % oder mehr homolog zu den durch sie codierten Aminosäuresequenzen bzw. einer Aminosäuresequenz der Sequenz SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO: 10. Im Sinne der Erfindung ist unter Homologie oder homolog, Identität oder identisch zu verstehen.

Unter wesentlicher enzymatischer bzw. biologischer Aktivität der verwendeten Enzyme ist zu verstehen, dass sie gegenüber den durch die Sequenzen mit SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder den von den Sequenzen SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO: 10 abgeleiteten Sequenzen und deren Derivate codierten Proteinen/Enzymen im Vergleich noch mindestens eine enzymatische bzw. biologische Aktivität von mindestens 10 %, bevorzugt 20 %, besonders bevorzugt 30 % und ganz besonders 40 % aufweisen und damit am Stoffwechsel von Aminosäuren in einer Pflanzen- oder Mikroorganismenzelle notwendigen Verbindungen oder am Transport von Molekülen über Membranen teilnehmen können, wobei vorteilhaft die Aminosäuren Methionin oder Lysin gemeint sind.

Im Verfahren verwendbare Nukleinsäuren stammen vorteilhaft aus Hefen wie der Familie Saccharomycetaceae wie der vorteilhaften Gattung Saccharomyces oder Hefegattungen wie Candida, Hansenula, Rhodotorula oder Schizosaccharomyces und der besonders vorteilhaften Gattung und Art Saccharomyces cerevisiae. Ihre Sequenz ist unter den EMBL-Accession Nummern Z49330, Y13136 bzw. YJL055W in der EMBL-Datenbank als "hypothetical 26.9 kDa Protein oder U18779, L10830 bzw. U00092 in der EMBL-Datenbank als Produkt Gly1 p (protein required for glycine prototrophy), CDS complement (14603..15766), mit der Protein ID ="AAB64996.1" und db_xref="Gl: 603634" hinterlegt

Alternativ können im erfindungsgemäßen Verfahren isolierte Nukleotidsequenzen verwendet werden, die für putative Aldolasen oder Decarboxylasen codieren und die an eine Nukleotidsequenz der SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder in einer anderen vorteilhaften Ausführungsform an eine Sequenz, die sich von den Sequenzen SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO: 10 ableitet, hybridisieren, z.B. unter stringenten Bedingungen hybridisieren.

Die Hybridisierung sollte vorteilhaft mit Fragmenten von einer Länge von mindestens 200 bp, vorteilhaft mindestens 400 bp, bevorzugt mindestens 600 bp, besonders bevorzugt von mindestens 800 bp, ganz besonders bevorzugt von mindestens 1000 bp durchgeführt werden. In einer besonders bevorzugten Ausführungsform sollte die Hybridisierung mit der gesamten Nukleinsäuresequenz durchgeführt werden.

Die im Verfahren verwendeten Nukleinsäuresequenzen werden vorteilhaft in einer Expressionskassette (= Nukleinsäurekonstrukt), die die Expression der Nukleinsäuren in einem Organismus vorteilhaft einer Pflanze oder einem Mikroorganismus ermöglicht, eingebracht.

Zum Einbringen wird der kodogene Genabschnitt vorteilhaft einer Amplifikation und ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung. Geeignete Klonierungsvektoren sind dem Fachmann allgemein bekannt Hierzu gehören insbesondere Vektoren, die in bakteriellen Systemen replizierbar sind, also vor allem Vektoren, die eine effiziente Klonierung in E. coli gewährleisten, und die stabile Transformation von Pflanzen ermöglichen. Zu nennen sind Insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vit-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt Dadurch sind letztere Vektoren relativ klein, leicht zu manipulieren und sowohl in E.-coll als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Bin19, pBI101, pBinAR, pGPTV und pCAMBIA Eine Übersicht über binäre Vektoren und ihre Verwendung gibt Hellens et al, Trends in Plant Science (2000) 5, 446-451. Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittenen und erforderlichenfalls gereinigten Amplifikat mit ähnlich präparierten Vektorfragmenten mit Einsatz von Ligase kloniert Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, Insbesondere Escherichia coli und Agrobacterium tumefaciens, unter selektiven Bedingungen stabil propagieren und ermöglichen einen Transfer von heterologer DNA in Pflanzen oder andere Mikroorganismen. Gemäß einer besonderen Ausführungsform basieren die Konstrukte auf binären Vektoren (Übersicht zu binären Vektoren in Hellens et al., 2000). Diese beinhalten in der Regel prokaryotische regulatorische Sequenzen, wie Replikationsursprung und Selektionsmarker, zur Vermehrung in Mikroorganismen wie Escherichia coli und Agrobacterium tumefaciens, und Agrobakterium-T-DNA-Sequenzen zwecks Transfer von DNA in Pflanzengenome. Von der gesamtern T-DNA-Sequenz aus Agrobacterium wird zumindest die rechte, etwa 25 Basenpaare umfassende Border-Sequenz benötigt Gewöhnlich beinhalten die erfnäungsgemäßen Vektorkonstrukte T-DNA-Sequenzen sowohl aus dem rechten wie auch aus dem linken Begrenzungsbereich, welche zweckmäßige Erkennungsstellen für ortsspezifisch agierende Enzyme, die wiederum von einem Teil der vir-Gene kodiert werden, beinhalten. Geeignete Wirtsorganismen sind dem Fachmann bekannt Vorteilhafte Organismen sind in dieser Anmeldung weiter oben beschrieben. Hierzu zählen vor allem bakterielle Wirte, von denen einige bereits oben im Rahmen von Spender-Mikroorganismen genannt sind, z.B. Mikroorganismen wie Pilze wie die Gattung Claviceps oder Aspergillus oder gram-positive Bakterien wie die Gattungen Bacillus, Corynebacterium, Micrococcus, Brevibacterium, Rhodococcus, Nocardia, Caseobacter oder Arthrobacter oder gram-negative Bakterien wie die Gattungen Escherichia, Flavobacterium oder Salmonella oder Hefen wie die Gattungen Rhodotorula, Hansenula oder Candida. Besonders vorteilhaft sind Organismen ausgewählt aus der Gruppe der Gattungen Corynebacterium, Brevibacterium, Escherichia, Bacillus, Rhodotorula, Hansenula, Candida, Claviceps oder Flavobacterium. Gans besonders vorteilhaft werden im erfindungsgemäßen Verfahren Mikroorganismen ausgewählt aus der Gruppe der Gattungen und Arten bestehend aus Hansenula anomala, Candida utilis, Claviceps purpurea, Bacillus circulans, Bacillus subtilis, Bacillus sp., Brevibacterium albidum, Brevibacterium album, Brevibacterium cerinum, Brevibacterium flavum, Brevibacterium glutamigenes, Brevibacterium iodinum, Brevibacterium ketoglutamicum, Brevibacterium lactofermentum, Brevibacterium linens, Brevibacterium roseum, Brevibacterium saccharolyticum, Brevibacterium sp., Corynebacterium acetoacidophilum, Corynebacterium acetoglutamicum, Corynebacterium ammoniagenes, Corynebacterium glutamicum (= Micrococcus glutamicum), Corynebacterium melassecola, Corynebacterium sp. oder Escherichia coli speziell Escherichia coli K12 und dessen beschriebene Stämme verwendet Erfindungsgemäß vorteilhaft bevorzugt sind Wirtsorganismen der Gattung Escherichia, Insbesondere Escherichia coli, und Agrobacterium, insbesondere Agrobacterium tumefaciens oder Pflanzen ausgewählt aus den Familien Aceraceae, Anacardiaceae, Apiaceae, Asteraceae, Brassicaceae, Cactaceae, Cucurbitaceae, Euphorbiaceae, Fabaceae, Malvaceae, Nymphaeaceae, Papaveraceae, Rosaceae, Salicaceae, Solanaceae, Arecaceae, Bromeliaceae, Cyperaceae, Iridaceae, Liliaceae, Orchidaceae, Gentianaceae, Labiaceae, Magnoliaceae, Ranunculaceae, Carifolaceae, Rubiaceae, Scrophulariaceae, Caryophyllaceae, Ericaceae, Polygonaceae, Violaceae, Juncaceae oder Poaceae bevorzugt einer Pflanze ausgewählt aus den Gruppe der Familien Apiaceae, Asteraceae, Brassicaceae, Cucurbitaceae, Fabaceae, Papaveraceae, Rosaceae, Solanaceae, Liliaceae oder Poaceae. Weitere vorteilhafte bevorzugte Pflanzen sind Nutzpflanzen vorteilhaft ausgewählt aus der Gruppe der Gattung der Erdnuss, Raps, Canola, Sonnenblume, Safflor (Farberdistel), Olive, Sesam, Haselnuss, Mandel, Avocado, Lorbeer, Kürbis, Lein, Soja, Pistazien, Borretsch, Mais, Weizen, Roggen, Hafer, Hirse, Triticale, Reis, Gerste, Maniok, Kartoffel, Zuckerrübe, Futterrübe, Aubergine sowie ausdauernde Gräser und Futterfeldfrüchte, Ölpalme, Gemüse (Kohlgemüse, Wurtelgemüse, Knollengemüse, Hülsengemüse, Fruchtgemüse, Zwiebelgemüse, Blatt- und Stielgemüse), Buchweizen, Topinambur, Ackerbohne, Wicken, Linse, , Alfaalfa, Buschbohne, Lupine, Klee und Luzeme. Zum Einbringen der im erfindungsgemäßen Verfahren verwendeten Nukleinsäuren in eine Pflanze hat es sich als vorteilhaft erwiesen diese zunächst in einen Zwischenwirt, z.B. ein Bakterium, zu übertragen. Als zweckmäßig hat sich hierbei die Transformation in E. coli erwiesen, die in an sich bekannter Weise, z.B. mittels Hitzeschock oder Elektroporation, durchgeführt werden kann. So können die transformierten E. coli-Kolonien hinsichtlich der Klonierungsefflzienz untersucht werden. Dies kann mit Hilfe einer PCR erfolgen. Dabei kann sowohl die Identität als auch die Integrität des Plasmidkonstrukts anhand einer definierten Kolonienzahl überprüft werden, indem man ein Aliquot der Kolonien besagter PCR unterwirft. Hierfür werden in der Regel universelle, von Vektorsequenzen abgeleitete Primer eingesetzt, wobei der forward-Primer stromaufwärts vom Start-ATG und der reverse-Primer stromabwärts vom Stop-Kodon des kodogenen Genabschnitts angeordnet ist Die Amplifikate werden elektrophoretisch aufgetrennt und hinsichtlich Quantität und Qualität bewertet. Wird ein Fragment in der entsprechenden Größe detektiert, erfolgt eine positive Bewertung. Die gegebenenfalls überprüften Plasmldkonstrukte werden anschließend für die Transformation der Pflanzen verwendet Hierfür kann es zunächst erforderlich sein, die Konstrukte aus dem Zwischenwirt zu gewinnen. Beispielsweise lassen sich die Konstrukte als Plasmide aus bakteriellen Wirten in Anlehnung an eine herkömmliche Plasmidisolierung gewinnen. Es sind zahlreiche Verfahren zur Transformation von Pflanzen bekannt. Da erfindungsgemäß eine stabile Integration heterologer DNA in das Genom von Pflanzen von Vorteil ist, hat sich insbesondere die T-DNA-vermittelte Transformation als zweckmäßig erwiesen. Hierzu ist es zunächst erforderlich, geeignete Vehikel, insbesondere Agrobakterien, mit dem kodogenen Genabschnitt bzw. dem entsprechenden Plasmidkonstrukt zu transformieren. Dies kann in an sich bekannter Weise erfolgen. Beispielsweise kann man das obigen Ausführungen entsprechend erzeugte Plasmidkonstrukt mittels Elektroporation oder Hitzeschock in kompetente Agrobakterien transformieren. Prinzipiell ist hierbei zwischen der Bildung co-integrierter Vektoren einerseits und der Transformation mit binären Vektoren zu unterscheiden. Bei der ersten Alternative weisen die den kodogenen Genschnitt umfassenden Vektorkonstrukte keine T-DNA-Sequenzen auf, sondern die Bildung der co-integrierten Vektoren erfolgt in den Agrobakterien durch homologe Rekombination des Vektorkonstruktes mit T-DNA. Die T-DNA liegt in den Agrobakterien in Form von Ti- oder Ri-Plasmiden, in denen die Onkogene zweckmäßigerweise durch exogene DNA ersetzt wurden, vor. Verwendet man binäre Vektoren, so können diese durch bakterielle Konjugation oder direkten Transfer auf Agrobakterien übertragen werden. Diese Agrobakterien enthalten zweckmäßigerweise bereits den Vektor, der die vir-Gene trägt (häufig als Helfer-Ti(Ri)-Plasmid bezeichnet). Zusammen mit dem Plasmidkonstrukt und der T-DNA können zweckmäßigerweise auch ein oder mehrere Marker verwendet werden, anhand derer die Selektion transformierter Agrobakterien und transformierter Pflanzenzellen möglich ist. Für diesen Zweck wurde eine Vielzahl von Markern entwickelt Hierzu gehören beispielsweise solche, die eine Resistenz gegen Chloramphenicol, Kanamycin, dem Aminoglykosid G418, Hygromycin und ähnliches verleihen.. In der Regel ist es erwünscht, dass die Plasmidkonstrukte an einer oder beiden Seiten des kodogenen Genabschnitts durch T-DNA flankiert sind. Dies ist insbesondere dann von Nutzen, wenn Bakterien der Arten Agrobacterium tumefaciens oder Agrobacterium rhizogenes für die Transformation verwendet werden. Eine erfindungsgemäß bevorzugte Methode ist die Transformation mit Hilfe von Agrobacterium tumefaciens. Aber auch biolistische Methoden können vorteilhaft zum Einbringen der Sequenzen im erfindungsgemäßen Verfahren verwendet werden, auch das Einbringen mittels PEG ist möglich. Die transformierten Agrobakterien können in an sich bekannter Weise kultiviert werden und stehen damit für eine zweckmäßige Transformation der Pflanzen bereit Die zu transformierenden Pflanzen oder Pflanzenteile werden in herkömmlicher Weise angezogen bzw. bereitgestellt. Anschließend lässt man die transformierten Agrobakterien so lange auf die Pflanzen bzw. Pflanzenteile einwirken, bis eine hinreichende Transformationsrate erreicht ist Das Einwirken der Agrobakterien auf die Pflanzen bzw. Pflanzenteile kann in unterschiedlicher Art und Weise erfolgen. Beispielsweise kann man eine Kultur morphogener pflanzlicher Zellen oder Gewebe verwenden. Im Anschluss an den T-DNA-Transfer werden die Bakterien in der Regel durch Antibiotika eliminiert und die Regeneration pflanzlichen Gewebes induziert. Insbesondere verwendet man hierzu geeignete pflanzliche Hormone, um nach anfänglicher Kallus-Bildung die Sprossentwicklung zu fördern. Eine vorteilhafte Transformationsmethode ist die Transformation in planta. Hierzu kann man beispielsweise die Agrobakterien auf pflanzliche Samen einwirken lassen oder Pflanzenmeristem mit Agrobakterien inokulieren. Erfindungsgemäß hat es sich insbesondere als zweckmäßig erwiesen, eine Suspension transformierter Agrobakterien auf die gesamte Pflanze oder zumindest die Blütenanlagen einwirken zu lassen. Diese wird anschließend weiter angezogen, bis Samen der behandelten Pflanze gewonnen werden (Clough und Bent, Plant J. (1998) 16, 735-743). Zur Auswahl transformierter Pflanzen wird das aus der Transformation gewonnene pflanzliche Material in der Regel selektiven Bedingungen unterworfen, so dass transformierte von nichttransformierten Pflanzen unterschieden werden können. Beispielsweise können die in der vorstehend beschriebenen Art und Weise gewonnenen Samen erneut ausgebracht und nach Anzucht einer geeigneten Sprühselektion unterworfen werden. Eine weitere Möglichkeit besteht darin, die Samen, erforderlichenfalls nach Sterilisation, auf Agarplatten unter Verwendung eines geeigneten Selektionsagens so anzuziehen, dass lediglich die transformierten Samen zu Pflanzen anwachsen können. Weitere vorteilhafte Transformationsmethoden insbesondere von Pflanzen sind dem Fachmann bekannt und werden im folgenden beschrieben.

Im erfindungsgemäßen Verfahren werden die für im erfindungsgemäßen Verfahren verwendeten Threonin-Aldolase und/oder Lysin-Decarboxylase codierenden Nukleinsäuresequenzen mit einem oder mehreren Regulationssignalen vorteilhafterweise zur Erhöhung der Genexpression funktionell verknüpft. Diese regulatorischen Sequenzen sollen die gezielte Expression der Gene und die Proteinexpression ermöglichen. Dies kann beispielsweise je nach Wirtsorganismus (= transgener Organismus z.B. Pflanze oder Mikroorganismus) bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Die Expressionskassette (= Expressionskonstrukt = Genkonstrukt = Nukleinsäurekonstrukt) kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen Inseriert werden wie weitere regulatorische Elemente oder Terminatoren. Die Nukleinsäuresequenz(en), die für die Threonin-Aldolase-Proteine codiert(en), kann (können) in einer oder mehreren Kopien in der Expressionskassette (= Nukleinsäurekonstrukt) enthalten sein. Vorteilhaft liegt nur jeweils eine Kopie der Gene in der Expressionskassette vor. Dieses Nukleinsäurekonstrukt oder die Nukleinsäurekonstrukte können zusammen im Wirtsorganismus exprimiert werden. Dabei kann das Nukleinsäurekonstrukt oder die Nukleinsäurekonstrukte in einem oder mehreren Vektoren inseriert sein und frei in der Zelle vorliegen oder aber im Genom inseriert sein, vorteilhaft. Im Falle von Pflanzen kann eine Integration ins Plastidengenom oder bevorzugt in das Zellgenom erfolgt sein. Es ist vorteilhaft für die Insertion weiterer Gene im Wirtsgenom, wenn die zu exprimierenden Gene zusammen in einem Genkonstrukt vorliegen.

Regulatorische Sequenzen sind in der Regel stromaufwärts (5'), innerhalb und/oder stromabwärts (3') in Bezug auf eine bestimmte Nukleinsäure bzw. einen bestimmten kodogenen Genabschnitts angeordnet. Sie kontrollieren insbesondere die Transkription und/oder Translation sowie die Transkriptstabilität des kodogenen Genabschnitts, gegebenenfalls in Zusammenspiel mit weiteren zelleigenen funktionellen Systemen wie dem Proteinblosynthese-Apparat der Zelle.

Zu regulatorischen Sequenzen gehören vor allem stromaufwärts (5') angeordnete Sequenzen, welche insbesondere die Regulation der Transkriptionsinitiation betreffen, wie Promotoren, und stromabwärts (3') angeordnete Sequenzen, welche vor allem die Regulation der Transkriptionstermination betreffen, wie Polyadanylierungssignale.

Grundsätzlich sind alle Promotoren einsetzbar, welche die Transkription von Genen in Organismen wie Mikroorganismen, Pflanzen oder Nicht-humanen Tieren stimulieren können. Geeignete in diesen Organismen funktionsfähige Promotoren sind allgemein bekannt. Es kann sich um konstitutive oder induzierbare Promotoren handeln. Geeignete Promotoren können in mehrzelligen Eukaryonten eine entwicklungs- und/oder gewebespezifisch Expression ermöglichen, so können In Pflanzen vorteilhaft Blatt-, Wurzel-, Blüten-, Samen-, Schließzellen- oder Fruchtspezifische Promotoren verwendet werden.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem z B. Translationsenhancersequenzen eingeführt werden oder die Stabilität der mRNA verbessert wird.

Eine weitere Ausführungsform der Erfindung sind ein oder mehrere Nukleinsäurakonstrukte, die eine oder mehrere Nukleinsäuresequenzen enthalten, die durch SEQ ID NO: 1, SEQ ID NO: 11, SEQ ID NO: 13, SEQ ID NO: 15, SEC ID NO: 17, SEQ ID NO: 19, SEQ ID NO: 21, SEQ ID NO: 23 oder SEQ ID NO: 25 definiert sind und für die in SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 wiedergegebenen Polypeptide kodieren. Eine weitere vorteilhafte Ausführungsform der Erfindung sind ein oder mehrere Nukleinsäurekonstrukte, die eine oder mehrere Nukleinsäuresequenzen enthalten, die sich von den erfindungsgemäßen Sequenzen SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO: 10 ableiten lassen. Die genannten Polypeptide haben vorteilhaft eine Threonin-Aldolase- Aktivität. Gleiches gilt für ihre Homologen, Derivate oder Analoga, die funktionsfähig mit einem oder mehreren Regulationssignalen, vorteilhafterweise zur Steigerung der Genexpression, verbunden sind.

Vorteilhafte Regulationssequenzen für das neue Verfahren liegen beispielsweise in Promotoren vor, wie dem cos-, tac-, rha-, trp-, tet-, trp-tet-, Ipp-, lac-, Ipp-lac-, lacl^{q-,} T7-, T5-, T3-, gal-, tro-, ara-, SP6-, λ-P_{R}- oder λ-P_{L}-Promotor, die vorteilhafterweise in Gram-negativen Bakterien verwendet werden. Weitere vorteilhafte Regulationssequenzen liegen beispielsweise in den Gram-positiven Promotoren amy, dnaK, xylS und SPO2, in den Hefe- oder Pilzpromotoren ADC1, MFα, AC, P-60, UASH, MCB, PHO, CYC1, GAPDH, TEF, rp28, ADH oder in den Pflanzenpromotoren CaMV/35S [Franck et al., Cell 21 (1980) 285-294, US 5,352,605], PRP1 [Ward et al., Plant. Mol. Biol. 22 (1993)], SSU, PGEL1, OCS [Leisner and Gelvin (1988) Proc Natl Acad Sci USA 85(5):2553-2557], lib4, usp, mas [Comai et al. (1990) Plant Mol Biol 15 (3):373-381], STLS1, ScBV Schenk et al. (1999) Plant Mol Biol 39(6):1221-1230, B33, SAD1 oder SAD2 (Flachspromotoren, Jain et al., Crop Science, 39 (6), 1999: 1696 - 1701) oder nos [Shaw et al. (1984) Nucleic Acids Res. 12(20):7831-7846]. Auch die verschiedenen Ubiquitinpromotoren aus Arabidopsis [Callis et al.(1990) J. Biol. Chem., 265:12486-12493; Holtorf S et al. (1995) Plant. Mol. Biol., 29:637-747], Pinus, Mais [(Ubi1 und Ubi2), US 5,510,474; US 6,020,190 und US 6,054574] oder Petersilie [Kawalleck et al., Plant Molecular Biology, 21, 1993: 673 - 684] oder Phaseolin-Promotor können vorteilhaft verwandt werden. In diesem Zusammenhang vorteilhaft sind ebenfalls induzierbare Promotoren, wie die in EP-A-0 388 186 (Benzylsulfonamidinduzierbar), Plant J. 2, 1992:397-404 (Gatz et al., Tetracyclin-induzierbar), EP-A-0 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in spezifischen Geweben ermöglichen oder eine präferenzielle Expression in bestimmten Geweben zeigen. Vorteilhaft sind auch samenspezifische Promotoren, wie der ausführungsgemäße USP Promotor aber auch andere Promotoren wie der LeB4-, DC3-, SAD1-. Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen Ipt-2- oder Ipt-1-Promotor aus Gerste (WO 95/15389 und WO 95/23230), Hordein-Promotor aus Gerste, der Ubiquitin-Promotor aus Mais und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. beschrieben in WO 99/16890.

Um einen besonders effektiven Gehalt an Threonin-Aldolase- und/oder Lysin-Decarboxylase-Proteinen in transgenen Pflanzen zu erzielen, können die codierten Biosynthesegene vorteilhaft konstitutiv und/oder samen-, frucht- oder knollenspezifisch in Pflanzen exprimiert werden. In einer weiteren vorteilhaften Ausführungsform können sie aber auch induzierbar exprimiert werden, so dass sie gezielt in einer gewünschten Wachstumsphase der Pflanze induziert und damit exprimiert werden. Hierzu können Samen-spezifische Promotoren verwendet werden, bzw. solche Promotoren die im Embryo und/oder im Endosperm aktiv sind. Samen-spezifische Promotoren können prinzipiell sowohl aus dikotolydonen als auch aus monokotolydonen Pflanzen isoliert werden. Im folgenden sind vorteilhafte bevorzugte Promotoren aufgeführt USP (= unknown seed protein) und Vicilin (Vicia faba) [Bäumlein et al., Mol. Gen Genet., 1991, 225(3)], Napin (Raps) [US 5,608,152], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und Ipt1 (Gerste) [WO 95/15389 u. WO95/23230], Samen-spezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder β-Amylase (Gerste) [EP 781 849].

Die Pflanzengenexpression lässt sich auch über einen chemisch Induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt Beispiele für solche Promotoren sind ein Salicylsäure-induzierbarer Promotor (WO 95/19443), ein Tetracyclin-Induzierbarer Promotor (Gatz et al. (1892) Plant J. 2, 397-404) und ein Ethanol-induzierbarer Promotor.

Auch eine Expression spezifisch in Gymnospermen oder Angiospermen ist prinzipiell möglich Um eine stabile Integration im erfindungsgemäßen Verfahren verwendeten Nukleinsäuresequenzen in Kombination mit weiteren Biosynthesegene in die transgene Pflanze über mehrere Generation sicherzustellen, sollte jede der im Verfahren verwendeten Nukleinsäuren, die für die Aldolasen und/oder Decarboxylasen codieren, unter der Kontrolle eines eigenen bevorzugt eines unterschiedlichen Promotors exprimiert werden, da sich wiederholende Sequenzmotive zu instabilität der T-DNA bzw. zu Rekombinationsereignissen bzw zu Silencing führen können. Die Expressionskassette ist dabei vorteilhaft so aufgebaut, dass einem Promotor eine geeignete Schnittstelle zur Insertion der zu expremierenden Nukleinsäure folgt vorteilhaft in einem Polylinker anschließend gegebenenfalls ein Terminator hinter dem Polylinker liegt. Diese Abfolge wiederholt sich mehrfach bevorzugt drei-, vier- oder fünfmal, so dass bis zu fünf Gene in einem Konstrukt zusammengeführt werden und so zur Expression In die transgene Pflanze eingebracht werden können. Vorteilhaft wiederholt sich die Abfolge bis zu dreimal. Die Nukleinsäuresequenzen werden zur Expression über die geeignete Schnittstelle beispielsweise im Polylinker hinter den Promotor inseriert. Vorteilhaft hat jede Nukleinsäuresequenz ihren eigenen Promotor und gegebenenfalls ihren eigenen Terminator. Es ist aber auch möglich mehrere Nukleinsäuresequenzen hinter einem Promotor und ggf. vor einem Terminator zu inserieren. Dabei ist die insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch die Expression wesentlich beeinflusst wird. Es können in der Expressionskassette vorteilhaft unterschiedliche Promotoren wie beispielsweise der USP-, der LegB4-, der DC3-Promotor oder der Ubiquitin-Promotor aus Petersilie und unterschiedliche Terminatoren verwendet werden. Es ist aber auch möglich nur einen Promotortyp in der Kassette zu verwenden. Dies kann jedoch zu unerwünschten Rekombinationsereignissen oder Silencingeffekten führen. Eine weitere vorteilhafte Nukleinsäuresequenz, die in Kombination mit den im Verfahren verwendeten Sequenzen und/oder den vorgenannten Biosynthesegenen exprimiert werden kann, ist die Sequenz für einen ATP/ADP-Translokator wie sie in WO 01/20009 beschrieben wird. Dieser ATP/ADP-Translokator führt zu einer Steigerung der Synthese der essentiellen Aminosäuren Lysin und/oder Methionin vorteilhaft von Methionin.

Wie oben beschrieben sollte die Transkription der eingebrachten Gene vorteilhaft durch geeignete Terminatoren am 3'-Ende der eingebrachten Biosynthesegene (hinter dem Stopcodon) abgebrochen werden. Verwendet werden kann hier z.B. der OCS1 Terminator. Wie auch für die Promotoren, so sollten hierfür jedes Gen unterschiedliche Terminatorsequenzen verwendet werden.

Das Genkonstrukt kann, wie oben beschrieben, auch weitere Gene umfassen, die in die Organismen eingebracht werden sollen. Es ist möglich und vorteilhaft, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Weiterhin können vorteilhaft im Nukleinsäurekonstrukt bzw. Genkonstrukt weitere Biosynthesegene enthalten sein oder aber diese Gene können auf einem weiteren oder mehreren weiteren Nukleinsäurekonstrukten liegen. Vorteilhaft werden als Biosynthesegene Gene des Aminosäurestoffwechsels, der Glykolyse, des Tricarbonsäurestoffwechsels oder deren Kombinationen verwendet.

Dabei können die vorgenannten Polypeptide bzw. Enzyme in Kombination mit weiteren Genen in den Nukleinsaurekonstrukten oder Vektoren kloniert werden und zur Transformation von Mikroorganismen oder Pflanzen mithilfe von z.B. Agrobacterium eingesetzt werden.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert oder eine Translationsenhancersequenz eingefügt wird. Die Expressionskassetten können prinzipiell direkt zum Einbringen in die Pflanze verwendet werden oder aber in einen Vektoren eingebracht werden.

Diese vorteilhaften Vektoren, vorzugsweise Expressionsvektoren, enthalten die im Verfahren verwendeten Nukleinsäure, die für Threonin-Aldolase- und/oder Lysin-Decarboxylase-Proteine codieren, oder ein Nukleinsäurekonstrukt, die die verwendeten Nukleinsäure allein oder in Kombination mit weiteren Genen wie den Biosynthesegenen des Aminosäurestoffwachsels enthält. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", welches für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere bevorzugte Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Sie können, wie vorgenannt, autonom replizieren oder in das Wirtsgenom integriert sein. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die erfindungsgemäßen Nukleinsäuren oder das erfindungsgemäße Nukleinsäurekonstrukt in einer Form, die sich zur Expression der verwendeten Nukleinsäuren in einer Wirtszelle eignen, was bedeutet, dass die rekombinanten Expressionsvektoren eine oder mehrere Regulationssequenzen, ausgewählt auf der Basis der zur Expression zu verwendenden Wirtszellen, die mit der zu expremierenden Nukleinsäuresequenz funktionsfähig verbunden ist, umfasst In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann.

Die verwendeten rekombinanten Expressionsvektoren können speziell zur Expression von im Verfahren verwendeten Nukleinsäuresequenzen in prokaryotischen oder eukaryotischen Zellen ausgestaltet sein. Dies ist vorteilhaft, da häufig Zwischenschritte der Vektorkonstruktion der einfachheithalber in Mikroorganismen durchgeführt werden. Beispielsweise können die Aminosäure-, Lysin-Decarboxylase- und/oder Threonin-Aldoalse-Gene in bakteriellen Zellen, Insektenzellen (unter Verwendung von Baculovirus-Expressionsvektoren), Hefe- und anderen Pilzzellen [siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations In Fungi, J.W. Bennet & L.L. Lasure, Hrsgb., S. 396-428: Academic Press: San Diego; und van den Hondel, C.A.M.J.J.. & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge], Algen [Falciatore et al., 1999, Marine Biotechnology, 1, 3:239-251] mit Vektoren nach einem Transformationsverfahren, wie beschrieben in WO 98/01572, sowie bevorzugt in Zellen vielzelliger Pflanzen [siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43: Potrykus, Annu. Rev. Plant Physiol. Plant Molec. Biol. 42 (1991), 205-225 (und darin zitierte Literaturstellen)] exprimiert werden. Geeignete Wirtszellen werden ferner erörtert in Goeddel, Gene Expression Technology: Methods in Enzymology 185, Academic Press, San Diego, CA (1990). Die Sequenz des rekombinanten Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc, Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird.

Beispiele für geeignete induzierbare nicht-Fusions-E. coli-Expressionsvektoren sind u.a. pTrc (Amann et al. (1988) Gene 69:301-315) und pET 11d [Studier et al., Gene Expression Technology: Methods in Enzymology 185, Academic Press, San Diego, Kalifornien (1990) 60-89]. Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 gn1-Gen unter der Transkriptionskontrolle des lacUV 5-Promotors birgt.

Andere In prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III¹¹³-B1, λgt11 or pBdCl, In Streptomyces plJ101, plJ364, plJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasec1 (Baldari et al. (1987) Embo J. 6:229-234), pMFa (Kurjan und Herskowitz (1982) Cell 30:933-843), pJRY88 (Schultz et al. (1987) Gene 54:113-123) sowie pYES2 (Invitrogen Corporation. San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. [(1991) "Gene transfer systems and vector development for filamentous fungi, in: Applied Molecular Genetics of fungi, J.F. Peberdy et al., Hrsgb., S. 1-28, Cambridge University Press: Cambridge; oder in: More Gene Manipulations in Fungi; J.W. Bennet & L.L. Lasure, Hrsgb., S. 396-428: Academic Press: San Diego]. Weitere geeignete Hefevektoren sind beispielsweise 2αM, pAG-1, YEp6, YEp13 oder pEMBLYe23.

Als weitere Vektoren seien in Pilzen pALS1, pIL2 oder pBB116 oder in Pflanzen pLGV23, pGHlac*, pBIN19, pAK2004 oder pDH51 beispielhaft genannt.

Alternativ können die Nukleinsauresequenzen in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol., 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Bei einer weiteren vorteilhaften Ausführungsform des Verfahrens können die Nukleinsäuresequenzen in einzelligen Pflanzenzellen (wie Algen), siehe Falciatore et al., 1999, Marine Blotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell. J., und Masterson, R. [(1992) "New plant binary vectors with selectable markers located proximal to the left border". Plant Mol. Biol. 20:1195-1197] und Bevan, M.W. [(1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization. Hrsgb.: Kung und R. Wu, Academic Press, 1993, S. 15-38]. Eine Übersicht über binäre Vektoren und ihre Verwendung findet sich auch in Hellens, R., Mullineaux, P. und Klee H. , [(2000)" A guide to Agrobacterium binary vectors ,Trends in Plant Science, Vol 5 No.10. 446-451.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbundene Sequenzen, wie Translationsenhancer, beispielsweise die Overdrive-Sequenz, welche die 5'-untranslatierte Leader-Sequenz aus Tabakmosaikvirus, die das Protein/RNA-Verhaltnis erhöht, enthält (Gallie et al., 1987, Nucl. Acids Research 15:8693-8711).

Zur Expression in Pflanzen müssen die Nukleinsäuresequenzen wie oben beschrieben funktionsfähig mit einem geeigneten Promotor verbunden sein, der die Genexpression auf rechtzeitige, zell- oder gewebespezifische Weise durchführt. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913), 34S FMV (Sanger et al., Plant Mol. Biol., 14, 1990: 433 - 443), der Ubiquitin-Promotor aus Petersilie oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor erleichtern (siehe eine Übersicht in Gatz 1997, Annu. Rev. Plant Physiol. Plant Mol. Biol., 48:89-108). Chemisch Induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alphaamylase-Promotor aus Kartoffel (WO 96/12814) oder der durch Verwundung induzierbare pinII-Promotor (EP-A-0 375 091).

Es sind insbesondere solche Promotoren bevorzugt, welche die Genexpression in Geweben und Organen herbeiführen, in denen die Aminosäure-Biosynthese stattfindet, in Samenzellen, wie den Zellen des Endosperms und des sich entwickeinden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980), der arc5-Promotor aus der Bohne, der DcG3-Promotor aus der Karotte oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Vorteilhafte samenspezifische Promotoren sind der Sucrose-Binding-Protein-Promotor (WO 00/26388), der Phaseolin-Promotor und der Napin-Promotor. Geeignete beachtenswerte Promotoren sind der lpt2- oder Ipt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen).

Insbesondere kann die multiparallele Expression der im Verfahren verwendeten Nukleinsäuren allein oder in Kombination mit anderen Genen bzw. Nukleinsäuren gewünscht sein. Die Einführung solcher Expressionskassetten kann über eine simultane Transformation mehrerer einzeiner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Ebenfalls besonders geeignet sind Promotoren, welche die plastidenspezifische Expression herbeiführen. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Für die starke Expression heterologer Sequenzen in möglichst vielen Geweben, insbesondere auch Blättern, werden neben verschiedenen der oben genannten viralen und bakteriellen Promotoren, bevorzugt pflanzliche Promotoren von Actin- oder Ubiquitin-Genen, wie beispielsweise der Actin1-Promotor aus Reis verwendet. Einen weiteres Beispiel für konstitutive pflanzliche Promotoren stellen die V-ATPase-Promotoren aus Zuckerrübe dar (WO 01/14572). Beispielhaft für synthetische konstitutive Promotoren sind der Super-Promotor (WO 95/14096) und von G-Boxen abgeleitete Promotoren (WO 94/12015) zu nennen. Weiterhin können unter Umständen auch chemisch induzierbare Promotoren genutzt werden, vergleiche EP-A 388186, EP-A 335528, WO 97/06268. Auch stehen für die Expression von Genen in Pflanzen blattspezifische Promotoren wie in DE-A 19644478 beschrieben, oder lichtregulierte Promotoren, wie beispielsweise der petE-Promotor aus Erbse zur Verfügung.

Von den Polyadenylierungssignalen ist insbesondere die Poly-A-Additionssequenz aus dem ocs-Gen oder nos-Gen von *Agrobacterium tumefaciens* zu nennen. Zu weiteren gegebenenfalls zweckmäßigen regulatorischen Sequenzen gehören auch Sequenzen, die den Transport und/oder die Lokalisierung der Expressionsprodukte steuern (Targeting). Hier sind insbesondere die an sich bekannten Signalpeptid oder Transitpeptid kodierenden Sequenzen zu nennen. Beispielsweise gelingt es mit Hilfe von Plastid-Transitpeptid kodierenden Sequenzen, das Expressionsprodukt in die Plastide einer Pflanzenzelle zu leiten. Als Empfängerpflanzen werden wie oben beschrieben insbesondere Pflanzen bevorzugt, die in zweckmäßiger Weise transformiert werden können. Hierzu gehören mono- und dikotelydone Pflanzen. Insbesondere sind landwirtschaftliche Nutzpflanzen wie Getreide und Gräser, z.B.Triticum spp., Zea mays, Hordeum vulgare, Hafer, Secale cereale, Oryza sativa, Pennisetum glaucum, Sorghum bicolor, Triticale, Agrostis spp., Cenchrus ciliaris, Dactylis glomerata, Festuca arundinacea, Lolium spp., Medicago spp. und Saccharum spp., Hülsenfrüchte und Ölfrüchte, z.B. Brassica juncea, Brassica napus, Glycine max, Arachis hypogaea, Gossypium hirsutum, Cicer arietinum, Helianthus annuus, Lens culinaris, Linum usitatissimum, Sinapis alba, Trifolium repens und Vicla narbonensis, Gemüse und Früchte, z.B. Bananen, Weintrauben, Lycopersicon esculentum, Spargel, Kohl, Wassermelonen, Kiwis, Solanum tuberosum, Beta vulgaris, Cassava und Chicory, Bäume, z.8. Coffea species, Citrus spp., Eucalyptus spp., Picea spp., Pinus spp. und Populus spp., medizinische Pflanzen und Bäume sowie Blumen zu nennen. Gemäß einer besonderen Ausführungsform betrifft die vorliegende Erfindung transgene Pflanzen der Gattung *Arabidopsis,* z.B. *Arabidopsis thaliana* und der Gattung *Oryza*.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, PEG-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern wie Methods in Molecular Biology, 1995, Bd. 44, Agrobacterium protocols, Hrsgb: Gartland und Davey, Humana Press, Totowa, New Jersey.

Der Begriff "Nukleinsäure(molekül bzw. -sequenz)", wie hier verwendet, kann zudem die am 3'-und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs mit umfassen. Vorteilhaft wird zur Klonierung und Expression nur der codierende Bereich genommen. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte im erfindungsgemäßen Verfahren verwendete Nukleinsäuremolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt flankieren.

Die im Verfahren verwendeten Nukleinsäuremoleküle, z.B. ein Nukleinsäuremolekül mit einer Nukleotidsequenz der SEQ ID NO:1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder eines Teils davon, kann unter Verwendung molekularbiologischer Standardtechniken und der hier bereitgestellten Sequenzinformation isoliert werden. Auch kann mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Auft., Cold Spring Harbor Laboratory, Cold Spring Harbor Laboratory Press, Cold Spring Harbor, NY, 1989) zur Isolierung weiterer Im Verfahren nützlicher Nukleinsäuresequenzen verwendet werden. Überdies lässt sich ein Nukleinsäuremolekül, umfassend eine vollständige Sequenz der SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder einen Teil davon, durch Polymerasekettenreaktion isolieren, wobei Oligonukleotidprimer, die auf der Basis dieser Sequenz oder von Teilen davon, verwendet werden (z.B. kann ein Nukleinsauremolekül, umfassend die vollständigen Sequenz oder einen Teil davon, durch Polymerasekettenreaktion unter Verwendung von Oligonukleotidprimern Isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, er hältlich von Seikagaku America, Inc., St.Petersburg, FL) herstellen. Synthetische Oligonukleotidprimer zur Ampllflzlerung mittels Polymerasekettenreaktion lassen sich auf der Basis einer der in SEQ ID NO: 1, SEQ ID NO: 11, SEQ ID NO: 13. SEQ ID NO: 15, SEQ ID NO: 17, SEQ ID NO: 19. SEQ ID NO: 21, SEQ ID NO: 23 oder SEQ ID NO: 25 oder mithilfe der in SEQ ID NO: 2, SEQ ID NO:12, SEQ ID NO:14, SEQ ID NO:16, SEQ ID NO: 18, SEQ ID NO: 20, SEQ ID NO: 22, SEQ ID NO: 24 oder SEQ ID NO: 26 dargestellten Aminosäuresequenzen erstellen.Weiterhin können durch Proteinsequenzvergleiche von Threonin Aldolasen oder Lysin-Decarboxylasen aus verschiedenen Organismen konservierte Bereiche identifiziert werden, von denen dann wiederum degenerierte Primer abgeleitet werden können. Derartige degenerierte Primer lassen sich aus den Consensus-Sequenzen H[x]₂G[X]R[X]₁₉D[X]₇K[X]₂₇G, HXDGAR[X]₃A[X]₁₅D[X]₄CXSK[X]₄PXGS[X]₃G[X]₇A[X]₄K[X]₂GGGXRQXG, G[X]₄GIM[X]₄₅**M[X]₂RK[X]₂M[X]₁₁GGXG[X]₃E[X]₂**E[X]₃W, oder LG[X]₉ LVYGG[X]₃GIMGXVA[X]₉G[X]₃GXIP[X]₂₄**MHXRK[X]₂M[X]₈F[X]₉PGGXGTXEE[X]₂** E[X]₂TW[X]₂IG[X]₃KP[X]₄N[X]₃FY[X]₁₄F ableiten. Diese degenerierten Primer können dann mittels PCR zur Amplifikation von Fragmenten neuer Threonin-Aldolasen und/oder Lysin-Decarboxylasen aus weiteren Organismen genutzt werden. Diese Fragmente können dann als Hybridiserungssonde für die Isolierung der vollständigen Gensequenz genutzt werden. Alternative können die fehlenden 5'und 3'-Sequenzen mittels RACE-PCR Isoliert werden. Eine erfindungsgemäße Nukleinsäure kann unter Verwendung von cDNA oder alternativ von genomischer DNA als Matrize und geeigneten Oligonukleotidprimern gemäß Standard-PCR-Ampfifikationstechniken ampfifiziert werden. Die so amplifizierte Nukleinsäure kann in einen geeigneten Vektor kloniert werden und mittels DNA-Sequenzanaiyse charakterisiert werden. Oligonukleotide, die einer der im Verfahren verwendeten Nukleotidsequenz entsprechen, können durch Standard-Syntheseverfahren, beispielsweise mit einem automatischen DNA-Synthesegerät, hergestellt werden.

Für das erfindungsgemäße Verfahren vorteilhafte Nukleinaauremolokole können auf der Grundlage ihrer Homologie zu den hier offenbarten Nukleinsäuren unter Verwendung der Sequenzen oder eines Teils davon als Hybridisierungssonde gemäß Standard-Hybridisierungstechniken unter stringenten Hybridisierungsbedingungen isoliert werden. Dabei können beispielsweise isolierte Nukleinsäuremoleküle verwendet werden, die mindestens 15 Nukleotide lang sind und unter stringenten Bedingungen mit dem Nukleinsäuremolekülen, die eine Nukleotidsequenz der SEQ ID NO: 1, SEQ ID NO: 11, SEQ ID NO: 13, SEQ ID NO: 15, SEQ ID NO: 17. SEQ ID NO: 19, SEQ ID NO: 21, SEQ ID NO: 23 oder SEQ ID NO: 25 umfassen, hybridisieren. Es können auch Nukleinsäuren mindestens 25, 50, 100, 250 oder mehr Nukleotide verwendet werden. Der Begriff "hybridisiert unter stringenten Bedingungen", wie hier verwendet, soll Hybridisierungs-und Waschbedingungen beschreiben, unter denen Nukleofidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, stärker bevorzugt mindestens etwa 70 % und noch stärker bevorzugt mindestens etwa 75 % oder stärker zueinander homolog sind, gewöhnlich aneinander hybridisiert bleiben. Unter Homolog oder Homologie ist im Sinne der Erfindung identisch oder Identität zu verstehen. Diese stringenten Bedingungen sind dem Fachmann bekannt und lassen sich in Current Protocols in Molecular Biology, John Wiley & Sons, N. Y. (1989), 6.3.1-6.3,6., finden. Ein bevorzugtes, nicht einschränkendes Beispiel für stringente Hybridisierungsbedingungen sind Hybridisierungen in 6 x Natriumchlorid/Natriumcitrat (sodium chloride/sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure- und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42°C. Vorzugsweise sind die Hybridisterungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt Der Fachmann weiß, wie die erforderlichen Hybrldisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford: Brown (Hrsgb.) 1991. "Essential Molecular Biology: A Practical Approach°, IRL Press at Oxford University Press, Oxford, bestimmt werden können.

Zur Bestimmung der prozentualen Homologie (= Identität) von zwei Aminosäuresequenzen (z.B. der SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO: 10) oder von zwei Nukleinsäuren (z.B. der Sequenzen SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25) werden die Sequenzen zum Zweck des optimalen Vergleichs untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste öder Nukleotide an den entsprechenden Aminosäurepositionen oder Nukfeotidpositionen werden dann verglichen. Wenn eine Position in einer Sequenz durch den gleichen Aminosäurerest oder das gleiche Nukleotid wie die entsprechende Stelle In der anderen Sequenz belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der Identischen Positionen/Gesamtanzahl der Positionen x 100). Die Begriffe Homologie und Identität sind damit als Synonym anzusehen.

Ein isoliertes Nukleinsäuremolekül, das für eine Threomin-Aldoalse oder Lysin-Decarboxylase kodiert, die zu einer Proteinsequenz der SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO, 24 oder SEQ ID NO: 26 oder den Sequenzen SEQ ID NO: 3, SEQ ID NO: 4, SEQ ID NO: 5, SEQ ID NO: 6, SEQ ID NO: 7. SEQ ID NO: 8, SEQ ID NO: 9 oder SEQ ID NO: 10 homolog ist, kann durch Einbringen einer oder mehrerer Nukleotidsubstitutionen, -additionen oder -deletionen In eine Nukleotidsequenz der SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder in die den von den vorgenannten Aminosäuresequenzen abgeleiteten Nukleinsäuresequenzen erzeugt werden, so dass eine oder mehrere AminosAuresubstitutionen, -additionen oder-deletionen in das kodierte Protein eingebracht werden. Mutationen können in eine der Sequenzen der SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 durch Standardtechniken, wie stellenspezifische Mutagenese und PCR-vermittelte Mutagenese, eingebracht werden. Vorzugsweise werden konservative Aminosäuresubstitutionen an einer oder mehreren der vorhergesagten nicht-essentiellen Aminosäureresten hergestellt Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.8. Asparaginsäure. Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), betaverzweigten Seitenketten (z.B. Threonin, Valin, Isoleucin) und aromatischen Seitenketten (z.B. Tyrosin, Phenylalanin, Tryptophan. Histidin). Ein vorhergesagter nicht-essentieller Aminosaurerest in einer Proteinsequenz wie SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO:10, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der kodierenden Sequenz eingebracht werden, zB. durch Sättigungsmutagenese, und die resultierenden Mutanten können auf ihre biologische Aktivität das heißt die Aminoseureproduktion hin durchmustert werden, um Mutanten zu identifizieren, die die biologische Aktivität beibehalten bzw erhöht haben. Nach der Mutagenese einer der Sequenzen der SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder der von den vorgenannten Sequenzen ableitbaren Nukleinsäuresequenz kann das kodierte Protein rekombinant exprimiert werden, und die Aktivität des Proteins kann z.B. unter Verwendung der hier beschriebenen Tests bestimmt werden.

Homologe der verwendeten Nukleinsäuresequenzen mit der Sequenz SEQ ID NO; 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder der von den Sequenzen SEQ ID NO: 3, SEQ ID NO: 4, SEQ ID NO: 5, SEQ ID NO: 6. SEQ ID NO: 7, SEQ ID NO: 8, SEQ ID NO: 9 oderSEQ ID NO: 10 abgeleiteten Nukleinsäuresequenzen bedeuten beispielsweise allelische Varianten mit mindestens etwa 30 bis 50 %, vorzugsweise mindestens etwa 50 bis 70 %, stärker bevorzugt mindestens etwa 70 bis 80 %, 80 bis 90 % oder 90 bis 95 % und noch stärker bevorzugt mindestens etwa 95 %, 96 %, 97 %. 98 %, 99 % oder mehr Homologie zu einer in SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 gezeigten Nukleotidsequenzen oder den vorgenannten abgeleiteten Nukleinsäuresequenzen oder ihren Homologen, Derivaten oder Analoga oder Teilen davon. Weiterhin sind isolierte Nukleinsäuremoleküle einer Nukleotidsequenz, die an eine der in SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 gezeigten Nukleotidsequenzen, den abgeleiteten Nukleinsäuresequenzenoder einen Teil davon hybridisieren, z.B. unter stringenten Bedingungen hybridisiert Allelische Varianten umfassen insbesondere funktionelle Varianten, die sich durch Deletion, Insertion oder Substitution von Nukleotiden aus/in der in SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 dargestellten Sequenz oder den abgeleiteten Nukleinsäuresequenzen erhalten lassen, wobei aber die Absicht ist, dass die Enzymaktivität bzw. die biologische Aktivität der davon herrührenden synthetisierten Proteine für die Insertion eines oder mehrerer Gene vorteilhafterweise beibehalten wird. Proteine, die noch die wesentliche enzymatische Aktivität der Threonin-Aldolase besitzen, das heißt deren Aktivität im wesentlichen nicht reduziert ist, bedeutet Proteine mit mindestens 10 %, vorzugsweise 20 %, besonders bevorzugt 30 %, ganz besonders bevorzugt 40 % der ursprünglichen biologischen bzw. Enzym-Aktivität, vorteilhaft verglichen mit dem durch SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24 oder SEQ ID NO: 26 kodierten Protein.

Homologen der SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder der abgeleiteten Sequenzen bedeuten beispielsweise auch bakterielle, Pilz- und Pflanzenhomologen, verkürzte Sequenzen, einzelsträngige DNA oder RNA der kodierenden und nicht-kodierenden DNA-Sequenz.

Unter Homologen der SEQ ID NO: 1, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 oder der abgeleiteten Sequenzen sind auch Derivate, wie beispielsweise Promotorvarianten, zu verstehen. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Die vorgenannten Nukleinsäuren und Proteinmoleküle mit Threonin-Aldolase-Aktivitat und/oder Lysin-Decarboxytase-Aktivität, die am Stoffwechsel von Aminosäuren beteiligt sind, werden zur Steigerung der Ausbeute, Produktion und/oder Effizienz der Produktion einer gewünschten Verbindung oder einer Abnahme unerwünschter Verbindungen verwendet

Die im erfindungsgemäßen Verfahren verwendeten Organismen werden je nach Wirtsorganismus in dem Fachmann bekannter Weise angezogen bzw. gezüchtet Mikroorganismen werden in der Regel in einem flüssigen Medium, das eine Kohlenstoffquelle meist In Form von Zuckern, eine Stickstoffquelle meist in Form von organischen Stickstoffquellen wie Hefeextrakt oder Salzen wie Ammoniumsulfat Spurenelemente wie Eisen-, Mangan-, Magnesiumsalze und gegebenenfalls Vitamine enthält, bei Temperaturen zwischen 0 °C und 100 °C, bevorzugt zwischen 10 °C bis 60 °C unter Sauerstoffbegasung angezogen. Dabei kann der pH der Nährflüssigkeit auf einen festen Wert gehalten werden, das heißt während der Anzucht reguliert werden oder nicht. Die Anzucht kann batch weise, semi batch weise oder kontinuierlich erfolgen. Nährstoffe können zu Beginn der Fermentation vorgelegt oder semikonbnuierlich oder kontinuierlich nachgefüttert werden. Die hergestellten Aminosäuren können nach dem Fachmann bekannten Verfahren aus den Organismen isoliert werden. Beispielsweise über Extraktion, Salzfällung und/oder lonenaustauschchromatographie. Die Organismen können dazu vorher noch aufgeschlossen werden.

Das erfindungsgemäße Verfahren wird, wenn es sich bei den Wirtsorganismen um Mikroorganismen handelt, vorteilhaft bei einer Temperatur zwischen 0 °C bis 95 °C. bevorzugt zwischen 10 °C bis 85 °C, besonders bevorzugt zwischen 15 °C bis 75 °C, ganz besonders bevorzugt zwischen 15 °C bis 45 °C durchgeführt

Der pH-Wert wird dabei vorteilhaft zwischen pH 4 und 12, bevorzugt zwischen pH 6 und 9, besonders bevorzugt zwischen pH 7 und 8 gehalten.

Das erfindungsgemäße Verfahren kann batchweise, semi-batchweise oder kontinuierlich betrieben werden. Eine Zusammenfassung über bekannte Kultivierungsmethoden Ist im Lehrbuch von Chmiel (Bloprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschwelg/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen wie oben beschrieben gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-. Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl, Sonnenblumenöl, Erdnussöl und/oder Kokosfett, Fettsäuren wie z. B. Palmitinsäure, Stearinsäure und/oder Linolsäure, Alkohole und/oder Polyalkohole wie z. 8. Glycerin, Methanol und/oder Ethanol und/oder organische Säuren wie z. B. Essigsäure und/oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak in flüssiger- oder gasform oder Ammoniumsalze, wie Ammoniumsulfat, Ammonlumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle für die Herstellung von schwefelhaltigen Feinchemikalien, insbesondere von Methionin, können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Chelatbildner können zum Medium gegeben werden, um die Metallionen In Lösung zu halten. Besonders geeignete Chelatbildner umfassen Dihydroxyphenole, wie Catechol oder Protocatechuat, oder organische Säuren, wie Citronensäure.

Die erfindungsgemäß zur Kultivierung von Mikroorganismen eingesetzten Fermentatlonsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt. Melassen, Maisquellwasser und derglelchen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Apptied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121 °C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bel 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder sauren Verbindungen wie Phosphorsäure oder Schwefelsäure kontrollieren. Zur Kontrolle der Schaumentwicklung können Antischaummittel wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff haltige Gasmischungen, wie z. B. Umgebungsluft, in die Kultur eingetragen. Die Temperatur der Kultur liegt normalerweise bel 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen, insbesondere L-Methionin und/oder L-Lysin vorteilhaft L-Methionin enthalteriden, Fermentationsbrühen haben üblicherweise eine Trockenmasse von 7,5 bis 25 Gew.-%.

Vorteilhaft ist außerdem auch, wenn die Fermentation zumindest am Ende, insbesondere jedoch über mindestens 30% der Fermentationsdauer zuckerlimitiert gefahren wird. Das heißt, dass während dieser Zeit die Konzentration an verwertbarem Zucker im Fermentationsmedium auf ≥ 0 bis 3 g/l gehalten, beziehungsweise abgesenkt wird.

Die Fermentationsbrühe wird anschließend weiterverarbeitet Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Anschließend kann die Fermentationsbrühe mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann anschließend durch Gefriertrocknung, Sprühtrocknung, Sprühgranulation oder durch anderweitige Verfahren aufgearbeltet werden.

Es ist aber auch möglich die Aminosäure weiter aufzureinigen. Hierzu wird die produkthaltige . Brühe nach dem Abtrennen der Biomasse einer Chromatographie mit einem geeigneten Harz unterworfen, wobei das gewünschte Produkt oder die Verunreinigungen ganz oder teilweise auf dem Chromatographieharz zurückgehalten werden. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung bewandert. Das gereinigte Produkt kann durch Filtration oder Ultrafiltration konzentriert und bel einer Temperatur aufbewahrt werden, bei der die Stabilität des Produktes maximal ist.

Die Identität und Reinheit der isolierten Verbindung(en) kann durch Techniken des Standes der Technik bestimmt werden. Diese umfassen Hochleistungs-Flüssigkeitschromatographie (HPLC), spektroskopische Verfahren, Massenspektrometrie, Farbeverfahren. Dünnschichtchromatographie, NIRS, Enzymtest oder mikrobiologische Tests. Diese Analyseverfahren sind zusammengefasst in: Patek et al. (1984) Appl. Environ. Microbiol. 60:133-140; Malakhova et al. (1996) Biotekhnologiya 11 27-32: und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

Die im Verfahren gewonnenen Aminosäuren eignen sich als Ausgangsmaterial für die Synthese von weiteren Wertprodukten. Sie können beispielsweise in Kombination miteinander oder allein zur Herstellung von Pharmaka, Nahrungsmittel, Tierfutter oder Kosmetika verwendet werden.

Die Übertragung von Fremdgenen in das Genom einer Pflanze wird wie oben beschrieben als Transformation bezeichnet. Es werden dabei die beschriebenen Methoden zur Transformation und Regeneration von Pflanzen aus Pflanzengeweben oder Pflanzenzellen zur transienten oder stabilen Transformation genutzt. Geeignete Methoden sind die Protoplastentransformation durch Polyethylenglykol-induzierte DNA-Aufnahme, das biolistische Verfahren mit der Genkanone - die sogenannte particle bombardment Methode, die Elektroporation, die Inkubation trockener Embryonen in DNA-haltiger Lösung, die Mikroinjektion und der durch Agrobacterium vermittelte Gentransfer. Die genannten Verfahren sind beispielsweise in B. Jenes et al., Techniques for Gene Transfer, in: Transgenic Plants, Vol. 1, Engineering and Utilization, herausgegeben von S.D. Kung und R. Wu, Academic Press (1993) 128-143 sowie in Potrykus Annu. Rev. Plant Physiol. Plant Molec Biol. 42 (1991) 205-225) beschrieben. Vorzugsweise wird das zu exprimierende Konstrukt in einen Vektor kloniert, der geeignet ist, Agrobacterium tumefaciens zu transformieren, beispielsweise pBin19 (Bevan et al., Nucl. Acids Res. 12 (1984) 8711). Mit einem solchen Vektor transformierte Agrobakterien können dann in bekannter Weise zur Transformation von Pflanzen, insbesondere von Kulturpflanzen, wie z.B. von Tabakpflanzen, verwendet werden, indem beispielsweise verwundete Blätter oder Blattstücke in einer Agrobakterienlösung gebadet und anschließend In geeigneten Medien kultiviert werden. Die Transformation von Pflanzen mit Agrobacterium tumefaciens wird beispielsweise von Höfgen und Willmitzer in Nucl. Acid Res. (1988) 16, 9877 beschrieben oder ist unter anderem bekannt aus F.F. White, Vectors for Gene Transfer in Higher Plants; in Transgenic Plants, Vol. 1, Engineering and Utilization, herausgegeben von S.D. Kung und R. Wu, Academic Press, 1993, S. 15-38.

Für die Selektion einer erfolgreichen Einbringung der erfindungsgemäßen Nukleinsäuren in einen Wirtsorganismus werden vorteilhaft Markergene verwendet. Diese Markergene ermöglichen die identifizierung einer erfolgreichen Einbringung der erfindungsgemäßen Nukleinsäuren über ein Reihe verschiedener Prinzipien beispielsweise über eine visuelle Erkennung mit Hilfe von Fluoreszenz, Lumineszenz oder im für den Mensch sichtbaren Wellenbereich des Lichtes, über eine Herbizid-oder Antibiotikaresistenz, über sogenannte nutritive (Auxotrophiemarker) oder anti-nutritive Marker, über Enzymassays oder über Phytohormone. Als Beispiele für derartige Maker seien hier das GFP (= Green fluorescent Protein); das Luciferin/Luceferacesystem; die β-Galactosidase mit ihren farbigen Substraten z.B. X-Gal; die Herbizideresistenzen gegen z.B. Imidazolinon, Glyphosat, Phosphothrlcin oder Sulfonylharnstoff, die Antibiotikaresistenzen gegen z.B. Bleomycin, Hygromycin, Streptomycin, Kanamycin, Tetracyclin, Chloramphenicol, Ampicillin, Gentamycin, Geneticin (G418), Spectinomycin oder Blasticidin um nur einige zu nennen; nutritive Marker wie die Mannose- oder Xyloseverwertung oder anti-nutritive Marker wie 2-Deoxyglukoseresistenz genannt. Diese Liste gibt einen kleinen Ausschnitt möglicher Marker wieder. Dem Fachmann sind derartige Marker wohl bekannt. Je nach Organismus und Selektionsmethode sind unterschiedliche Marker bevorzugt

Über die stabile oder transiente Integration von Nukleinsäuren in Pflanzenzellen ist bekannt, dass je nach verwendetem Expressionsvektor und verwendeter Transfektionstechnik nur ein kleiner Teil der Zellen die fremde DNA aufnimmt und falls gewünscht in ihr Genom integriert. Zur Identifikation und Selektion dieser Integranten wird gewöhnlich ein Gen, das einen selektierbaren Marker (z.B. Resistenz gegen Antibiotika) kodiert, zusammen mit dem Gen von Interesse in die Wirtszellen eingebracht Bevorzugte selektierbare Marker umfassen in Pflanzen solche, welche Resistenz gegen ein Herbizid, wie Glyphosphat oder Glufosinat, verleihen. Weitere geeignete Marker sind beispielsweise Marker, welche Gene kodieren, die an Biosynthesewegen von zum Beispiel Zuckern oder Aminosäuren beteiligt sind, wie ß-Galactodsidase, ura3 oder ilv2. Marker, welche Gene, wie Luziferase, gfp oder andere Fluoreszenzgene kodieren, sind ebenfalls geeignet. Diese Marker lassen sich in Mutanten verwenden, in denen diese Gene nicht funktionell sind, da sie beispielsweise mittels herkömmlicher Verfahren deletiert worden sind. Ferner können Marker, welche eine Nukleinsäure kodieren, die einen selektierbaren Marker kodiert, in eine Wirtszelle auf dem gleichen Vektor eingebracht werden, wie derjenige, der für die im Verfahren verwendeten Threonin-Aldolasen und/oder Lysin-Decarboxylasen kodiert, oder können auf einem gesonderten Vektor eingebracht werden. Zellen, die mit der eingebrachten Nukleinsäure stabil transfiziert worden sind, können zum Beispiel durch Selektion identifiziert werden (z.B. überleben Zellen, die den selektierbaren Marker integriert haben, wohingegen die anderen Zellen absterben).

Da die Markergene in der Regel speziell die Antibiotika- und Herbizidresistenzgen in der transgenen Wirtszelle nach erfolgreicher Einbringung der Nukleinsäuren nicht mehr benötigt werden bzw. unerwünscht sind, werden im erfindungsgemäßen Verfahren zur Einbringung der Nukleinsäuren vorteilhaft Techniken verwendet, die eine Entfernung bzw. Exzision dieser Markergene ermöglicht. Eine derartige Methode ist die sogenannte Co-Transformation. Bei der Co-Transformation werden zur Transformation zwei Vektoren gleichzeitig verwendet, wobei ein Vektor die erfindungsgemäßen Nukleinsäuren und ein zweiter das oder die Markergene trägt. Ein hoher Teil der Transformanten erhält bzw. enthält bei Pflanzen (bis zu 40 % der Transformanten und mehr) beide Vektoren. Durch Kreuzung lassen sich anschließend die Markergene aus der transformierten Pflanze entfernen. Eine weitere Methode verwendet Markergene, die in ein Transposon integriert wurden, zur Transformation zusammen mit den gewünschten Nukleinsäuren (sog. Ac/Ds-Technologie). In einigen Fällen (ca. 10 %) springt das Transposon nach erfolgreicher Transformation aus dem Genom der Wirtszelle und geht verloren. In eine weiteren Anzahl von Fällen springt das Transposon an eine andere Stelle. In diesen Fällen muss das Markergen wieder ausgekreuzt werden. In der Mikrobiologie wurden Techniken entwickelt. die eine Detektion derartiger Ereignisse ermöglicht bzw. erleichtert. Eine weitere vorteilhafte Methode verwendet sogenannte Rekombinationssysteme, die den Vorteil haben, dass auf eine Auskreuzung verzichtet werden kann. Das bekannteste derartige System ist das sogenannte Cre/lox-System. Cre1 ist eine Rekombinase, die die Sequenzen, die zwischen der loxP-Sequenz liegen, entfernt. Wird das Markergen zwischen die loxP-Sequenz integriert, so wird es nach erfolgreicher Transformation durch Expression der Rekombinase entfernt. Weitere Rekombinasesysteme sind das HIN/HIX-, das FLP/FRT- und das REP/STB-System (Tribble et al., J.Biol. Chem., 275, 2000: 22255 - 22267; Velmurugan et al., J. Cell Biol., 149, 2000: 553 - 566). Auch eine gezielte Integration der erfindungsgemäßen Nukleinsäuresequenzen in das Pflanzengenom ist prinzipiell möglich, aber aufgrund des hohen Arbeitsaufwandes weniger bevorzugt. Diese Methoden sind natürlich auch bei Mikroorganismen wie Hefen, Pilze oder Bakterien anwendbar.

Mit einem erfindungsgemäßen Expressionsvektor transformierte Agrobakterien können ebenfalls in bekannter Weise zur Transformation von Pflanzen wie Testpflanzen wie Arabidopsis oder Kulturpflanzen wie beispielsweise Getreide, Mais, Hafer, Roggen, Gerste, Weizen, Soja, Reis, Baumwolle, Zuckerrübe, Canola, Sonnenblume, Flachs, Hanf, Kartoffel, Tabak, Tomate, Karotte, Paprika, Raps, Tapioka, Manlok, Pfeilwurz, Tagetes, Alfalfa, Salat und den verschiedenen Baum-, Nuß- und Weinspezies, insbesondere von Öl-haltigen Kulturpflanzen, wie Soja, Erdnuß, Rizinus, Sonnenblume, Mais, Baumwolle, Flachs, Raps, Kokosnuß, Ölpalme, Färbersaflor (Carthamus tinctorius) oder Kakaobohne verwendet werden, z.B. indem verwundete Blätter oder Blattstücke in einer Agrobakterienlösung gebadet und anschließend in geeigneten Medien kultiviert werden.

Die genetisch veränderten Pflanzenzellen können über alle dem Fachmann bekannten Methoden regeneriert werden. Entsprechende Methoden können den oben genannten Schriften von S.D. Kung und R. Wu, Potrykus oder Höfgen und Willmitzer entnommen werden.

Neben der Transformation von somatischen Zellen, welche dann zu Pflanzen regeneriert werden müssen, können auch Zellen pflanzlicher Meristeme und insbesondere solche Zellen, die sich zu Gameten entwickeln, transformlert werden. In diesem Fall führen die transformierten Gameten auf dem Weg der natürlichen Pflanzenentwicklung zu transgenen Pflanzen. So werden beispielsweise Samen von Arabidopsis mit Agrobakterien behandelt und von den sich daraus entwickelnden Pflanzen Samen gewonnen, die dann zu einer bestimmten Rate transformiert, daher transgen sind (Feldman, KA und Marks MD (1987), Agrobacterium-mediated transformation of germinating seeds of Arabidopsis thaliana: a non tissue culture approach. Mol Gen Genet 208:274-289; Feldmann K (1992) T-DNA insertion mutagenesis in Arabidopsis: seed infection transformation. In C Koncz, N-H Chua und J Shell, eds, Methods in Arabidopsis Research. Word Scientific, Singapore, pp274-289). Alternative Methoden beruhen auf der wiederholten Entfernung der Infloreszenzen und der Inkubation der Schnittstelle im Zentrum der Rossette mit transformierten Agrobakterien, wodurch ebenfalls später transformierte Samen gewonnen werden können (Chang, SS. Park SK, Kim, BC, Kang, BJ, KimDU und Nam, HG (1994) Stable genetic transformation of Arabidopsis thaliana by Agrobacterium inoculation in planta. Plant J. 5: 551-558; Katavic, V, Haughn, GW, Reed, D, Martin, M und Kunst, L (1994) In planta transformation of Arabidopsis thaliana. Mol Gen Genet, 245: 363-370). Besonders effizient ist jedoch die Methode der Vakuum-Infiltration mit ihren Abwandlungen wie "Floral dip". Bei der Vakuuminfiltration von Arabidopsis werden ganze Pflanzen unter Vakuum mit einer Agorbakteriumsuspension behandelt (Bechthold, N, Ellis, J, und Pelletier, G (1993) In planta Agrobacterium-mediated gene transfer by infiltration of adult Arabidopsis thalina plants. C R Acad Sci Paris Life Sci, 316: 1194-1199), während bei der "Floral dip"-Methode das sich entwickelnde Blütengewebe in einer mit einem Surfactant versetzten Agrabakteriumsuspension kurzzeitig inkubiert wird (Clough, SJ und Bent, AF (1998) Floral dip: a simple method for Agrobacterium-mediated transformation of Arabidopsis thaliana. The Plant J. 16, 735-743). In beiden Fällen werden zu einem bestimmten Prozentsatz transgene Samen geerntet, die durch Anzucht unter den bereits beschriebenen selektiven Bedingungen von nicht transgenen Samen unterschieden werden können.

Ein weiterer Aspekt der Erfindung betrifft deshalb transgene Organismen, transformiert mit wenigstens einer erfindungsgemäßen Nukleinsäuresequenz, Expressionskassette oder einem erfindungsgemäßen Vektor, sowie Zellen, Zellkulturen, Gewebe, Teile -wie zum Beispiel bei pflanzlichen Organismen Blätter, Wurzeln usw.- oder Vermehrungsgut abgeleitet von solchen Organismen. Die Begriffe "Wirtsorganismus" "Wirtszelle", "rekombinanter (Wirts)organismus", "rekombinante (Wirts)zelle", "transgener (Wirts)organismus" und "transgene (Wirts)zelle" werden hier untereinander austauschbar verwendet. Selbstverständlich betreffen diese Begriffe nicht nur den bestimmten Wirtsorganismus oder die bestimmte Zielzelle, sondern auch die Nachkommen oder potentiellen Nachkommen dieser Organismen bzw. Zellen. Da in aufeinanderfolgenden Generationen aufgrund von Mutation oder Umwelteinflüssen bestimmte Modifikationen auftreten können, sind diese Nachkommen nicht unbedingt mit der Parentalzelle identisch, sind jedoch immer noch vom Umfang des Begriffs, wie hier verwendet, umfasst.

Ein anderer Aspekt der Erfindung sind die in SEQ ID NO: 3, SEQ ID NO: 4, SEQ ID NO: 5. SEQ ID NO: 6, SEQ ID NO: 7, SEQ ID NO: 8, SEQ ID NO: 9 oder SEQ ID NO: 10 genannten Aminosauresequenzen.

Diese Erfindung wird durch die nachstehenden Beispiele weiter veranschaulicht die nicht als beschränkend aufgefasst werden sollten. Der Inhalt sämtlicher in dieser Patentanmeldung zitierten Literaturstellen, Patentanmeldungen, Patente und veröffentlichten Patentanmeldungen ist hier durch Bezugnahme aufgenommen.

### Beispiele:

### Beispiel 1: Klonierung von SEQ ID NO: 1 in Escherichia coli

SEQ ID NO: 1 wurde gemäß bekannter und gut eingeführter Verfahren (siehe bspw. Sambrook, J. et al. (1989) "Molecular Cloning: A Laboratory Manual". Cold Spring Harbor Laboratory Press oder Ausubel, F.M. et al. (1994) "Current Protocols in Molecular Biology", John Wiley & Sons) in die Plasmide pBR322 (Sutcliffe, J.G. (1979) Proc. Natl Acad. Sci. USA, 75: 3737-3741); pACYC177 (Change & Cohen (1978) J. Bacteriol. 134: 1141-1156); Plasmide der pBS-Reihe (pBSSK+, pBSSK- und andere; Stratagene, LaJolla, USA) oder Cosmide, wie SuperCos1 (Stratagene, LaJolla, USA) oder Lorist6 (Gibson, T.J. Rosenthal, A., und Waterson, R.H. (1987) Gene 53: 283-286) zur Expression In E. coli kloniert.

Analog wurden die Sequenzen, SEQ ID NO: 11, SEQ ID NO: 13, SEQ ID NO: 15, SEQ ID NO: 17, SEQ ID NO: 19, SEQ ID NO: 21, SEQ ID NO: 23 oder SEQ ID NO: 25 cloniert.

### Beispiel 2: DNA-Sequenzierung und Computer-Funktionsanalyse

Die DNA-Sequenzierung wurde gemäß Standard-Verfahren, insbesondere dem Kettenabbruchverfahren mit ABI377-Sequenziermaschinen (s. z.B. Fleischman, R.D. et al. (1995) "Wholegenome Random Sequencing and Assembly of Haemophilus Influenzae Rd., Science 269; 496-512) durchgeführt'.

### Beispiel 3: In-vivo-Mutagenese

In vivo-Mutagenese von Corynebacterium glutamicum kann durchgeführt werden, indem eine Plasmid- (oder andere Vektor-) DNA durch E. coli oder andere Mikroorganismen (z.B. Bacillus spp. oder Hefen, wie Saccharomyces cerevisiae) geleitet wird, die die Integrität ihrer genetischen Information nicht aufrechterhalten können. Übliche Mutatorstämme weisen Mutationen in den Genen für das DNA-Reparatursystem auf [z.B., mutHLS, mutD. mutT, usw., zum Vergleich siehe Rupp. W.D. (1996) DNA repair mechanisms in Escherichia coli and Salmonella, S. 2277-2294, ASM: Washington]. Diese Stämme sind dem Fachmann bekannt Die Verwendung dieser Stämme ist bspw. in Greener, A. und Callahan, M. (1994) Strategies 7; 32-34 veranschaulicht.

### Beispiel 4: DNA-Transfer zwischen Escherichia coli und Corynebacterium glutamicum

Mehrere Corynebacterium- und Brevibacterium-Arten enthalten endogene Plasmide (wie bspw. pHM1519 oder pBL1) die autonom replizieren (für einen Überblick siehe bspw. Martin, J.F. et al. (1987) Biotechnology 5: 137-146). Shuttle-Vektoren für Escherichia coli und Corynebacterium glutamicum lassen sich leicht mittels Standard-Vektoren für E. coli konstruieren (Sambrook, J. et al., (1989), "Molecular Cloning: A Laboratory Manual", Cold Spring Harbor Laboratory Press oder Ausubel, F.M. et al. (1994) "Current Protocols in Molecular Biology", John Wiley & Sons), denen ein Replikationsursprung für und ein geeigneter Marker aus Corynebacterium glutamicum beigegeben wird. Solche Replikationsursprünge werden vorzugsweise von endogenen Plasmiden entnommen, die aus Corynebacterium- und Brevibactertium-Arten isoliert worden sind. Besondere Verwendung als Transformationsmarker für diese Arten sind Gene für Kanamycin-Resistenz (wie solche, die vom Tn5- oder Tn-903-Transposon stammen) oder für Chloramphenicol (Winnacker, E.L. (1987) "From Genes to Clones - Introduction to Gene Technology, VCH, Weinheim). Es gibt zahlreiche Beispiele in der Literatur zur Herstellung einer großen Vielzahl von Shuttle-Vektoren, die in E. coli und C. glutamicum repliziert werden, und die für verschiedene Zwecke verwendet werden können, einschließlich Gen-Überexpression (siehe bspw. Yoshihama, M. et al. (1885) J. Bacteriol. 162: 591-597, Martin, J.F. et al., (1987) Biotechnology, 5: 137-146 und Eikmanns, B.J. et al. (1992) Gene 102: 93-98). Geignete Vektoren, die in coryneformen Bakterien replizieren, sind beispielsweise pZ1 (Menkel et al., Appl. Environ. Microbiol., 84, 1989: 549 - 554) pEkEx1 (Eikmanns et al., Gene 102, 1991: 93 - 98) oder pHS2-1 (Sonnen et al, Gene 107, 1991: 69 - 74). Diese Vektoren beruhen auf den krytischen Plasmiden pHM1519, pBL1 oder pGA1. Andere Plasmidvektoren wie zum Beispiel solche, die auf pCG4 (US 4,489,160), pNG2 (Serwold-Davis et al., FEMS Microbiol. Lett., 66, 1990: 119 -124) oder pAG1 (US 5,158,891) beruhen, können in gleicher Weise verwendet werden.

Mittels Standard-Verfahren ist es möglich, ein Gen von Interesse in einen der vorstehend beschriebenen Shuttle-Vektoren zu klonieren und solche Hybrid-Vektoren in Corynebacterium glutamicum-Stämme einzubringen. Die Transformation von C. glutamicum lässt sich durch Protoplastentransformation (Kastsumata, R. et al., (1984) J. Bacteriol. 159, 306-311), Elektroporation (Liebl, E. et al., (1989) FEMS Microbiol. Letters, 53: 399-303) und in Fallen, bei denen spezielle Vektoren verwendet werden, auch durch Konjugation erzielen (wie z.B. beschrieben in Schäfer, A., et (1990) J. Bacteriol. 172: 1663-1666). Es ist ebenfalls möglich, die Shuttle-Vektoren für C. glutamicum auf E. coli zu übertragen, indem Plasmid-DNA aus C. glutamicum (mittels im Fachgebiet bekannter Standard-Verfahren) präpariert wird und in E. coli transformiert wird. Dieser Transformationsschritt kann mit Standard-Verfahren erfolgen, jedoch wird vorteilhafterweise ein Mcr-defizienter E. coli-Stamm verwendet, wie NM522 (Gough & Murray (1983) Mol. Biol. 166: 1-19).

Soll vorteilhaferweise die Integration der transformierten Sequenz(en) in das Genom der coryneformen Bakterien erfolgen, so sind auch hierfür dem Fachmann Standardtechniken bekannt Hierfür werden beispielsweise Plasmidvektoren verwendet, wie sie von Remscheid et al. (Appl. Environ. Microbiol., 60, 1994: 126 -132) zur Duplikarion bzw. Ampilfikation des hom-thrB-Operons beschrieben wurden. Bei dieser Methode wird das vollständige Gen in einen Plasmidvektor kloniert, der in einem Wirt wie E. coli, nicht aber in C. glutamicum replizieren kann. Als Vektoren kommen beispielsweise pSUP301 (Simon et al., Bio/Technology 1, 1983: 784 - 791), pKIBmob oder pK19mob (Schäfer et al., Gene 145. 1994: 69 - 73), pGEM-T (Promega Corp., Madison, WI, USA), pCR2.1-TOPO (Schuman, J. Biol. Chem., 269, 1994: 32678 - 32684, US 5,487,993), pCR^{®}Blunt (Firma Invitrogen, Groningen, Niderlande) oder pEM1 (Schrumpf et al., J. Bacteriol., 173, 1991: 4510 - 4516) in Frage.

### Beispiel 5: Bestimmung der Expression des mutanten/transgenen Proteins

Die Beobachtungen der Aktivität eines mutierten bzw. transgenen Proteins in einer transformierten Wirtszelle beruhen auf der Tatsache, dass das Protein auf ähnliche Weise und in ähnlicher Menge exprimiert wird wie das Wildtyp-Protein. Ein geeignetes Verfahren zur Bestimmung der Transkriptionsmenge des mutanten bzw. transgenen Gens (ein Anzeichen für die mRNA-. Menge, die für die Translation des Genprodukts verfügbar ist) ist die Durchführung eines Northem-Blots (s. bspw. Ausubel et al., (1988) Current Protocols in Molecular Biology, Wiley: New York), wobei ein Primer, der so ausgestaltet ist, dass er an das Gen von Interesse bindet, mit einer nachweisbaren (gewöhnlich radioaktiven oder chemilumineszierenden) Markierung versehen wird, so dass - wenn die Gesamt-RNA einer Kultur des Organismus extrahiert, auf einem Gel aufgetrennt, auf eine stabile Matrix übertragen und mit dieser Sonde inkubiert wird - die Bindung und die Quantität der Bindung der Sonde das Vorliegen und auch die Menge von mRNA für dieses Gen anzeigt Diese information ist ein Nachweis für das Ausmaß der Transkription des Gens. Gesamt-Zell-RNA lässt sich durch verschiedene Verfahren aus Corynebacterium glutamicum isolieren, die im Fachgebiet bekannt sind, wie beschrieben in Bormann, E.R. et al., (1992) Mol. Microbiol. 6: 317-326.

Zur Bestimmung des Vorliegens oder der relativen Menge von Protein, das aus dieser mRNA translatiert wird, können Standard-Techniken, wie Western-Blot, eingesetzt werden (s. bspw. Ausubel et al. (1988) "Current Protocols in Molecular Biology", Wiley, New York). Bei diesem Verfahren werden Gesamt-Zellproteine extrahiert, durch Gelelektrophorese getrennt, auf eine Matrix, wie Nitrocellulose, übertragen und mit einer Sonde, wie einem Antikörper, inkubiert, die an das gewünschte Protein spezifisch bindet. Diese Sonde ist gewöhnlich direkt oder indirekt mit einer chemilumineszierenden oder kolorimetrischen Markierung versehen, die sich leicht nachweisen lässt. Das Vorliegen und die beobachtete Menge an Markierung zeigt das Vorliegen und die Menge des gesuchten Mutantenproteins in der Zelle an.

### Beispiel 6: Wachstum von genetisch verändertem Corynebacterium glutamicum - Medien und Anzuchtbedingungen

Genetisch veränderte Corynebakterien werden in synthetischen oder natürlichen Wachstums. medien gezüchtet. Eine Anzahl unterschiedlicher Wachstumsmedien für Corynebakterien sind bekannt und leicht erhältlich (Lieb et al. (1989) Appl. Microbiol. Biotechnol. 32: 205-210; von der Osten et al. (1998) Biotechnology Letters 11: 11-16; Patent DE 4 120 867: Liebl (1992) "The Genus Corynebacterium", in: The Procaryotes, Bd. II, Balows, A., et al., Hrsg. Springer-Verlag). Diese Medien bestehen aus einer oder mehreren Kohlenstoffquellen, Stickstoffquellen, anorganischen Salzen, Vitaminen und Spurenelementen. Bevorzugte Kohlenstoffquellen sind Zucker, wie Mono-, Di- oder Polysaccharide. Sehr gute Kohlenstoffquellen sind bspw. Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte aus der Zucker-Raffinierung zu den Medien geben. Es kann auch vorteilhaft sein, Gemische verschiedener Kohlenstoffquellen zuzugeben. Andere mögliche Kohlenstoffquellen sind Alkohole und/oder organische Säuren, wie Methanol, Ethanol, Essigsäure oder Milchsäure. Stickstoffquellen sind gewöhnlich organische oder anorganische Stickstoffverbindungen oder Materialien, die diese Verbindungen enthalten. Beispielhafte Stickstoffquellen umfassen Ammoniak-Gas, wässrige Ammoniaklösungen oder Ammoniumsalze, wie NH₄Cl oder (NH₄)₂SO₄, NH₄OH, Nitrate, Harnstoff, Aminosäuren oder komplexe Stickstoffquellen, wie Maisquellwasser, Sojamehl, Sojaprotein, Hefeextrakte, Fleischextrakte und andere. Auch Mischungen der vorgenannten Stickstoffquellen können vorteilhaft verwendet werden.

Anorganische Salzverbindungen, die in den Medien enthalten sein können, umfassen die Chlorid-, Phosphor-, oder Sulfatsalze von Calcium, Magnesium, Natrium, Kobalt, Molybdän, Kalium, Mangan, Zink, Kupfer und Eisen. Chelatbildner können zum Medium gegeben werden, um die Metallionen in Lösung zu halten. Besonders geeignete Chelatbildner umfassen Dihydroxyphenole, wie Catechol oder Protocatechuat oder organische Säuren, wie Citronensäure. Die Medien enthalten üblicherweise auch andere Wachstumsfaktoren, wie Vitamine oder Wachstumsförderer, zu denen bspw. Biotin, Riboflavin, Thiamin, Folsäure, Nikotinsäure, Panthothenat und Pyridoxin gehören. Wachstumsfaktoren und Salze stammen häuflg von komplexen Medienkomponenten, wie Hefeextrakt, Melassen, Maisquellwasser und dergleichen. Die genaue Zusammensetzung der Medienverbindungen hängt stark vom jeweiligen Experiment ab und wird für jeden Fall individuell entschieden. Information über die Medienoptimierung ist beispielsweise erhältlich aus dem Lehrbuch "Applied Microbiol. Physiology, A Practical Approach" (Hrsg. P.M. Rhodes. P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten sind sterilisiert, entweder durch Hitze (20 min bei 1,5 bar und 121 °C) oder durch Sterilfiltration. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Anzuchtbedingungen werden für jedes Experiment gesondert definiert. Die Temperatur sollte zwischen 15°C und 45°C liegen und kann während des Experimentes konstant gehalten oder verändert werden. Der pH-Wert des Mediums sollte im Bereich von 5 bis 8,5, vorzugsweise um 7,0 liegen, und kann durch Zugabe von Puffern zu den Medien aufrechterhalten werden. Ein beispielhafter Puffer für diesen Zweck ist ein Kaliumphosphatpuffer. Synthetische Puffer, wie MOPS, HEPES; ACES usw., können alternativ oder gleichzeitig verwendet werden. Der Anzucht-pH-Wert lässt sich während der Anzucht auch durch Zugabe von z.B. NaOH oder NH₄OH konstant halten. Werden komplexe Medienkomponenten, wie Hefe-Extrakt verwendet, sinkt der Bedarf an zusätzlichen Puffern, da viele komplexe Verbindungen eine hohe Pufferkapazitat aufweisen. Beim Einsatz eines Fermenters für die Anzucht von Mikroorganismen kann der pH-Wert auch mit gasförmigem Ammoniak reguliert werden.

Die Inkubationsdauer liegt gewöhnlich in einem Bereich von mehreren Stunden bis zu mehreren Tagen. Diese Zeit wird so ausgewählt, dass sich die maximale Menge Produkt in der Fermentationsbrühe ansammelt. Die offenbarten Wachstumsexperimente können in einer Vielzahl von Behältern, wie Mikrotiterplatten, Glasröhrchen, Glaskolben oder Glas- oder Metallfermentern unterschiedlicher Größen durchgeführt werden. Zum Screening einer großen Anzahl von Klonen sollten die Mikroorganismen in Mikrokiterplatten, Glasröhrchen oder Schottelkolben entweder mit oder ohne Schikanen gezüchtet werden. Vorzugsweise werden 100-ml-Schüttelkolben verwendet, die mit 10% (bezogen auf das Volumen) des erforderlichen Wachstumsmediums gefüllt sind. Die Kolben sollten auf einem Kreiselschüttler (Amplitude 25 mm) mit einer Geschwindigkeit im Bereich von 100-300 U/min geschüttelt werden. Verdampfungsverluste können durch Aufrechterhalten einer feuchten Atmosphäre verringert werden; alternativ sollte für die Verdampfungsverluste eine mathematische Korrektur durchgeführt werden.

Werden genetisch modifizierte Klone untersucht, sollten auch ein unmodifizierter Kontrollkion oder ein Kontrollklon getestet werden, der das Basisplasmid ohne Insertion enthält Soll eine transgene Sequenz exprimiert werden, so sollte vorteilhafterweise auch in diesem Fall ein Kontroliklon mit getestet werden. Das Medium wird vorteilhaft auf eine OD600 von 0.5 - 1,5 angeimpft, wobei Zellen verwendet werden, die auf Agarplatten gezüchtet wurden, wie CM-Platten (10 g/l Glucose, 2,5 g/l NaCl, 2 g/l Harnstoff, 10 g/l Polypepton, 5 g/l Hefeextrakt, 5 g/l Fleischextrakt, 22 g/l Agar pH-Wert 6,8 mit 2 M NaOH), die bei 30°C inkubiert worden sind. Das Animpfen der Medien erfolgt entweder durch Einbringen einer Kochsalzlösung von C. glutamicum-Zellen von CM-Platten oder durch Zugabe einer flüssigen Vorkultur dieses Bakteriums.

### Beispiel 7: In-vitro-Analyse der Funktion der durch die transformierten Sequenzen codierten Proteine

Die Bestimmung der Aktivitäten und kinetischen Parameter von Enzymen ist im Fachgebiet gut bekannt. Experimente zur Bestimmung der Aktivität eines bestimmten veränderten Enzyms müssen an die spezifische Aktivität des Wildtypenzyms angepasst werden, was innerhalb der Fähigkeiten des Fachmann liegt Überblicke über Enzyme im Allgemeinen sowie spezifische Einzelheiten, die die Struktur, Kinetiken, Prinzipien, Verfahren, Anwendungen und Beispiele zur Bestimmung vieler Enzymaktivitäten betreffen, können bspw. in den nachstehenden Literaturstellen gefunden werden: Dixon, M., und Webb, E.C: (1979) Enzymes, Longmans, London; Fersht (1985) Enzyme Structure and Mechanism, Freeman, New York; Walsh (1979) Enzymatic Reaction Mechanisms. Freeman, San Francisco; Price, N.C., Stevens, L. (1982) Fundamentals of Enzymology. Oxford Univ. Press: Oxford; Boyer, P.D: Hrsg. (1983) The Enzymes, 3. Aufl. Academic Press, New York; Bisswanger, H. (1994) Enzymkinetik, 2. Aufl. VCH, Weinheim (ISBN 3527300325); Bergmeyer, H.U., Bergmeyer, J., Graßl, M. Hrsg. (1983-1986) Methods of Enzymatic Analysis, 3. Aufl. Bd. I-XII, Verlag Chemie: Weinhelm; und Ullmann's Encyclopedia of Industrial Chemistry (1987) Bd. A9, "Enzymes", VCH, Weinheim, S. 352-363.

### Beispiel 8: Analyse des Einflusses der Nukleinsäuren auf die Produktion der Aminosäuren

Die Wirkung der genetischen Modifikation in C. glutamicum auf die Produktion einer Aminosäure kann bestimmt werden, indem die modifizierten Mikroorganismen unter geeigneten Bedingungen (wie solchen, die vorstehend beschrieben sind) gezüchtet werden und das Medium und/oder die zellulären Komponenten auf die erhöhte Produktion der Aminosäure untersucht wird. Solche Analysetechniken sind dem Fachmann wohlbekannt und umfassen Spektroskopie, Massenspektrometrie, Dünnschichtchromatographie, Färbeverfahren verschiedener Art, enzymatische und mikrobiologische Verfahren sowie analytische Chromatographie, wie Hochleistungs-Flüssigkeitschromatographie (s. bspw. Ullman, Encyclopedia of industrial Chemistry, Bd. A2, S. 89-90 und S. 443-613, VCH: Weinheim (1985); Falion, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Beiter. P.A. et al. (1988) Bioseparations: downstream processing for Biotechnology, John Wiley and Sons; Kennedy, J.F. und Cabral, J.M.S. (1992) Recovery processes for biological Materials, John Wiley and Sons; Shaeiwitz, J.A. und Henry, J.D. (1988) Biochemical Separations, in Ullmann's Encyclopedia of Industrial Chemistry, Bd. B3; Kapitel 11, S. 1-27, VCH: Weinhelm; und Dechow, F.J. (1989) Separation and purification techniques in biotechnology, Noyes Publications).

Zusätzlich zur Messung des Fermentationsendproduktes ist es ebenfalls möglich, andere Komponenten der Stoffwechselwege zu analysieren, die zur Produktion der gewünschten Verbindung verwendet werden, wie Zwischen- und Nebenprodukte, um die Gesamt-Produktivität des Organismus, die Ausbeute und/oder die Effizienz der Produktion der Verbindung zu bestimmen. Die Analyseverfahren umfassen Messungen der Nährstoffmengen im Medium (bspw. Zucker. Kohlenwasserstoffe, Stickstoffquellen, Phosphat und andere Ionen), Messungen der Biomassezusammensetrung und des Wachstums, Analyse der Produktion gewöhnlicher Metabolite aus Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsg. IRL Press, S. 103-129; 131-163 und 165-192 (ISBN: 0199635773) und den darin angegebenen Literaturstellen beschrieben.

### Beispiel 9: Reinigung der Aminosäure aus C. glutamicum-Kultur

Die Gewinnung der Aminosäure aus C. glutamicum-Zellen und/oder aus dem Überstand der vorstehend beschriebenen Kultur kann durch verschiedene, im Fachgebiet bekannte Verfahren erfolgen. Hierzu wird zunächst der Kulturüberstand gewonnen, dazu werden die Zellen aus der Kultur durch langsame Zentrifugation geerntet, die Zellen können anschließend durch Standard-Techniken, wie mechanische Kraft oder Ultrabeschallung, zertrümmert bzw. lysiert werden. Die Zelltrümmer werden durch Zentrifugation entfernt, und die Überstandsfraktion, wird zusammen mit dem Kulturüberstand zur welteren Reinigung der Aminosäure genommen. Es kann aber auch der Überstand alleine aufgearbeitet werden, wenn die Konzentration der Aminosäure ausreichend im Überstand enthalten ist Die Aminosäure bzw. das Aminosäuregemisch kann dann weiter über beispielsweise eine Extraktion und/oder Salzfällung oder über eine ionenaustauschchromatographie weiter aufgereinigt werden.

Falls erforderlich und gewünscht können weitere Chromatographieschritte mit einem geeigneten Harz folgen, wobei die Aminosäure entweder auf dem Chromatographieharz zurückgehalten wird, viele Verunreinigungen in der Probe jedoch nicht, oder wobei die Verunreinigungen auf dem Harz zurückbleiben, die Probe mit dem Produkt (Aminosäure) hingegen nicht Diese Chromatographieschritte können nötigenfalls wiederholt werden, wobei die gleichen oder andere Chromatographieharze verwendet werden. Der Fachmann ist in der Auswahl der geeigneten Chromatographieharze und der wirksamsten Anwendung für ein bestimmtes, zu reinigendes Molekül bewandert. Das gereinigte Produkt kann durch Filtration oder Ultrafltration konzentriert und bei einer Temperatur aufbewahrt werden, bei der die Stabilität des Produktes maximal ist Im Fachgebiet sind viele Reinigungsverfahren bekannt, die nicht auf das vorhergehende Reinigungsverfahren eingeschränkt sind. Diese sind bspw. beschrieben in Bailey, J.E. & Ollis, D.F. Biochemical Engineering Fundamentals, McGraw-HIII: New York (1986).

Die Identität und Reinheit der isolierten Aminosäure kann durch Standard-Techniken des Fachgebiets bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefasst in: Patek et al. (1994) Appl. Environ. Microbiol. 60: 133-140; Malakhova et al. (1996) Biotekhnologiya 11: 27-32; und Schmidt et al. (1998) Bioprocess Engineer. 19: 67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

### Beispiel 10: Klonierung der SEQ ID: No 1 für die Expression in Pflanzen

Soweit nichts anderes angegeben ist, werden Standardmethoden nach Sambrook et al., Molecular Cloning: A laboratory manual, Cold Spring Harbor 1989, Cold Spring Harbor Laboratory Press, verwendet.

Die PCR-Amplifikation von SEQ ID: No1 erfolgte entsprechend dem Protokoll der *Pfu* Turbo DNA-Polymerase (Fa. Stratagene). Die Zusammensetzung war wie folgt: 1x PCR-Puffer [20 mM Tris-HCl (pH 8,8), 2 mM MgSO₄, 10 mM KCl, 10mM (NH₄)SO₄, 0,1 % Triton X-100, 0,1 mg/ml BSA] 0,2 mM d-Thio-dNTP und dNTP (1:125), 100 ng genomische DNA von *Saccharomyces cerevisiae* (Stamm S288C; Fa. Research Genetics, Inc., jetzt Invitrogen), 50 pmol forward-Primer, 50 pmol reverse-Primer, 2,5 u *Pfu* Turbo DNA Polymerase. Die Amplifikationszyklen waren wie folgt
1 Zyklus für 3' bei 95°C, gefolgt von 36 Zyklen jeweils mit 1' 95°C, 45" 50°C, und 210" 72 °C, gefolgt von 1 Zyklus für 8 bei 72 °C, dann 4 °C .

Folgende Primer-Sequenzen wurden gewählt für das Gen SEQ ID: NO 1:
i) forward-Primer (SEQ ID NO:1)
   5'-GGAATTCCAGCTGACCACCATGACTGAATTCGAATTGCCTCCAA
ii) reverse-Primer (SEQ ID NO: 1)
   5'-GATCCCCGGAATTGCCATGTCAGTATTTGTAGGTTTTTATTTCGC

Die ersten 19 Nukleotide des oben angegebenen forward-Pimers enthalten als universellen Teil des Primers Schnittstellen für die Klonierung der Gene. Der folgende Teil des Primers, im angegebenen Fall 25 Nukleotide, sind spezifisch für das zu klonierende Gen. Der universelle Teil des reversen Primers enthält am 5'-Ende (20 Nukleotide) wieder Schnittstellen für die Klonierung. Der spezifische Teil, hier 26 Nukleotide, ist wieder für das zu klonierende Gen spezifisch. Der universelle Teil des forward-Pimers enthält eine EcoRI-Schnittstelle, während der unverselle Teil des reversen Primers eine Smal-Schnittstelle enthält. Beide Schnittstellen wurden zur Klonierung der Nukleinsäuresequenzen verwendet. Die Restriktion wurde wie unten beschrieben durchgeführt. Im Anschluß wurde das Amplifikat über QIAquick-Säulen nach Standardprotokoll (Fa. Qiagen) gereinigt.

Analog wurden Pimer für die weiteren im erfindungsgemäßen Verfahren verwendeten Sequenzen hergestellt und verwendet.

Die Restriktion der Vektor-DNA (30 ng) wurde mit *Eco*RI und *Sma*l nach dem Standard-Protokoll geschnitten, die *Eco*RI-Schnittstelle nach dem Standard-Protokoll aufgefüllt (Fa. MBI-Fermentas) und durch Zugabe von Hochsalzpuffer gestoppt. Die Reinigung der geschnittenen Vektorfragmente erfolgte über Nucleobond-Säulen nach Standardprotokoll (Machery-Nagel). Es wurde ein binärer Vektor verwendet, der zwischen den T-DNA-Bordersequenzen eine Selektionskassette (Promotor, Selektionsmarker, Terminator) sowie eine Expressionskassette mit Promotor, Klonierungskassette und Terminatorsequenz, enthielt. Außer in der Klonierungskassette besitzt der binäre Vektor keine *EcoRI* und *Sma*I-Schnittstellen Verwendbare einsetzbare binären Vektoren sind dem Fachmann bekannt und eine Übersicht über binäre Vektoren und ihre Verwendung gibt Hellens, R., Mullineaux, P. und Klee H., [(2000) " A guide to Agrobacterium binary vectors ,Trends in Plant Science, Vol 5 No.10, 446-451. Die Klonierung kann jenach verwendetem Vektor auch vorteilhaft über andere Restriktionsenzyme erfolgen. Entsprechende vorteilhafte Schnittstellen können dem ORF durch die Verwendung entsprechender Primer für die PCR-Ampliflkation angefügt werden.

Es wurden ca. 30 ng präparierter Vektor und eine definierte Menge präpariertes Amplifikat gemischt und durch Zugabe von Ligase ligiert.

Die Transformation der ligierten Vektoren erfolgte im gleichen Reaktionsgefäß durch Zugabe von kompetenten *E*. *col*i-Zellen (Stamm DH5alpha) und Inkubation für 20° bei 1 °C, gefolgt von einem Hltzeschock für 90" bei 42 °C und Abkühlung auf 4 °C. Dann erfolgte die Zugabe von Vollmedium (SOC) und eine Inkubation für 45' bei 37 °C. Im Anschluss wurde der gesamte Ansatz auf einer Agarplatte mit Antibiotika (ausgewählt, je nach verwendeten binären Vektor) ausplattiert und über Nacht bei 37 °C inkubiert.

Die erfolgreiche Klonierung wurde durch eine Amplifikation mit Hilfe von Primern überprüft, die stromaufwärts und stromabwärts von der Restriktionsschnittstelle binden und somit die Amplifikation der Insertion ermöglichen. Die Amplifikation erfolgte entsprechend dem Protokoll der *Taq* DNA-Polymerase (Fa. Gibco-BRL). Die Zusammensetzung war wie folgt: 1x PCR-Puffer [20 mM Tris-HCL (pH 8,4), 1,5 mM MgCl₂, 50 mM KCl, 0,2 mM dNTP, 5 pmol forward-Primer, 5 pmol reverse-Primer, 0,625 u Taq DNA-Polymerase.

Die Amplifikationszyklen waren wie folgt 1 Zyklus für 5' bei 94 °C, gefolgt von 35 Zyklen jeweils mit 15" 94 °C, 15" 66 °C und 5' 72 °C, gefolgt von 1 Zyklus für 10' bei 72 °C, dann 4 °C .

Es wurden mehrere Kolonien überprüft, wobei nur eine Kolonie weiterverwendet wurde, für die ein PCR-Produkt der erwarteten Größe detektiert wurde.

Ein Aliquot dieser positiven Kolonie wurde in ein mit Vollmedium (LB) befülltes Reaktionsgefäß transferiert und über Nacht bei 37 °C inkubiert. Das LB-Medium enthielt für die Selektion des Klones ein Antibiotikum das je nach verwendeten binären Vektor und dem darin enthaltenen Resistenzgen ausgewählt wurde.

Die Plasmidpräparation erfolgte nach den Vorgaben des Qlaprep-Standardprotokolls (Fa. Qiagen).

### Beispiel 11; Herstellung von transgenen Pflanzen, die SEQ ID: NO 1 exprimieren

1 ng der isolierten Plasmid-DNA wurde mittels Elektroporation in kompetente Zellen von *Agrobacterium tumefaciens, beispielsweise des* Stamms GV 3101 pMP90 (Koncz and Schell, Mol. Gen. Gent. 204, 383-396, 1986) transformiert Die Auswahl des Agrobakterienstamms, hängt von der Wahl des binären Vektors ab. Eine Übersicht über mögliche Stämme und ihre Eigenschaften findet sich in Hellens, R., Mullineaux, P. und Klee H. , [(2000)" A guide to Agrobacterium binary vectors ,Trends in Plant Science, Vol 5 No.10, 446-451. Darauf erfolgte die Zugabe von Vollmedium (YEP) und der Transfer in ein neues Reaktionsgefäß für 3 h bei 28 °C. Im Anschluss wurde der gesamte Ansatz auf YEP-Agarplatten mit den respektiven Antibiotika, bspw. Rifampicin und Gentamycin für GV3101 pMP90, sowie ein weiteres Antibiotika für die Selekion auf den binären Vektor ausplattiert und 48 h bei 28 °C inkubiert.

Die in den gemäß Beispiel 10 erzeugten Agrobakterien mit dem Plasmidkonstrukt wurden dann für die Pflanzentransformation verwendet.

Es wurde mit Hilfe einer Pipettenspitze eine Kolonie von der Agarplatte gepickt und in 3 ml TB Medium flüssig, das auch entsprechende Antibiotika, je nach Agrobakterienstamm und binären Plasmid enthielt, aufgenommen. Die Vorkultur wuchs 48 h bei 28 °C und 120 rpm.

Zur Hauptkultur wurden 400 ml LB Medium, das die gleichen Antibiotika wie zuvor enthielt, verwendet Die Vorkultur wurde in die Hauptkultur überführt. Diese wuchs 18 h bei 28 °C und 120 rpm. Nach dem Zentrifugieren bei 4000 rpm wurde das Pellet in Infiltrationsmedium (MS-Medium, 10 % Saccharose) resuspendiert.

Für die Anzucht der Pflanzen für die Transformation wurden Schalen (Piki Saat 80, grün mit Siebboden, 30 x 20 x 4,5 cm, Firma Wiesauplast, Kunststofftechnik, Deutschland) mit einem GS 90 Substrat (Einheitserde, Werkverband E.V., Deutschland) bis zur Hälfte gefüllt Die Schalen wurden über Nacht mit 0,05 % Previcur Lösung (Previcur N, Aventis CropScience oder Proplant, Chimac-Agriphar, Belgien) gewässert. *Arabidopsis thaliana* C24 Samen (Nottingham Arabidopsis Stock Centre, UK ; NASC Stock N906) wurden auf die Schale gestreut, etwa 1000 Samen je Schale. Die Schalen wurden mit einer Haube abgedeckt und in die Stratifizierung gestellt (8 h,110µ µmol/m²/s⁻¹, 22 °C; 16 h, dunkel, 6 °C). Nach 5 Tagen wurden die Schalen in das Kurztag-Phytotron gestellt (8h 130 µmol/m²/s⁻¹, 22 °C; 18 h, dunkel 20 °C). Hier blieben sie etwa 10 Tage, bis die ersten Laubblätter gebildet wurden.

Die Keimlinge wurden in Töpfe, die das gleiche Substrat enthielten, transferiert (Teku-Töpfe, 7 oder 10cm, Serie LC, Hersteller Pöppelmann GmbH&Co, Deutschland). Es wurden fünf oder neun Pflanzen in einen Topf pikiert. Die Töpfe wurden dann wieder zum weiteren Wachstum in das Kurztag-Phytotron gestellt.

Nach 10 Tagen kamen die Pflanzen dann in die Gewächshauskabine (Zusatzbeleuchtung, 16 h, 340 µE, 22 °C; 8 h, dunkel, 20 °C). Hier wuchsen sie noch 17 Tage weiter.

Für die Transformation wurden 6 Wochen alte, gerade blühende Arabidopsis-Pflanzen für 10 sec in die oben beschriebene Agrobakterien-Suspenslon getaucht. Dieses wurden zuvor mit 10µl Silwett L77 (Crompton S.A., Osi Specialties, Schweiz) versetzt. Die entsprechende Methode ist beschrieben in Clough und Bent, 1998 (Clough, JC and Bent, AF. 1998 Floral dip: a simplifled method for Agrobacterium-mediated transformation of Arabidopsis thaliana, Plant J. 16:735-743

Anschließend wurden die Pflanzen 18 h in eine feuchte Kammer gelegt. Danach stellte man die Töpfe zum weiteren Wachstum wieder ins Gewächshaus. Hier verblieben die Pflanzen noch 10 Wochen, bis die Samen geerntet werden konnten.

Je nach für die Selektion der transformierten Pflanzen verwendetem Resistenzmarker wurden die geernteten Samen im Gewächshaus ausgebracht und der Sprühselektion unterworfen oder aber nach Sterilisation auf Agarplatten mit dem respektiven Selektionsagens angezogen. Nach ca. 10-14 Tagen unterschieden sich die transformierten resistenten Pflanzen deutlich von den abgestorbenen Wildtypkeimlingen und konnten in 6-cm-Töpfe pikiert werden Die Samen der transgenen *A. thaliana* Pflanzen wurden im Gefrierschrank (bei -20 °C) aufbewahrt

Analog wurden auch die anderen im Verfahren verwendeten Sequenzen in Pflanzen exprimiert

### Beispiel 12 Anzucht der Pflanzen für bioanalytische Untersuchungen.

Um die transgene Pflanzen bioanalytisch zu untersuchen wurde sie in einer speziellen Anzucht gleichmäßig kultiviert. Dazu wurde als Bodengemisch das GS-90 Substrat in der Topfmaschine (Lalble System GmbH, Singen, Deutschland) gebracht und in die Töpfe gefüllt. Danach wurden 35 Töpfe in eine Schale zusammengestellt und mit Previcur behandelt. Für die Behandlung wurden 25 ml Previcur in 10 l Leitungswasser aufgenommen. Diese Menge reichte aus, um ca. 200 Töpfe zu behandeln. Die Töpfe wurden in die Previcur-Lösung gestellt und von oben zusätzlich mit Leitungswasser ohne Previcur begossen. Die Aussaat fand am gleichen oder innerhalb von drei Tagen statt.

Für die Aussaat wurden die im Kühlschrank (bei -20 °C) aufbewahrten Samen aus den Eppendorfröhrchen mit Hilfe eines Zahnstochers entnommen und in die Töpfe mit der Erde überführt. Insgesamt wurden ca. 5-12 Samen in dem Topf mittig verteilt.

Nach der Aussaat wurden die Schafen mit den Töpfen mit einer dazu passenden Plastikhaube bedeckt und in die Stratifizierungskammer für 4 Tage bei einer Dunkelheit und 4 °C gestellt. Die Feuchtigkeit betrug ca. 80-90 %. Nach der Stratifizierung wurden die Testpflanzen 22-23 Tage bei einem 16 h Licht und 8 h Dunkeirhythmus bei 20 °C, einer Luftfeuchtigkeit von 60 % und einer CO₂-Konzentration von 400 ppm kultiviert. Als Lichtquelle dienten Powerstar HQI-T 250 W/D Daylight Lampen von Osram, die ein dem Sonnenfarbspektrum ähnelndes Licht mit einer Lichtintensität von ca. 220 µE/m²/s⁻¹ erzeugen.

Im Alter von 8, 9 und 10 Tagen wurden die Pflanzen der Selektion auf den Resistenzmarker unterworfen. Nach weiteren 3-4 Tagen konnten dann deutlich die transgenen, resistenten Keimlinge (Pflänzchen im Vierblattstadium) von den nicht transformierten Pflänzchen unterschieden werden. Die nicht transgenen Keimlinge waren ausgebleicht oder abgestorben. Die transgenen resistenten Pflanzen wurden im Alter von 14 Tagen vereinzelt. Die am optimalsten in der Mitte des Topfes gewachsenen Pflanzen wurden als Zielpflanze betrachtet Mit Hilfe von Metalipinzetten wurden alle übrigen Pflanzen vorsichtig entfernt und verworfen.

Während des Wachstums wurden die Pflanzen mit destilliertem Wasser von oben (auf die Erde) und von unten in die Aufstellrinnen gegossen. Im Alter von 23 Tagen wurden dann die gewachsenen Pflanzen geerntet.

Analog wurden auch die Pflanzen mit den weiteren im erfindungsgemäßen Verfahren verwendeten Sequenzen analysiert.

### Beispiel 13: Metabolische Analyse von transformierten Pflanzen

Die erfindungsgemaß identifizierten Änderungen in dem Inhalt an beschriebenen Metaboliten wurden durch folgendes Verfahren identifiziert.

### a) Probennahme und Lagerung der Proben

Die Probenahme fand direkt in der Phytotronkammer statt. Die Pflanzen wurden mit einer kleinen Laborschere abgeschnitten, rasch auf einer Laborwaage gewogen, in eine vorgekühlte Extraktionshülse überfuhrt und in ein Aluminiumrack das durch Flüssigstickstoff gekühlt ist, gesteckt. Wenn erforderlich, können die Extraktionshülsen bei -80 °C im Gefrierschrank gelagert werden. Die Zeit vom Abschneiden der Pflanze bis zum Einfrieren in Flüssigstickstoff betrug nicht mehr als 10 - 20 s.

### b) Gefriertrocknung

Es wurde darauf geachtet, dass während des Versuches die Pflanzen bis zum ersten Kontakt mit Lösemitteln entweder in tiefkaltem Zustand blieben (Temperaturen < -40 °C) oder durch Gefriertrocknen vom Wasser befreit wurden.

Das Aluminiumrack mit den Pflanzenproben in den Extraktionshülsen wurde in die vorgekühlte (-40 °C) Gefiertrocknungsanlage gestellt. Die Anfangstemperatur während der Haupttrocknung betrug -35 °C, der Druck betrug 0,120 mbar. Während der Trocknung wurden die Parameter entsprechend eines Druck- und Temperaturprogrammes verändert Die Endtemperatur nach 12 Stunden betrug +30 °C, und der Enddruck lag bei 0,001 bis 0,004 mbar. Nach dem Abschalten der Vakuumpumpe und der Kältemaschine wurde das System mit Luft (durch ein Trockenrohr getrocknet) oder Argon belüftet

### c) Extraktion

Die Extraktionshülsen mit dem gefriergetrockneten Pflanzenmaterial wurden unmittelbar nach der Belüftung des Gefriertrocknungsgerates in die 5-mL-Extraktionskartuschen eines ASE-Gerätes (Acceleratad Solvent Extractor ASE 200 mit Solvent Controller und AutoASE-Software (Firma DIONEX)) überführt.

Die 24 Probenpositionen des ASE-Gerätes wurden mit Pflanzenproben gefüllt.

Die polaren Substanzen wurden mit ca. 10 mL Methanol/Wasser (80/20, v/v) bei T = 70 °C und p = 140 bar, 5 min Aufheizphase, 1 min statische Extraktion, extrahiert. Die lipophileren Substanzen wurden mit ca. 10 mL Methanol/Dichtormethan (40/60, v/v) bei T = 70 °C und p = 140 bar, 5 min Aufheizphase, 1 min statische Extraktion, extrahiert. Beide Lösemittelgemische wurden in dasselbe Probengläschen (Zentrifugengläser, 50 mL mit Schraubkappe und durchstechbarem Septum für die ASE (DIONEX)) extrahiert.

Die Lösung wurde mit internen Standards versetzt Ribitol, L-Glycln-2,2-d₂, L-Alanin-2,3,3,3-d₄, Mathionin-mathyl-d₃ und α-Methylglucopyranosid und Nonadecansäure-Methylester, Undecansaure-Methylester, Tridecansäure-Methylester, Pentadecansaure-Methylester, Nonacosansäure-Methylester.

Der Gesamtextrakt wurde mit 8 mL Wasser versetzt. Der feste Rückstand der Pflanzenprobe und die Extraktionshülse wurden verworfen.

Der Extrakt wurde geschüttelt und dann bei mindestens 1400 g für 5 bis 10 min zentrifugiert, um die Phasentrennung zu beschleunigen. 1 mL der überstehenden Methanol/Wasser-Phase ("polare Phase", farblos) wurde für die weitere GC-Analyse abgenommen, 1 mL wurde für die LC-Analyse entnommen. Der Rest der Methanol/Wasser-Phase wurde verworfen. 0, 5 mL der organischen Phase ("Lipide Phase", dunkelgrün) würde für die weitere GC-Analyse entnommen, 0,5 mL wurden für die LC-Analyse entnommen. Alle entnommenen Aliquote wurden mit dem IR-Dancer Infrarot-Vakuum-Evaporator (Hettich) zur Trockne eingedampft. Die maximale Temperatur während des Eindampf-Vorgangs überschritt dabei nicht 40 °C. Der Druck im Gerät betrug nicht weniger als 10 mbar.

### d) Weiterverarbeitung der Lipid-Phase für die LC/MS- oder LC/MS/MS-Analyse

Der zur Trocknung eingedampfte Lipide Extrakt wurde in Laufmittel aufgenommen. Der HPLC-Lauf wurde mit Gradientenelution durchgeführt.

Der zur Trocknung eingedampfte polare Extrakt wurde in Laufmittel aufgenommen. Der HPLC-Lauf wurde mit einer Gradientenelution durchgeführt

### e) Derivatisierung der Lipid-Phase für die GC/MS-Analyse

Zur Transmethanolyse wurde eine Mischung aus 140 µL Chloroform, 37 µL Salzsäure (37 Gew.- % HCl in Wasser). 320 µL Methanol und 20 µL Toluol zu dem eingedampften Extrakt zugefügt. Das Gefäß wurde dicht verschlossen und unter Schütteln 2 h bei 100 °C erhitzt Anschließend wurde die Lösung zur Trockne eingedampft. Der Rückstand wurde vollständig getrocknet

Die Methoximierung der Carbonylgruppen erfolgte durch Reaktion mit Methoxyamin-Hydrochlorid (5 mg/mL in Pyridin, 100 µL für 1,5 h bei 60 °C im dicht verschlossenen Gefäß. 20 µL einer Lösung von ungeradzahligen, geradkettigen Fettsäuren (je 0,3 mg/mL von Fettsäuren mit 7 bis 25 Kohlenstoffatomen und je 0,6 mg/mL der Fettsäuren mit 27, 29 und 31 Kohlenstoffatomen gelöst in einem Gemisch aus 30 % Pyridin in Toluol v/v) wurden als Zeitstandards zugesetzt. Schließlich wurde mit 100 µL N-Methyl-N-(trimethylsilyl)-2,2,2-trifluoracetamid (MSTFA) für 30 min bei 60 °C im wieder dicht verschlossenen Gefäß derivatisiert. Das Endvolumen vor der GC-injektion war 220 µL.

### f) Derivatisierung der polaren Phase for die GC/MS-Analyse

Die Methoximierung der Carbonylgruppen erfolgte durch Reaktion mit Methoxyamin-Hydrochlorid (5 mg/mL in Pyridin, 50 µL für 1,5 h bei 60 °C im dicht verschlossenen Gefäß. 10 µL einer Lösung von ungeradzahligen, geradkettigen Fettsäuren (je 0,3 mg/mL von Fettsäuren mit 7 bis 25 Kohlenstoffatomen und je 0,6 mg/mL der Fettsäuren mit 27, 29 und 31 Kohlenstoffatomen gelöst in einem Gemisch aus 30 % Pyridin in Toluol v/v) wurden als Zeitstandards zugesetzt Schließlich wurde mit 50 µL N-Methyl-N-(trimethylsilyl)-2,2,2-trifluoracetamid (MSTFA) für 30 min bei 60 °C im wieder dicht verschlossenen Gefäß derivatisiert. Das Endvolumen vor der GC-Injektion war 110 µL.

### g) Analyse der verschiedenen Pflanzenproben

Die Proben wurden in einzelnen Serien zu jeweils 20 Pflanzenproben (sogenannte Sequenzen) gemessen, wobei jede Sequenz mindestens 5 Wildtyppflanzen als Kontrolle enthält. Die Peakfläche oder die Peakhöhe jedes Analyten wurde durch die Peakfläche des jeweiligen Internen Standards dividiert. Die Daten wurden auf das für die Pflanze eingewogene Frischgewicht normiert. Die so berechneten Werte wurden auf die Wildtypkontrollgruppe bezogen, indem sie durch den Mittelwert der entsprechenden Daten der Wildtypkontrollgruppe derselben Sequenz dividiert wurden. Die erhaltenen Werte wurden als xfache bezeichnet, sind sequenzubergreifend vergleichbar und geben an, wie vielfach sich die Analytkonzentration in der Mutante relativ zur Wlldtypkontrolle unterscheidet.

Alternative können die Aminosäuren vorteilhaft Ober eine HPLC-Auftrennung in ethanolischen Extrakten nach Geigenberger et al. (Plant Cell & Environ, 19, 1996: 43 - 55) nachgewiesen werden.

Die Ergebnisse der verschiedenen Analysen der Pflanzen sind der folgenden Tabelle zu entnehmen:
YEL046C

| **Analyt-Nr** | **Analyt** | **Ratio_by_WT** | **Ratio_by_median** | **GC/LC** |
|---|---|---|---|---|
| 10000032 | Methionin | 3,46-3,58 | 3,31-3,4 | LC |
| 10000034 | Threonin | 0,45-0,15 | 0,61-0,15 | LC |
| 10000006 | Threonin | 0,17-0.16 | 0,18-0,16 | GC |
| 10000008 | Methionin | 3,31-3,67 | 3,5-3,53 | GC |

Spalte 1 der Tabelle gibt die Probennummer wieder. Spalte 2 ist die analysierte Aminosäure zu entnehmen. Spalte 3 zeigt das Verhältnis der analysierten Aminosäure zwischen der trangenen Pflanze und dem Wildtyp. Spalte 4 gibt das Verhältnis der transgenen Pflanze gegenüber dem Median anderer transgener Pflanzen, die nicht mit dem Threonin-Aldolase-Gen transformiert wurden, wieder. Spalte 5 gibt die Analysenmethode wieder.

Alle Ergebnisse ergaben sich bei unabhängiger Wiederholung der Analysen als signifikant.
**YJL055w**

| **Analyt-Nr** | **Analyt** | **Ratio_by_WT** | **GC/LC** |
|---|---|---|---|
| 10000032 | Methionin | 1,32-2,38 | LC |
| 10000034 | Threonin | 1,37-2,22 | LC |
| 30000006 | Threonin | 1,19-1,89 | GC |
| 30000008 | Methionin | 1,31-2,18 | GC |

Spalte 1 der Tabelle gibt die Analytnummer wieder. Spalte 2 ist die analysierte Aminosäure- zu entnehmen. Spalte 3 zeigt das Verhältnis der analysierten Aminosäure zwischen der transgenen Pflanze und dem Wildtyp (x-fache nach der Ratio_by_WT-Methode). Spalte 4 gibt die Analysenmethode wieder.

Alle Ergebnisse ergaben sich bei unabhängiger Wiederholung der Analysen als signifikant.

## Patentansprüche

1. Verfahren zur Herstellung von L-Lysin, L-Threonin oder L-Methionin in transgenen Organismen **dadurch gekennzeichnet, dass** das Verfahren folgende Schritte umfasst:
a) Einbringen einer Nukleinsäuresequenz, die für ein Lysin-abbauendes Protein codiert, oder
b) Einbringen einer Nukleinsäuresequenz, die den Lysinabbau in den transgenen Organismen erhöht und
c) Expression einer unter (a) oder (b) genannten Nukleinsäuresequenz im transgenen Organismus.

2. Verfahren zur Herstellung von L-Lysin, L-Threonin oder L-Methioninin transgenen Organismen nach Anspruch 1, **dadurch gekennzeichnet, dass** das Verfahren folgende Schritte umfasst gelöst:
a) Einbringen einer Nukleinsäuresequenz, die für ein Lysin-abbauendes Protein codiert, das folgende Consensus-Sequenz enthält
G[X]₄GIM[X]₄₈M[X]₂RK[X]₂M[X]₁₁GGXG[X]₃[X]₂E[X]₃W, oder
LG[X]₉
LVYGG[X]₃GIMGXVA[X]₉G[X]₃GXIP[X]₂₄MHXRK[X]₂M[X]₆F[X]3PGGXGTXEE[ X]₂ E[X]₂TW[X]₂IG[X]₃KP[X]₄N[X]₃FY[X]₁₄F, oder
b) Einbringen einer Nukleinsäuresequenz, die den Lysinabbau in den transgenen Organismen erhöht und
c) Expression einer unter (a) oder (b) genannten Nukleinsäuresequenz im transgenen Organismus.

3. Verfahren zur Herstellung von L-Lysin, L-Threonin oder L-Methionin in transgenen Organismen nach Anspruch 1 **dadurch gekennzeichnet, dass** im Verfahrensschritt (a) gemäß den Ansprüchen 1 bis 2 eine Nukleinsäuresequenz eingebracht wird, die ausgewählt ist aus der Gruppe der Nukleinsäuresequenzen:
i) einer Nukleinsäuresequenz mit der in, SEQ ID NO: 11, SEQ ID NO: 17, SEQ ID NO: 19, SEQ ID NO: 21, SEQ ID NO: 23 oder SEQ ID NO: 25 dargestellten Sequenz;
ii) einer Nukleinsäuresequenz, die aufgrund des degenerierten genetischen Codes durch Rückübersetzung der in SEQ ID NO: 12, SEQ ID NO: 18, SEQ ID NO: 20, SEQ ID NO: 22, SEQ ID NO: 24 oder SEQ ID NO: 26 dargestellten Aminosäuresequenz erhalten wird und
iii) eines Derivats der in SEQ ID NO: 11, SEQ ID NO: 17, SEQ ID NO: 19, SEQ ID NO: 21, SEQ ID NO: 23 oder SEQ ID NO: 25 dargestellten Nukleinsäuresequenz, die für Polypeptide mit der in, SEQ ID NO: 12, SEQ ID NO: 18, SEQ ID NO: 20, SEQ ID NO: 22, SEQ ID NO: 24 oder SEQ ID NO: 26 dargestellten Aminosäuresequenz codiert und mindestens 50 % Homologie auf - Aminosäureebene aufweisen, ohne dass die biologische Aktivität der Polypeptide wesentlich reduziert ist.

4. Verfahren zur Herstellung von L-Lysin, L-Threonin oder L-Methionin in transgenen Organismen nach den Ansprüchen 1 bis 3, **dadurch gekennzeichnet, dass** der transgene Organismus nach Einbringen und Expression der Nukleinsäure kultiviert und geerntet wird.

5. Verfahren zur Herstellung von L-Lysin, L-Threonin oder L-Methionin in transgenen Organismen nach den Ansprüchen 1 bis 4, **dadurch gekennzeichnet, dass** die Aminosäure aus dem Organismus oder dem Kulturmedium oder dem Organismus und dem Kulturmedium isoliert wird.

6. Verfahren zur Herstellung von L-Lysin, L-Threonin oder L-Methionin in transgenen Organismen nach den Ansprüchen 1 bis 5, **dadurch gekennzeichnet, dass** die Aminosäure L-Methionin ist.

7. Verfahren zur Herstellung von L-Lysin, L-Threonin oder L-Methionin in transgenen Organismen nach den Ansprüchen 1 bis 6, **dadurch gekennzeichnet, dass** es sich bei dem transgenen Organismus um einen Mikroorganismus oder um eine Pflanze handelt.

8. Verfahren zur Herstellung von L-Lysin, L-Threonin oder L-Methionin in transgenen Organismen nach den Ansprüchen 1 bis 7, **dadurch gekennzeichnet, dass** es sich bei dem transgenen Organismus um einen Mikroorganismus ausgewählt aus der Gruppe der Gattungen Corynebacterium, Brevibacterium, Escherichia, Bacillus, Rhodotorula, Hansenula, Schizosaccharomyces, Saccharomyces, Candida, Claviceps oder Flavobacterium handelt.

9. Verfahren zur Herstellung von L-Lysin, L-Threonin oder L-Methionin in transgenen Organismen nach den Ansprüchen 1 bis 7, **dadurch gekennzeichnet, dass** es sich bei dem transgenen Organismus um eine Pflanze ausgewählt aus der Gruppe der Nutzpflanzen handelt.

10. Verfahren zur Herstellung von L-Lysin, L-Threonin oder L-Methionin in transgenen Organismen nach Anspruch 9, **dadurch gekennzeichnet, dass** es sich bei dem transgenen Organismus um eine Pflanze ausgewählt aus der Gruppe der Erdnuss, Raps, Canola, Sonnenblume, Safflor (Färberdistel), Olive, Sesam, Haselnuss, Mandel, Avocado, Lorbeer, Kürbis, Salat, Lein, Soja, Pistazien, Borretsch, Mais, Weizen, Roggen, Hafer, Hirse, Triticale, Reis, Gerste, Maniok, Kartoffel, Zuckerrübe, Futterrübe, Aubergine, Tomate, Erbse, Alfaalfa sowie ausdauernde Gräser und Futterfeldfrüchte handelt.

11. Verfahren zur Herstellung von L-Lysin, L-Threonin oder L-Methionin in transgenen Organismen nach den Ansprüchen 1 bis 10, **dadurch gekennzeichnet, dass** die Nukleinsäuresequenz aus einem Eukaryont stammt.

12. Verfahren zur Herstellung von L-Lysin, L-Threonin oder L-Methionin in transgenen Organismen nach den Ansprüchen 1 bis 11, **dadurch gekennzeichnet, dass** die Nukleinsäuresequenz aus der Gattung Saccharomyces stammt.

13. Verfahren zur Herstellung von L-Lysin, L-Threonin oder L-Methionin in transgenen Organismen nach den Ansprüchen 1 bis 12, **dadurch gekennzeichnet, dass** die Nukleinsäuresequenz zum Einbringen und zur Expression in ein Nukleinsäurekonstrukt oder einen Vektor eingebaut wird.

14. Verfahren zur Herstellung von L-Lysin, L-Threonin oder L-Methionin in transgenen Organismen nach den Ansprüchen 1 bis 13, **dadurch gekennzeichnet, dass** zusätzlich Biosynthesegene der im Verfahren hergestellten Aminosäure in den Organismus eingebracht werden.

15. Nukleinsäurekonstrukt enthaltend eine Nukleinsäuresequenz gemäß den Ansprüchen 2 bis 3, die funktionell mit einem oder mehreren Regulationssignalen verknüpft ist.

16. Vektor enthaltend eine Nukleinsäuresequenz gemäß den Ansprüchen 2 bis 3 oder ein Nukleinsäurekonstrukt gemäß Anspruch 15.

17. Transgener prokaryontischer oder eukaryontischer Organismus enthaltend mindestens eine Nukleinsäuresequenz gemäß den Ansprüchen 2 bis 3 oder mindestens ein Nukleinsäurekonstrukt gemäß Anspruch 15 oder mindestens einen Vektor gemäß Anspruch 16.

18. Transgener prokaryontischer oder eukaryontischer Organismus nach Anspruch 17, wobei es sich um einen Mikroorganismus oder um eine Pflanze handelt.

19. Transgener prokaryontischer oder eukaryontischer Organismus nach Anspruch 18, wobei es sich um einen Mikroorganismus der Gattung Corynebacterium oder Brevibacterium handelt.

20. Transgener prokaryontischer oder eukaryontischer Organismus nach Anspruch 18, wobei es sich um eine Pflanze ausgewählt aus der Gruppe der Gattung der Erdnuss, Raps, Canola, Sonnenblume, Safflor (Färberdistel), Olive, Sesam, Haselnuss, Mandel, Avocado, Lorbeer, Kürbis, Salat, Lein, Soja, Pistazien, Borretsch, Mais, Weizen, Roggen, Hafer, Hirse, Triticale, Reis, Gerste, Maniok, Kartoffel, Zuckerrübe, Futterrübe, Aubergine, Tomate, Erbse, Alfalfa sowie ausdauernde Gräser und Futterfeldfrüchte handelt.

21. Verwendung der transgenen Organismen gemäß den Ansprüchen 17 bis 20 oder von L-Lysin, L-Threonin oder L-Methionin hergestellt nach einem Verfahren gemäß den Ansprüchen 1 bis 14 zur Herstellung eines Futtermittel- oder Nahrungsmittel, zur Herstellung von Kosmetika oder Pharmazeutika.
